# EUROPEAN PATENT SPECIFICATION

(11) **EP 2 773 771 B1**
(45) Date of publication and mention of the grant of the patent: **05.09.2018**
(21) Application number: 12816101.5
(22) Date of filing: 30.10.2012
(51) Int. Cl.: C12Q 1/6816, C12Q 1/6827, C12Q 1/6886

(54) **METHODS FOR THE DETECTION, VISUALIZATION AND HIGH RESOLUTION PHYSICAL MAPPING OF GENOMIC REARRANGEMENTS IN BREAST AND OVARIAN CANCER GENES AND LOCI BRCA1 AND BRCA2 USING GENOMIC MORSE CODE IN CONJUNCTION WITH MOLECULAR COMBING**
VERFAHREN ZUM NACHWEIS, ZUR VISUALISIERUNG UND ZUR HOCHAUFLÖSENDEN PHYSIKALISCHEN KARTIERUNG VON GENOMNEUANORDNUNGEN IN BRUST-UND OVARIALKREBSGENEN UND DEN LOCI BRCA1 UND BRCA2 MITTELS EINES GENOMMORSE-CODES IN VERBINDUNG MIT MOLEKULARER KÄMMUNG
PROCÉDÉS DE DÉTECTION, VISUALISATION ET CARTOGRAPHIE PHYSIQUE À HAUTE RÉSOLUTION DE RÉARRANGEMENTS GÉNOMIQUES DANS DES GÈNES ET LOCI BRCA1 ET BRCA2 DU CANCER DU SEIN ET DE L'OVAIRE À L'AIDE D'UN CODE MORSE GÉNOMIQUE EN CONJONCTION AVEC UN PEIGNAGE MOLÉCULAIRE

(30) Priority: 31.10.2011 US 201161553906 P
(43) Date of publication of application: 10.09.2014
(73) Proprietor: Genomic Vision, 92220 Bagneux (FR)
(72) Inventor: BENSIMON, Aaron, F-92160 Antony (FR); CEPPI, Maurizio, F-92130 Issy-les-Moulineaux (FR); CHEESEMAN, Kevin, F-94500 Champigny-sur-Marne (FR); CONSEILLER, Emmanuel, F-75015 Paris (FR); WALRAFEN, Pierre, F-92120 Montrouge (FR)
(74) Representative: Desaix, Anne
(86) International application number: PCT/IB2012/002422
(87) International publication number: WO 2013/064895

(56) References cited:
- WO-A1-98/55650
- WO-A1-2008/028931
- GAD SOPHIE ET AL: "Color bar coding the BRCA1 gene on combed DNA: A useful strategy for detecting large gene rearrangements", GENES CHROMOSOMES & CANCER, JOHN WILEY & SONS, INC, US, vol. 31, no. 1, 1 May 2001 (2001-05-01), pages 75-84, XP002512886, ISSN: 1045-2257, DOI: 10.1002/GCC.1120 cited in the application
- S GAD: "bar code screening on combed DNA for large rearrangements of the BRCA1 and BRCA2 genes in French breast cancer families", J MED GENET, vol. 39, 1 January 2002 (2002-01-01), pages 817-821, XP55054670, cited in the application
- THOMAS V O HANSEN ET AL: "Large BRCA1 and BRCA2 genomic rearrangements in Danish high risk breast-ovarian cancer families", BREAST CANCER RESEARCH AND TREATMENT, KLUWER ACADEMIC PUBLISHERS, BO, vol. 115, no. 2, 12 June 2008 (2008-06-12) , pages 315-323, XP019671286, ISSN: 1573-7217
- SYLVIE MAZOYER: "Genomic rearrangements in theBRCA1 andBRCA2 genes", HUMAN MUTATION, vol. 25, no. 5, 1 January 2005 (2005-01-01), pages 415-422, XP55054669, ISSN: 1059-7794, DOI: 10.1002/humu.20169
- KEVIN CHEESEMAN ET AL: "A diagnostic genetic test for the physical mapping of germline rearrangements in the susceptibility breast cancer genes BRCA1 and BRCA2", HUMAN MUTATION, vol. 33, no. 6, 4 April 2012 (2012-04-04), pages 998-1009, XP55054668, ISSN: 1059-7794, DOI: 10.1002/humu.22060

## Description

### BACKGROUND OF THE INVENTION

### Field of the Invention

The invention relates to a method for detecting genomic rearrangements in BRCA1 and BRCA2 genes and loci at high resolution using Molecular Combing and relates to a method of determining a predisposition to diseases or disorders associated with these rearrangements including predisposition to ovarian cancer or breast cancer.

### Description of the Related Art

Breast cancer is the most common malignancy in women, affecting approximately 10% of the female population. Incidence rates are increasing annually and it is estimated that about 1.4 million women will be diagnosed with breast cancer annually worldwide and about 460,000 will die from the disease. Germline mutations in the hereditary breast and ovarian cancer susceptibility genes BRCA1 (MIM#113705) and BRCA2 (MIM# 600185) are highly penetrant (King et al., 2003), (Nathanson et al., 2001). Screening is important for genetic counseling of individuals with a positive family history and for early diagnosis or prevention in mutation carriers. When a BRCA1 or BRCA2 mutation is identified, predictive testing is offered to all family members older than 18 years. If a woman tests negative, her risk becomes again the risk of the general population. If she tests positive, a personalized surveillance protocol is proposed: it includes mammographic screening from an early age, and possibly prophylactic surgery. Chemoprevention of breast cancer with anti-estrogens is also currently tested in clinical trial and may be prescribed in the future.

Most deleterious mutations consist of either small frameshifts (insertions or deletions) or point mutations that give rise to premature stop codons, missense mutations in conserved domains, or splice-site mutations resulting in aberrant transcript processing (Szabo et al., 2000). However, mutations also include more complex rearrangements, including deletions and duplications of large genomic regions that escape detection by traditional PCR-based mutation screening combined with DNA sequencing (Mazoyer, 2005).

Techniques capable of detecting these complex rearrangements include Southern blot analysis combined with long-range PCR or the protein truncation test (PTT),quantitative multiplex PCR of short fluorescent fragments (QMPSF) (Hofmann et al., 2002), real-time PCR, fluorescent DNA microarray assays, multiplex ligation-dependent probe amplification (MLPA)(Casilli et al., 2002), (Hofmann et al., 2002) and high-resolution oligonucleotide array comparative genomic hybridization (aCGH) (Rouleau et al., 2007), (Staaf et al., 2008). New approaches that provide both prescreening and quantitative information, such as qPCR-HRM and EMMA, have recently been developed and genomic capture combined with massively parallel sequencing has been proposed for simultaneous detection of small mutations and large rearrangements affecting 21 genes involved in breast and ovarian cancer (Walsh et al., 2010).

Molecular Combing is a powerful FISH-based technique for direct visualization of single DNA molecules that are attached, uniformly and irreversibly, to specially treated glass surfaces (Herrick and Bensimon, 2009); (Schurra and Bensimon, 2009). This technology considerably improves the structural and functional analysis of DNA across the genome and is capable of visualizing the entire genome at high resolution (in the kb range) in a single analysis. Molecular Combing is particularly suited to the detection of genomic imbalances such as mosaicism, loss of heterozygosity (LOH), copy number variations (CNV), and complex rearrangements such as translocations and inversions (Caburet et al., 2005), thus extending the spectrum of mutations potentially detectable in breast cancer genes. Molecular Combing has been successfully employed for the detection of large rearrangements in BRCA1 ((Gad et al., 2001), (Gad et al., 2002a), (Gad et al., 2003) and BRCA2 (Gad et al., 2002b), using a first-generation **"color bar coding"** screening approach. However, these techniques lack resolution and cannot precisely detect large rearrangements in and around BRCA1 and BRCA2.

In distinction to the prior art techniques, as disclosed herein, the inventors provide a novel Genetic Morse Code Molecular Combing procedure that provides for high resolution visual inspection of genomic DNA samples, precise mapping of mutated exons, precise measurement of mutation size with robust statistics, simultaneous detection of BRCA1 and BRCA2 genetic structures or rearrangements, detection of genetic inversions or translocations, and substantial elimination of problems associated with repetitive DNA sequences such as Alu sequences in BRCA1 and BRCA2 loci.

### BRIEF SUMMARY OF THE INVENTION

The BRCA1 and BRCA2 genes are involved, with high penetrance, in breast and ovarian cancer susceptibility. About 2% to 4% of breast cancer patients with a positive family history who are negative for BRCA1 and BRCA2 point mutations can be expected to carry large genomic alterations (deletion or duplication) in one of the two genes, and especially BRCA1. However, large rearrangements are missed by direct sequencing. Molecular Combing is a powerful FISH-based technique for direct visualization of single DNA molecules, allowing the entire genome to be examined at high resolution in a single analysis. A novel predictive genetic test based on Molecular Combing is disclosed herein. For that purpose, specific BRCA1 and BRCA2 "Genomic Morse Codes" (GMC) were designed, covering coding and non-coding regions and including large genomic portions flanking both genes. The GMC is a series of colored signals distributed along a specific portion of the genomic DNA which signals arise from probe hybridization with the probes of the invention. The concept behind the GMC has been previously defined in WIPO patent application WO/2008/028931, and relates to the method of detection of the presence of at least one domain of interest on a macromolecule to test.

A measurement strategy is disclosed for the GMC signals, and has been validated by testing 6 breast cancer patients with a positive family history and 10 control patients. Large rearrangements, corresponding to deletions and duplications of one or several exons and with sizes ranging from 3 kb to 40 kb, were detected on both genes (BRCA1 and BRCA2). Importantly, the developed GMC allowed to unambiguously localize several tandem repeat duplications on both genes, and to precisely map large rearrangements in the problematic Alu-rich 5'-region of BRCA1. This new developed Molecular Combing genetic test is a valuable tool for the screening of large rearrangements in BRCA1 and BRCA2 and can optionally be combined in clinical settings with an assay that allows the detection of point mutations.

A substantial technical improvement compared to the prior color bar coding approach is disclosed here that is based on the design of second-generation high-resolution BRCA1 and BRCA2 Genomic Morse Codes (GMC). Importantly, repetitive sequences were eliminated from the DNA probes, thus reducing background noise and permitting robust measurement of the color signal lengths within the GMC. Both GMC were statistically validated on samples from 10 healthy controls and then tested on six breast cancer patients with a positive family history of breast cancer. Large rearrangements were detected, with a resolution similar to the one obtained with a CGH (1-3 kb). The detected mutation demonstrates the robustness of this technology, even for the detection of problematic mutations, such as tandem repeat duplications or mutations located in genomic regions rich of repetitive elements. The developed Molecular Combing platform permits simultaneous detection of large rearrangements in BRCA1 and BRCA2, and provides novel genetic tests and test kits for breast and ovarian cancer.

### BRIEF DESCRIPTION OF THE DRAWINGS

The patent or application file contains at least one drawing executed in color.

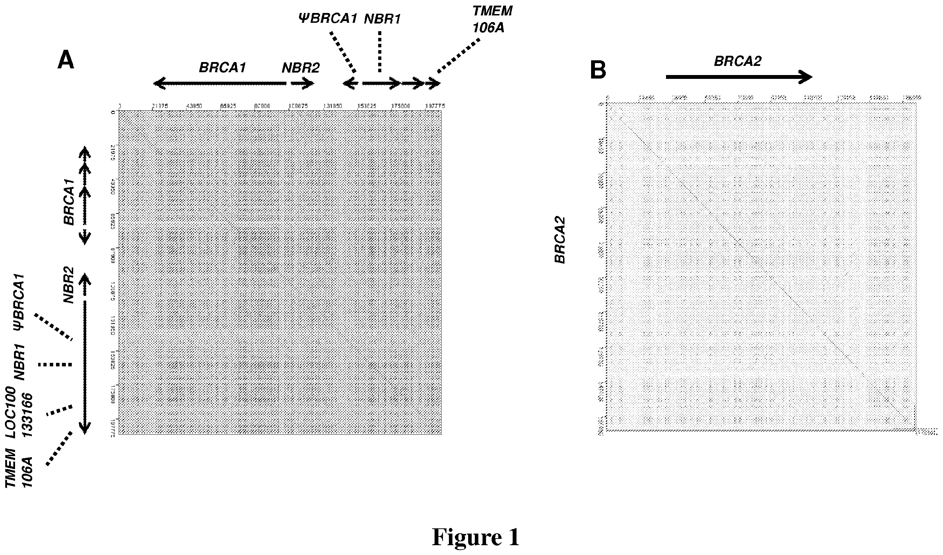

Figs 1A and 1B: Dot plot alignments of the human *BRCA1* and *BRCA2* genomic regions. Dot plot matrix showing self-alignment of the 207-kb genomic regions derived from the BAC RP11-831F13 (ch17:41172482-41379594) encoding *BRCA1*(1A), and the 172-kb genomic regions derived from the BAC RP11-486017 (ch13: 32858070-33030569) encoding *BRCA2* (1B), based on the GRCh37 genome assembly (also called hg19, April 2009 release) and using *JDotter* software (URL:http://_athena.bioc.uvic.ca/tools/JDotter). The main diagonal represents alignment of the sequence with itself, while the lines out of the main diagonal represent similar or repetitive patterns within the sequence. The dark regions contain large numbers of repetitive sequences, whereas the bright regions contain none. The genes are represented as arrows in the 5'→3' direction. The sizes and BAC coordinates of the genomic regions, encoding for repetitive sequences, not included in the DNA probes are indicated in the tables on the left. The bottom panels indicate the name and the size (in kb) of the DNA probes (35 for *BRCA1* and 27 for *BRCA2*) without potentially disturbing repetitive sequences, derived from the bioinformatics analysis.

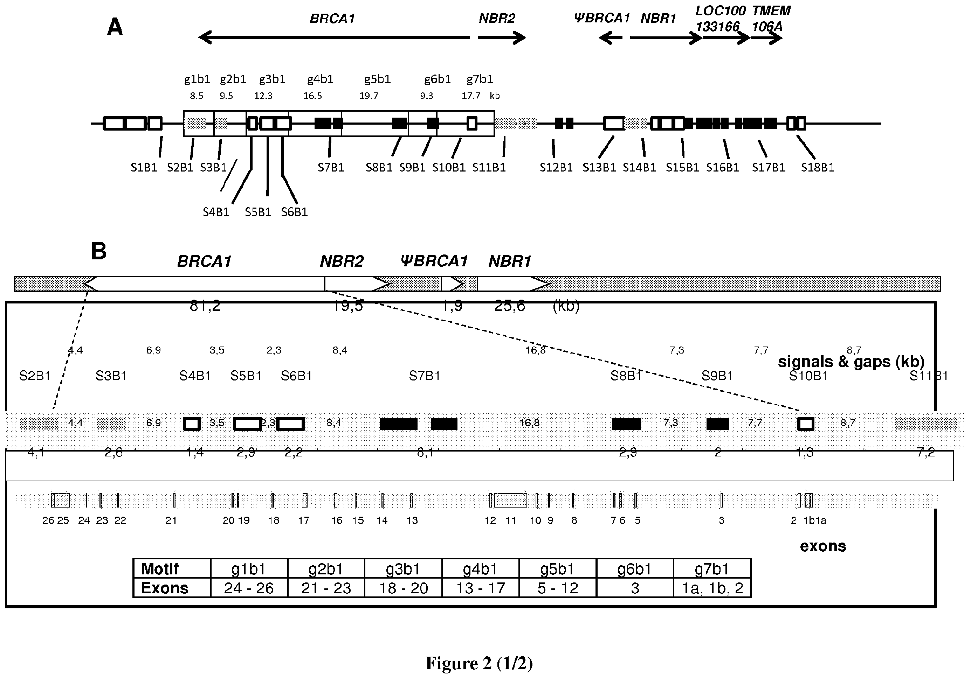

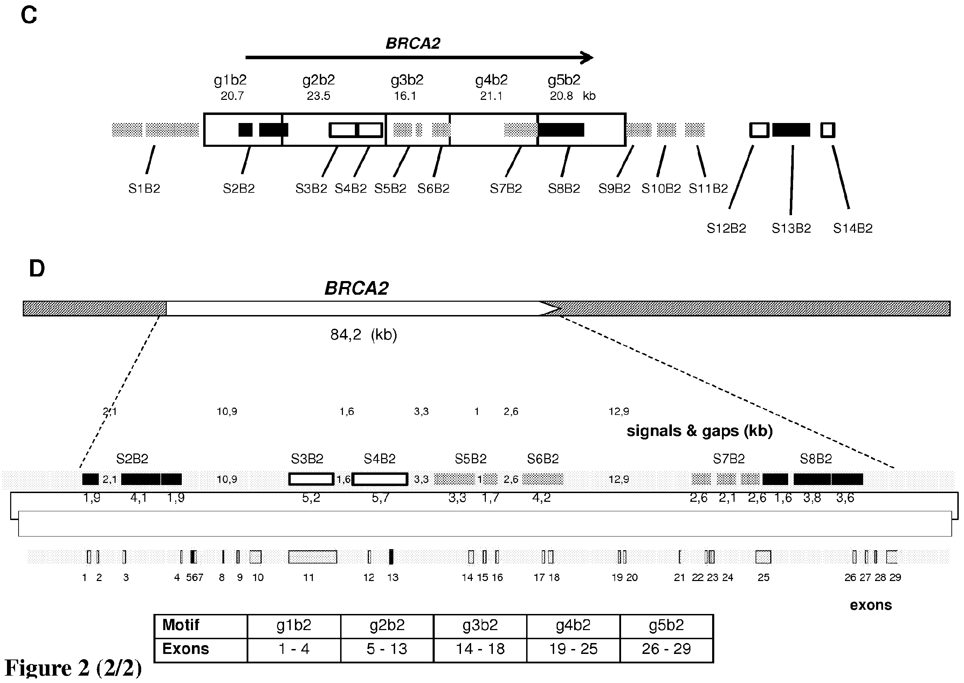

Figs 2A, 2B, 2C and 2D: *In silico*-generated Genomic Morse Codes designed for high-resolution physical mapping of the *BRCA1* and *BRCA2* genomic regions. Probes colors are represented here as grayscale variations: blue probes are shown as black boxes, green probes as white boxes and red probes as gray boxes. (2A) The complete *BRCA1* GMC covers a genomic region of 200 kb and is composed of 18 signals (S1B1 - S18B) of a distinct color (green, red or blue). Each signal is composed of 1 (*e.g.,* S2B1) to 3 small horizontal bars (*e.g.,* S15B1), each bar corresponding to a single DNA probe. The region encoding the *BRCA1* gene (81.2 kb) is composed of 7 "motifs" (g1b1-g7b1). Each motif is composed of 1 to 3 small horizontal bars and a black "gap" (no signal). (2B) Zoom-in on the *BRCA1* gene-specific signals and relative positions of the exons. (2C) The complete *BRCA2* GMC covers a genomic region of 172 kb and is composed of 14 signals (S1B2 - S14B2) of a distinct color (green, red or blue). Each signal is composed of 1 (*e.g.,* S14B2) to 5 small horizontal bars (*e.g.,* S1B2). The region encoding the *BRCA2* gene (84.2 kb) is composed of 5 motifs 24 (glb2-g5b2). Each motif is composed of 2 to 4 small horizontal bars and a black gap. (2D) Zoom-in on the *BRCA2* gene-specific signals and relative positions of the exons. Deletions or insertions, if present, will appear in the region covered by the motifs.

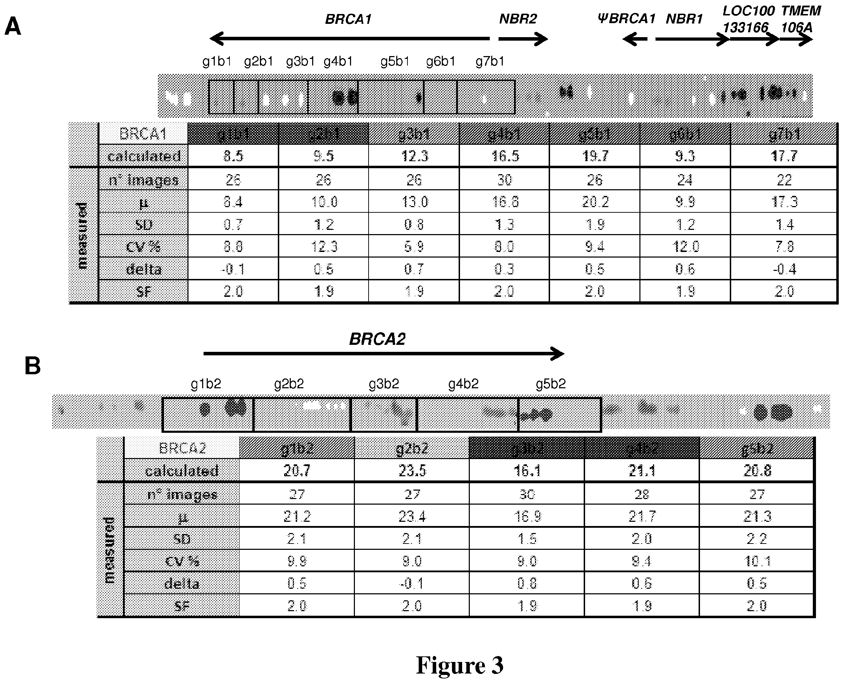

Figs 3A and 3B: Validation of *BRCA1* and *BRCA2* Genomic Morse Code signals in control patients. Original microscopy images consist of three channel images where each channel is the signal from a given fluorophore - these are acquired separately in the microscopy procedure. These channels are represented here as different shades on a grayscale: blue probes are shown in black, green probes in white and red probes in dark gray, while background (absence of signal) is light gray. In diagrams, the same convention as in figure 2 is used. The aspect ratio was not preserved, signals have been "widened" (i.e. stretched perpendicularly to the direction of the DNA fiber) in order to improve the visibility of the probes. Typical *BRCA1* (3A) and *BRCA2* (3B) Genomic Morse Code signals and measured motif lengths (kb) in one control patient (absence of large rearrangements) are reported. The *BRCA1* and *BRCA2* signals obtained after microscopic visualization are shown at the top of the tables, including the position of the motifs related to the gene of interest. Typically 20 to 40 images (*n° images*) were selected, and motifs were measured with GVLab software. For each motif, the following values were determined: the theoretical calculated length (*calculated* (kb)), the mean measured length (*µ* (kb)), the standard deviation (*SD* (kb)), the coefficient of variation (*CV* (%)), the difference between *µ* and *calculated* (*delta*), and the stretching factor (*SF* =(*calculated*/*µ*) *x 2*). In the absence of mutations, *SF* values are comprised between 1.8 and 2.2 and *delta* values are comprised between -1.9 kb and 1.9 kb (see Material and Methods in Example 1 for details).

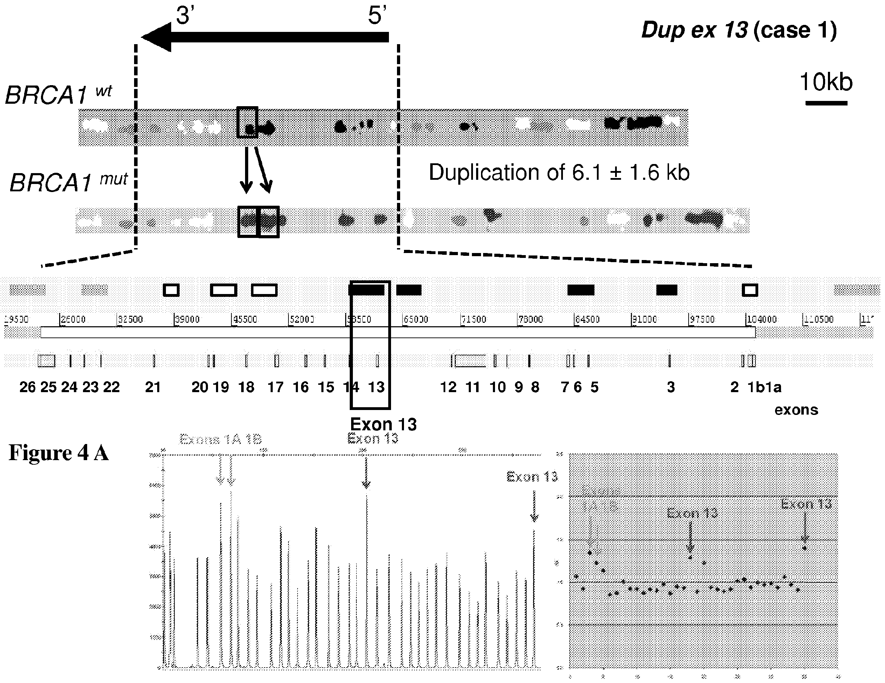

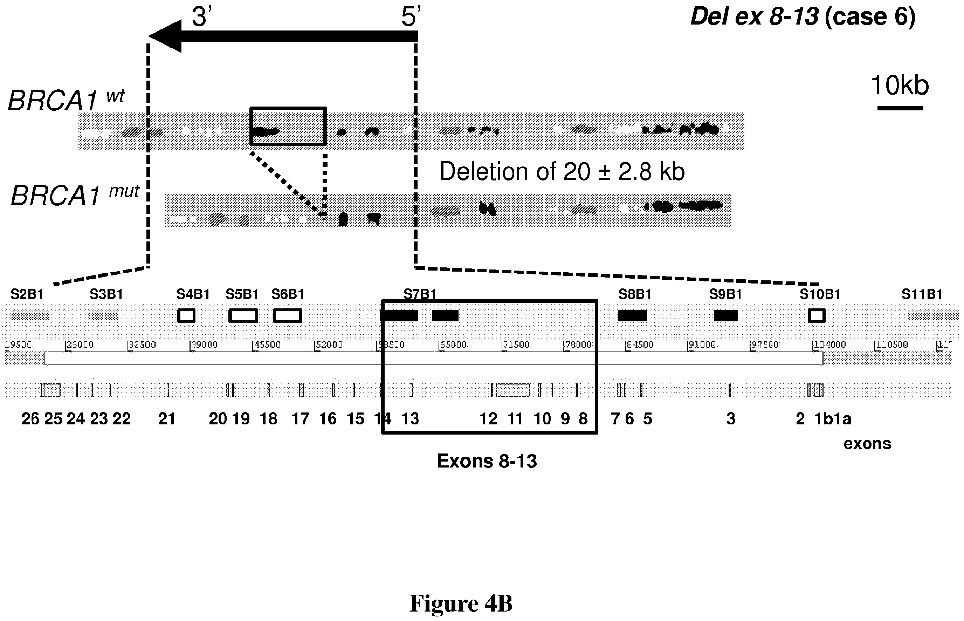

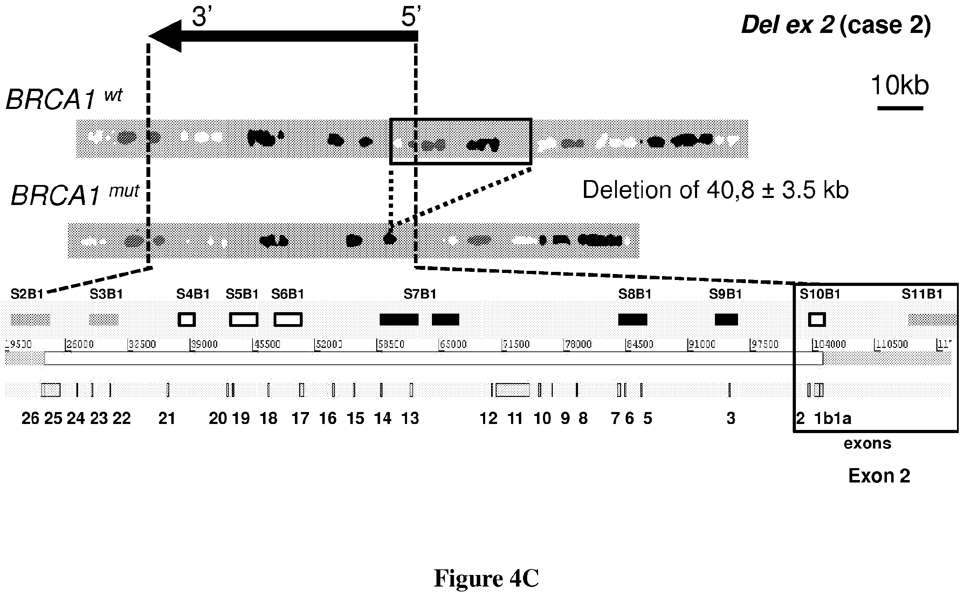

Figs 4A, 4B, and 4C: Known *BRCA1* large rearrangements detected in breast cancer patients.
   As in figures 2 and 3, diagrams and microscopy images are represented in shades of gray, with the following correspondence: blue is shown as black, green as white and red as dark gray (on a light gray background) and aspect ratio in microscopy images may have been modified for clarity.DNA isolated from EBV-immortalized B lymphocytes collected from breast cancer patients was analyzed by Molecular Combing to confirm known large rearrangements previously characterized by aCGH (see Table 3). Three large rearrangements out of seven are shown in the figure: (4A) Dup ex 13 (case 1), visible as a tandem repeat duplication of the blue signal S7B1. The *g4B1* motif (16.5 kb) was first measured on a mixed population of 40 images, comprising wild type and mutated alleles, and following values were obtained: *µ*(*BRCA1^{wt}* + *BRCA1^{mt}* signals) = 19 kb ± 3.5 kb, *delta* = 2.5 kb (duplication is confirmed since *delta* ≥ 2kb). The images were then divided in two groups: 21 images were classified as *BRCA1^{wt}*, and 19 images were classified as *BRCA1^{mt}*. The size was then calculated as the difference between the motif mean sizes of the two alleles: *µ*(*BRCA1^{wt}*) = 16.1 ± 1.6 kb, *µ*(*BRCA1^{mt}*) = 22.2 ± 2.0 kb, *mutation size* = *µ*(*BRCA1^{mt}*) - *µ*(*BRCA1^{wt}*) = 6.1 ± 1.6 kb. The bottom panel shows the MLPA fragment display (left) and the normalized MLPA results (right), arrows indicating exons interpreted as duplicated. (4B) Del ex 8 - 13 (case 6), visible as a deletion of the blue signal S7B1, including a large genomic portion between signals S7B1 and S8B1. The *g4B1* (16.5 kb) and the *g5b1* (19.7 kb) motifs were first measured on a mixed population of 23 images, yielding following values. For *g4b1*: *µ*(*BRCA1^{wt}* + *BRCA1^{mt}*) = 17.5 ± 4.0 kb, *delta* = - 2.2 kb (*delta* ≤ - 2kb); 13 images were then classified as *BRCA1^{wt}* and 10 images as *BRCA1^{mt}*: *µ*(*BRCA1^{wt}*) = 20.8 ± 1.6 kb, *µ*(*BRCA1^{mt}*) = 13.3 ± 1.1 kb, *µ*(*BRCA1^{mt}*) *- µ*(*BRCA1^{wt}*) = - 7.5 ± 1.6 kb. For *g5b1*: *µ*(*BRCA1^{wt}* + *BRCA1^{mt}*) = 12.8 ± 5.5 kb, *delta* = - 3.7 kb (*delta* ≤ - 2kb); 13 images were then classified as *BRCA1^{wt}*and 10 images as *BRCA1^{mt}: µ*(*BRCA1^{wt}*) = 18.3 ± 1.3 kb, *µ* (*BRCA1^{mt}*) = 5.8 ± 0.5 kb, *µ*(*BRCA1^{mt}*) - *µ*(*BRCA1^{wt}*) = - 12.5 ± 1.0 kb. Total *mutation size* = mutation size *g4B1* + mutation size *g5b1* = -20 ± 2.8 kb. (4C) Del ex 2 (case 2), visible as a deletion of the green signal S10B1, as well as a large genomic portion of the 5' region upstream of *BRCA1,* including S11B1 and S12B1. To confirm the presence of the deletion in the *BRCA1* gene, the *g7B1* (17.7 kb) motif was first measured on a mixed population of 20 images, yielding following values: *µ*(*BRCA1^{wt}* + *BRCA1^{mt}*)= 12.3 ± 2.9 kb, *delta* = - 5.4 kb (deletion is confirmed since *delta* ≤ - 2kb). To measure mutations size within the *BRCA1* gene, 11 images were then classified as *BRCA1^{wt}* and 9 images as *BRCA1^{mt},* yielding following values: *µ* (*BRCA1^{wt}*) = 18.1 ± 0.7 kb, *µ*(*BRCA1^{mt}*) = 8.1 ± 1.6 kb, *mutation size* = *µ*(*BRCA1^{mt}*) - *µ* (*BRCA1^{wt}*) = -10 ± 1.5 kb. To include the deleted genomic region upstream of *BRCA1* and determine the whole mutation size, we had to measure the genomic region between the signals S8B1 and S14B1 (89.9 kb). The S8B1-S14B1 region was first measured on 19 images, yielding following values: *µ*(*BRCA1^{wt}* + *BRCA1 ^{mt}*)= 62.3 ± 18.4 kb, *delta* = - 27.6 kb. 11 images were then classified as *BRCA1^{wt}*, and 8 images as *BRCA1^{mt},* yielding following values: *µ*(*BRCA1^{wt}*) = 92.2 ± 3.2 kb, *µ*(*BRCA1^{mt}*) = 51.4 ± 2.2 kb, *mutation size* = *µ*(*BRCA1^{mt}*) - *µ*(*BRCA1^{wt}*) = - 40.8 ± 3.5 kb. The *BRCA1* signals, derived from both the wild-type (= *BRCA1^{wt}*) and the mutated allele (= *BRCA1^{mt}*), obtained after microscopic visualization, are shown in the top panels. The position, nature (deletion or duplication) and size (in kb) of the detected large rearrangements are indicated in orange. The zoom-in on the *BRCA1* gene-specific signals and the relative positions of the mutated exons are shown in the bottom panels. *mt,* mutated allele; *wt,* wild-type allele.
Figure 5. GMC used for BRCA1. Another example of a high resolution genomic morse code to analyze the *BRCA1* gene region is shown here. As in figure 2, diagrams are represented with the following correspondence: blue probes are shown as black, green as white and red as dark gray.

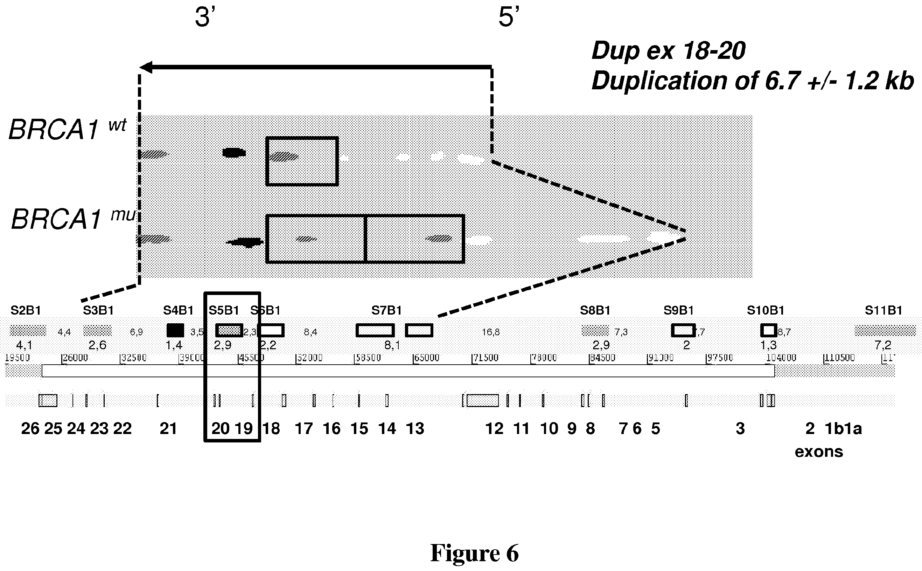

Fig 6: Duplication in exons 18-20 of BRCA1
   The GMC described in figure 2, with probe labels modified as shown in the diagram, was hybridized on this sample. As in figures 2 and 3, diagrams and microscopy images are represented in shades of gray, with the following correspondence: blue is shown as black, green as white and red as dark gray (on a light gray background) and aspect ratio in microscopy images may have been modified for clarity. By visual inspection, there appears to be a tandem duplication of the red signal S5B1. After measurement, the mutation was estimated to have a size of 6.7 ± 1.2 kb, restricted to a portion of the genome that encodes for exons 18 to 20. The estimated mutation size is fully in line with the 8.7 kb reported in the literature (Staaf, 2008). Details on the measurement and statistical analysis can be found in Example 1.

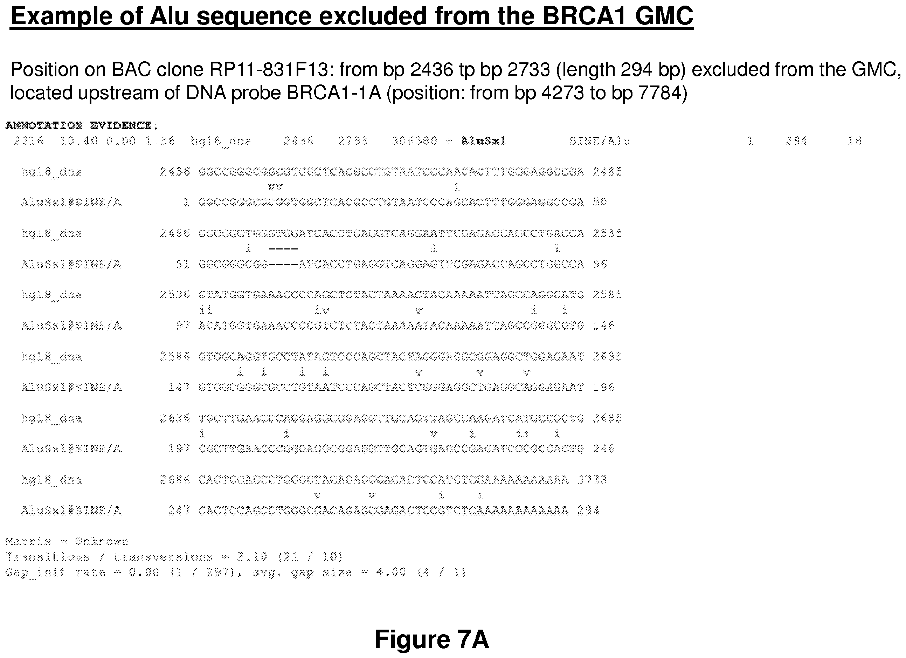

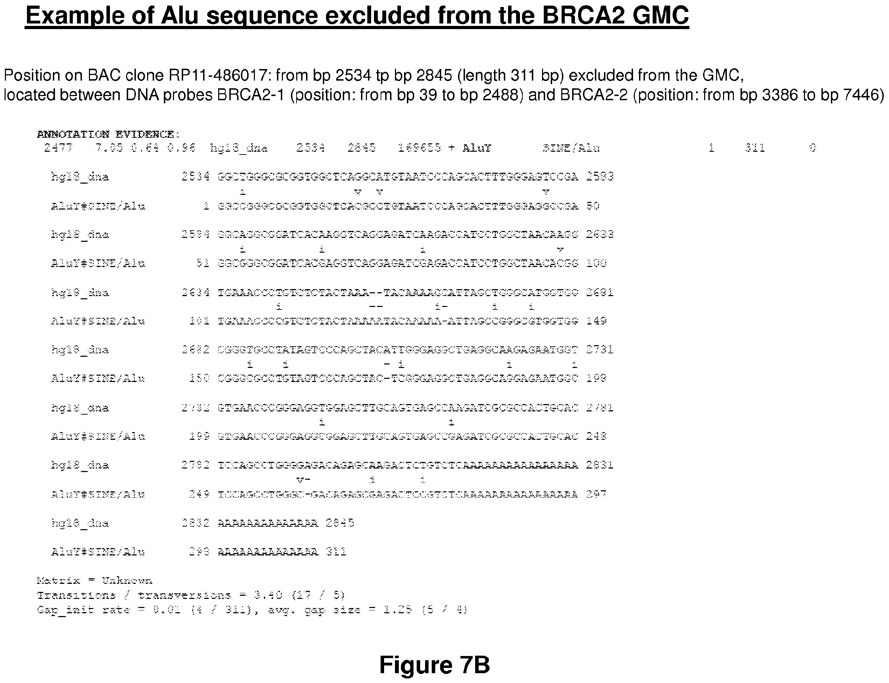

Fig. 7 9: examples of Alu sequences excluded from the *BRCA1* (A) and *BRCA2* (B) GMCs.

### DETAILED DESCRIPTION OF THE INVENTION

### Definitions

*Physical mapping*: is the creation of a genetic map defining the position of particular elements, mutations or markers on genomic DNA, employing molecular biology techniques. Physical mapping does not require previous sequencing of the analyzed genomic DNA.

*FISH*: Fluorescent *in situ* hybridization.

*Molecular Combing:* a FISH-based technique for direct visualization of single DNA molecules that are attached, uniformly and irreversibly, to specially treated glass surfaces.

Predictive genetic testing: screening procedure involving direct analysis of DNA molecules isolated from human biological samples (e.g.: blood), used to detect gene mutations associated with disorders that appear after birth, often later in life. These tests can be helpful to people who have a family member with a genetic disorder, but who have no features of the disorder themselves at the time of testing. Predictive testing can identify mutations that increase a person's chances of developing disorders with a genetic basis, such as certain types of cancer.

*Polynucleotides:* This term encompasses naturally occurring DNA and RNA polynucleotide molecules (also designated as sequences) as well as DNA or RNA analogs with modified structure, for example, that increases their stability. Genomic DNA used for Molecular Combing will generally be in an unmodified form as isolated from a biological sample. Polynucleotides, generally DNA, used as primers may be unmodified or modified, but will be in a form suitable for use in amplifying DNA. Similarly, polynucleotides used as probes may be unmodified or modified polynucleotides capable of binding to a complementary target sequence. This term encompasses polynucleotides that are fragments of other polynucleotides such as fragments having 5, 10, 15, 20, 30, 40, 50, 75, 100, 200 or more contiguous nucleotides.

*BRCA1 locus:* This locus encompasses the coding portion of the human BRCA1 gene (gene ID: 672, Reference Sequence NM_007294) located on the long (q) arm of chromosome 17 at band 21, from base pair 41,196,311 to base pair 41,277,499, with a size of 81 kb (reference genome Build GRCh37/hg19), as well as its introns and flanking sequences. Following flanking sequences have been included in the BRCA1 GMC: the 102 kb upstream of the BRCA1 gene (from 41,277,500 to 41,379,500) and the 24 kb downstream of the BRCA1 gene (from 41,196,310 to 41,172,310). Thus the BRCA1 GMC covers a genomic region of 207 kb.

*BRCA2 locus:* This locus encompasses the coding portion of the human BRCA2 gene (gene ID : 675, Reference Sequence NM_000059.3) located on the long (q) arm of chromosome 13 at position 12.3 (13q12.3), from base pair 32,889,617 to base pair 32,973,809, with a size of 84 kb (reference genome Build GRCh37/hg19), as well as its introns and flanking sequences. Following flanking sequences have been included in the BRCA2 GMC: the 32 kb upstream of the BRCA2 gene (from 32,857,616 to 32,889,616) and the 56kb downstream of the BRCA2 gene (from 32,973,810 to 33,029,810). Thus the BRCA2 GMC covers a genomic region of 172 kb.

*Germline rearrangements:* genetic mutations involving gene rearrangements occurring in any biological cells that give rise to the gametes of an organism that reproduces sexually, to be distinguished from somatic rearrangements occurring in somatic cells.

*Point mutations*: genetic mutations that cause the replacement of a single base nucleotide with another nucleotide of the genetic material, DNA or RNA. Often the term point mutation also includes insertions or deletions of a single base pair.

*Frameshift mutations:* genetic mutations caused by indels (insertions or deletions) of a number of nucleotides that is not evenly divisible by three from a DNA sequence. Due to the triplet nature of gene expression by codons, the insertion or deletion can change the reading frame (the grouping of the codons), resulting in a completely different translation from the original.

*Tandem repeats duplications:* mutations characterized by a stretch of DNA that is duplicated to produce two or more adjacent copies, resulting in tandem repeats.

*Tandem repeat array:* a stretch of DNA consisting of two or more adjacent copies of a sequence resulting in gene amplification. A single copy of this sequence in the repeat array is called a *repeat unit.* Gene amplifications occurring naturally are usually not completely conservative, i.e. in particular the extremities of the repeated units may be rearranged, mutated and / or truncated. In the present invention, two or more adjacent sequences with more than 90 % homology are considered a repeat array consisting of equivalent repeat units. Unless otherwise specified, no assumptions are made on the orientation of the repeat units within a tandem repeat array.

*Complex Rearrangements:* any gene rearrangement that can be distinguished from simple deletions or duplications. Examples are translocations or inversions.

*Probe:* This term is used in its usual sense for a polynucleotide of the invention that hybridizes to a complementary polynucleotide sequences (target) and thus serves to identify the complementary sequence. Generally, a probe will be tagged with a marker, such as a chemical or radioactive market that permits it to be detected once bound to its complement. The probes described herein are generally tagged with a visual marker, such as a fluorescent dye having a particular color such as blue, green or red dyes. Probes according to the invention are selected to recognize particular portions or segments of BRCA1 or BRCA2, their exons or flanking sequences. For BRCA1, probes generally range in length between 200 bp and 5,000 bp. For BRCA2, probes generally range in length between 200 bp and 6,000 bp. The name and the size of probes of the invention are described in Figure 2. Representative probes according to the invention, such as BRCA1-1A (3,458 bp) or BRCA2-1 (2,450 bp), are described in Tables 1 and 2. In a particular embodiment of the invention, the probes are said to be "free of repetitive nucleotidic sequences". Such probes may be located in genomic regions of interest which are devoid of repetitive sequences as defined herein.

*Detectable label or marker*: any molecule that can be attached to a polynucleotide and which position can be determined by means such as fluorescent microscopy, enzyme detection, radioactivity, etc, or described in the US application nr. US2010/0041036A1 published on 18 February 2010.

*Primer*: This term has its conventional meaning as a nucleic acid molecule (also designated sequence) that serves as a starting point for polynucleotide synthesis. In particular, Primers may have 20 to 40 nucleotides in length and may comprise nucleotides which do not base pair with the target, providing sufficient nucleotides in their 3'-end, especially at least 20, hybridize with said target. The primers of the invention which are described herein are used to produce probes for BRCA1 or BRCA2, for example, a pair of primers is used to produce a PCR amplicon from a bacterial artificial chromosome as template DNA. The sequences of the primers used herein are referenced as SEQ ID 1 to SEQ ID 130 in in Table 8. In some cases (details in table 1), the primers contained additional sequences to these at their 5' end for ease of cloning. These additional sequences are SEQ ID 134 (containing a poly-A and a restriction site for AscI) for forward primers and SEQ ID 135 (containing a poly-A and a restriction site for PacI) for reverse primers.

Tables 1 and 2 and 8 describe representative primer sequences and the corresponding probe coordinates.

*Genomic Morse Code(s):* A GMC is a series of "dots" (DNA probes with specific sizes and colors) and "dashes" (uncolored spaces with specific sizes located between the DNA probes), designed to physically map a particular genomic region. The GMC of a specific gene or locus is characterized by a unique colored "signature" that can be distinguished from the signals derived by the GMCs of other genes or loci. The design of DNA probes for high resolution GMC requires specific bioinformatics analysis and the physical cloning of the genomic regions of interest in plasmid vectors. Low resolution CBC has been established without any bioinformatics analysis or cloning procedure.
*Repetitive nucleotidic sequences:* the *BRCA1* and *BRCA2* gene loci contain repetitive sequences of different types: SINE, LINE, LTR and Alu. The repetitive sequences which are present in high quantity in the genome sequence but are absent from the probes, i.e. were removed from the *BRCA1* and *BRCA2* GMCs of the invention, are mainly Alu sequences, having lengths of about 300 bp (see Figure S1, S1, S2 and S3 for more details). This mainly means that the percentage of the remaining Alu-sequences within the DNA probes compared to percentage present in the reference genome is less than 10% and preferably less than 2%. Accordingly, a polynucleotide is said to be "free of repetitive nucleotidic sequences" when at least one type of repetitive sequences(e.g., Alu, SINE, LINE or LTR) selected from the types of repetitive sequences cited above is not contained in the considered probe, meaning that said probes contains less than 10%, preferably less than 2% compared to percentage present in the reference genome. Examples of Alu repeats found in the BRCA1 and 2 genes are given in figure 7A and 7B, while tables 3 and 4 list the repeats identified by RepeatMasker contained in the BAC clone RP11-831F13 covering the genomic region of BRCA1 (fig.7A) or in the BAC clone RP11-486017 covering the genomic region of BRCA2 (fig. 7B). In both cases, Alu repeats are counted separately in regions where our probes hybridize and in the regions excluded from this probe design.

The term *"intragenic large rearrangement"* as used herein refers to deletion and duplication events that can be observed in a gene sequence, said sequence comprising in a restricted view introns and exons; and in an extended view introns, exons, the 5' region of said gene and the 3' region of said gene. The intragenic large rearrangement can also cover any gain or loss of genomic material with a consequence in the expression of the gene of interest.

The term *"locus"* as used herein refers to a specific position of a gene or other sequence of interest on a chromosome. For BRCA1 and BRCA2, this term refer to the BRCA1 and BRCA2 genes, the introns and the flanking sequences refer to BRCA1/BRCA2 + introns and flanking sequences

The term *"nucleic acid"* as used herein means a polymer or molecule composed of nucleotides, e.g., deoxyribonucleotides or ribonucleotides, or compounds produced synthetically such as PNA which can hybridize with naturally occurring nucleic acids in a sequence specific manner analogous to that of two naturally occurring nucleic acids, e.g., can participate in Watson-Crick base pairing interactions. Nucleic acids may be single- or double-stranded or partially duplex.

The terms *"ribonucleic acid"* and "RNA" as used herein mean a polymer or molecule composed of ribonucleotides.

The terms *"deoxyribonucleic acid"* and "DNA" as used herein mean a polymer or molecule composed of deoxyribonucleotides.

The term *"sample"* as used herein relates to a material or mixture of materials, typically, although not necessarily, in fluid form, containing one or more components of interest. For Molecular Combing, the sample will contain genomic DNA from a biological source, for diagnostic applications usually from a patient. The invention concerns means, especially polynucleotides, and methods suitable for *in vitro* implementation on samples.

The terms *"nucleoside"* and *"nucleotide"* are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, *e.g.,* wherein one or more of the hydroxyl groups are replaced with halogen atoms or aliphatic groups, or are functionalized as ethers, amines, or the like.

The term *"stringent conditions"* as used herein refers to conditions that are compatible to produce binding pairs of nucleic acids, *e.g.,* surface bound and solution phase nucleic acids, of sufficient complementarity to provide for the desired level of specificity in the assay while being less compatible to the formation of binding pairs between binding members of insufficient complementarity to provide for the desired specificity. Stringent assay conditions are the summation or combination (totality) of both hybridization and wash conditions.

A *"stringent hybridization"* and "stringent hybridization wash conditions" in the context of nucleic acid hybridization (*e.g*., as required for Molecular Combing or for identifying probes useful for GMC) are sequence dependent, and are different under different experimental parameters. Stringent hybridization conditions that can be used to identify nucleic acids within the scope of the invention can include for example hybridization in a buffer comprising 50% formamide, 5X SSC, and 1% SDS at 42°C, or hybridization in a buffer comprising 5.times.SSC and 1% SDS at 65°C., both with a wash of 0.2X SSC and 0.1% SDS at 65°C. Exemplary stringent hybridization conditions can also include a hybridization in a buffer of 40% formamide, 1M NaCl, and 1% SDS at 37°C, and a wash in 1X SSC at 45°C. Alternatively, hybridization to filter-bound DNA in 0.5 MNaHPO₄, 7% sodium dodecyl sulfate (SDS), 1 mM EDTA at 65°C, and washing in 0.1X SSC/0.1% SDS at 68°C can be employed. Yet additional stringent hybridization conditions include hybridization at 60°C. or higher and 3X SSC (450 mM sodium chloride/45 mM sodium citrate) or incubation at 42°C. in a solution containing 30% formamide, 1 M NaCl, 0.5% sodium sarcosine, 50 mM MES, pH 6.5. Those of ordinary skill will readily recognize that alternative but comparable hybridization and wash conditions can be utilized to provide conditions of similar stringency.

A probe or primer *located* in a given genomic locus means a probe or a primer which hybridizes to the sequence in this locus of the human genome. Generally, probes are double stranded and thus contain a strand that is identical to and another that is reverse complementary to the sequence of the given locus. A primer is single stranded and unless otherwise specified or indicated by the context, its sequence is identical to that of the given locus. When specified, the sequence may be reverse complementary to that of the given locus. In certain embodiments, the stringency of the wash conditions that set forth the conditions that determine whether a nucleic acid is specifically hybridized to a surface bound nucleic acid. Wash conditions used to identify nucleic acids may include for example a salt concentration of about 0.02 molar at pH 7 and a temperature of at least about 50°C or about 55°C to about 60°C; or a salt concentration of about 0.15 M NaCl at 72°C. for about 15 minutes; or a salt concentration of about 0.2X SSC at a temperature of at least about 50°C. or about 55°C. to about 60°C. for about 15 to about 20 minutes; or, the hybridization complex is washed twice with a solution with a salt concentration of about 2X SSC containing 0.1% SDS at room temperature for 15 minutes and then washed twice by 0.1X SSC containing 0.1% SDS at 68°C. for 15 minutes; or, equivalent conditions. Stringent conditions for washing can also be for example 0.2X SSC/0.1% SDS at 42°C.

A specific example of stringent assay conditions is rotating hybridization at 65°C in a salt based hybridization buffer with a total monovalent cation concentration of 1.5 M followed by washes of 0.5X SSC and 0.1X SSC at room temperature.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may be employed, as appropriate.

*"Sensitivity"* describes the ability of an assay to detect the nucleic acid of interest in a sample. For example, an assay has high sensitivity if it can detect a small concentration of the nucleic acid of interest in sample. Conversely, a given assay has low sensitivity if it only detects a large concentration of the nucleic acid of interest in sample. A given assay's sensitivity is dependent on a number of parameters, including specificity of the reagents employed (such as types of labels, types of binding molecules, etc.), assay conditions employed, detection protocols employed, and the like. In the context of Molecular Combing and GMC hybridization, sensitivity of a given assay may be dependent upon one or more of: the nature of the surface immobilized nucleic acids, the nature of the hybridization and wash conditions, the nature of the labeling system, the nature of the detection system, etc.

### Design of high-resolution BRCA1 and BRCA2 Genomic Morse Codes

Molecular Combing has already been used to detect large rearrangements in the *BRCA1* and *BRCA2* genes, but the hybridization DNA probes originally used were part of a low resolution "color bar coding" screening approach and were composed of cosmids, PACs and long-range PCR products only partially covering the *BRCA1* and *BRCA2* loci. Of importance, the DNA probes also encoded repetitive sequences particularly abundant at the two loci (Gad et al., 2001), (Gad et al., 2002b). As a consequence, detection of the probes often resulted in the superposition of individual colored signals (*e.g*., yellow spots resulting from superposition of green and red signals) and in strong background noise, undermining the quality of the images and preventing the development of a robust strategy to measure the signals length. Such a low resolution screening approach did not allow the unambiguous visualization of complex mutations, such as tandem repeat duplications (Schurra and Bensimon, 2009), (Herrick and Bensimon, 2009).

The inventors found that high-resolution Genomic Morse Codes (GMC) that were designed by covering more of the *BRCA1* and *BRCA2* genomic regions and by removing the disturbing repetitive sequences from the DNA probes resolved the problems associated with the prior color bar coding approach.

To visualize the repetitive sequences, dot-plot alignments of the BAC clones used for DNA probe cloning were first performed, based on the Genome Reference Consortium GRCh37genome assembly (also called hg19, April 2009 release). Based on Repeat Masker analysis (www._repeatmasker.org), the percentages of Alu repetitive DNA in the *BRCA1*- and *BRCA2* - encoding BACs were 35% and 17%, respectively (data not shown). This resulted in a dark dot-plot matrix dense in repetitive sequences for *BRCA1* (1.6 Alu sequences per 1 kb of DNA, compared to an average in the human genome of only 0.25 Alu / kb), and a brighter dot-plot matrix for *BRCA2* (0.64 Alu / kb of DNA) (Fig. 1A and 1B).

35 genomic regions in the *BRCA1* locus and 27 regions in the *BRCA2* locus that had significantly less repetitive sequences were identified and were used to design and clone DNA hybridization probes compatible with the visualization process associated with Molecular Combing. The name, size and color of the DNA hybridization probes, and the exons covered by the probes, are shown in Figure 1 and listed in Tables 1 (*BRCA1*) and 2 (*BRCA2*)*.* Adjacent DNA probes of the same color form a signal. Thus, a Genomic Morse Code is composed of sequences of colored signals distributed along a specific portion of the genomic DNA. Colors were chosen to create unique non-repetitive sequences of signals, which differed between *BRCA1* and *BRCA2.* The sizes and the BAC coordinates of the genomic regions, encoding for repetitive sequences, excluded from the BRCA1 / BRCA2 GMC DNA probes are shown in Tables 3 & 4. 257 Alu sequences were excluded from the BRCA1 GMC and 85 Alu sequences were excluded from the BRCA2 GMC. Examples of removed Alu sequences from both GMCs are shown in Figure 7.

To facilitate Genomic Morse Code recognition and measurement, signals located on the genes were grouped together in specific patterns called "motifs". An electronic reconstruction of the designed *BRCA1* and *BRCA2* Genomic Morse Codes is shown in **Fig**. **2****.** In this design, the *BRCA1* Genomic Morse Code covers a region of 200 kb, including the upstream genes *NBR1, NBR2, LOC100133166,* and *TMEM106A,* as well as the pseudogene *ψBRCA1*.The complete *BRCA1* Genomic Morse Code is composed of 18 signals (S1B1 - S18B), and the 8 *BRCA1 -* specific signals are grouped together in 7 motifs (g1b1-g7b1) (Figure 2A and B). The *BRCA2* Genomic Morse Code covers a genomic region of 172 kb composed of 14 signals (S1B2 - S14B2), and the 7 *BRCA2*-specific signals are grouped together in 5 motifs (g1b2-g5b2) (**Fig. 2C and 2D**). Deletions or insertions, if present, are detected in the genomic regions covered by the motifs.

### Validation of BRCA1 and BRCA2 Genomic Morse Code signals in control patients

The newly designed Genomic Morse Codes were first validated on genomic DNA isolated from 10 randomly chosen control patients. Typical visualized signals and measured motif lengths for one control donor are reported in **Fig**. **3****,** with *BRCA1* at the top and *BRCA2* at the bottom. For each Genomic Morse Code, 20 to 30 images were typically analyzed by measuring the length of the different motifs(see *nr.* images in **Fig**. **3**). Importantly, for all the motifs, the measured values were always similar to the calculated values (compare *µ* and *calculated* in Fig.3). The robustness of *BRCA1* and *BRCA2* signal measurement was determined by calculating the mean of the measured motif lengths in all 10 control patients, and by comparing the mean measured values with the calculated values (see Table S1). For *BRCA1,* we obtained *delta* values (difference between *µ* and *calculated*) in the range of - 0.2 kb and +0.8 kb, whereas *BRCA2 delta* values were in the range of- 0.3 kb and + 0.4kb, underlining the precision of the developed measurement approach and confirming that the resolution of Molecular Combing is around ± 1kb (Michalet et al., 1997).Molecular Combing allows DNA molecules to be stretched uniformly with a physical distance to contour length correlation of 1 µm, equivalent to 2 kb (Michalet et al., 1997). As a consequence, in the absence of large rearrangements, the derived stretching factor (*SF*) has a value close to 2 kb /□µm (±0.2).This was confirmed in all the analyzed control donors, with *SF* values in the range of 1.8 - 2.2 kb / µm(see *SF* in **Fig. 3**). Accordingly, in the presence of large rearrangements in both *BRCA1* and *BRCA2, SF* values are expected to be ≥ 2.3 kb /µm (for deletions) or ≤ 1.7 kb /µm (for duplications) and the corresponding *delta* values are expected to be ≥ 2 kb (for duplications) or ≤ -2 kb (for deletions).Importantly, the presence of a large rearrangement is always validated by visual inspection of the corresponding Genomic Morse Code.

### Detection of known BRCA1 large rearrangements in breast cancer patients

Molecular Combing was then applied to 6 samples from patients with a severe family history of breast cancer and known to bear large rearrangements either on *BRCA1* or *BRCA2* (preliminary screening performed by MLPA or QMPSF). Importantly, the Molecular Combing analysis was a blind test, meaning that for each of the patient the identity of the mutation was unknown before the test, since it was revealed to the operator only after having completed the test on all the samples. 6 different large rearrangements were identified (see Table 5). Importantly, all 6 known mutations have been recently characterized by aCGH and break-point sequencing (Rouleau 2007) and were correctly identified and characterized by Molecular Combing. Complete characterization of the 3 most significant known *BRCA1* large rearrangements is reported in **Fig**. **4** and is described here below.

### Duplication of exon 13 (BRCA1)

By visual inspection via Molecular Combing, this mutation appears as a partial tandem duplication of the blue signal S7B1 (**Fig. 4A****,** top panel). After measurement, the mutation was estimated to have a size of 6.1 kb, restricted to a portion of the DNA probe *BRCA1-8* that encodes exon 13. The estimated mutation size is fully in line with the 6.1 kb reported in the literature (Puget 1999), and according to the Breast Cancer Information Core database, this mutation belongs to the 10 most frequent mutations in*BRCA1* (Szabo 2000). Duplications are difficult to detect with quantitative methods such as MLPA, often giving rise to false-positive signals (Cavalieri 2007, Staaf 2008). The characterized patient was therefore also analyzed by MLPA, and the duplication of exon 13 was confirmed. More importantly, we also detected a duplication of exons 1A + 1B (**Fig. 4A**, bottom panel), but this mutation could not be detected by Molecular Combing (a duplication of exon 13, if present, would yield two distinct S10B1signals). Therefore, we consider the exon 1A+1B mutation detected by MLPA to be a false-positive signal. The risk of false-positive signals is more limited in Molecular Combing.

### Deletion from exon 8 to exon 13 (BRCA1)

By visual inspection, the mutation appeared as a visible as a deletion of the blue signal S7B1, including a large genomic portion between signals S7B1 and S8B1 (Figure 4B). After measurement, the mutation was estimated to have a size of 26.7 kb in a portion of the *BRCA1* gene that encodes from exon 8 to exon 13. The size reported in the literature is 23.8 kb, and this is a recurrent mutation in the French population (Mazoyer 2005, Rouleau 2007).

### Deletion of the 5' region to exon 2 (BRCA1)

By visual inspection, the mutation appeared as a deletion of the green signal S10B1, as well as a large genomic portion of the 5' region upstream of *BRCA1,* including S11B1 and S12B1 (Figure 4C). After measurement, the mutation was estimated to have a size of 37.1 kb, encompassing the portion of the *BRCA1* gene that encodes exon 2, the entire *NBR2* gene (signal S11B1), the genomic region between *NBR2* and the pseudogene ψ*BRCA1* (signal S12B1), and a portion of ψ*BRCA1* (signal S13B1). Importantly, the reported size of this type of rearrangement is highly variable, originally in the range of 13.8 to 36.9 kb (Mazoyer 2005) and more recently between 40.4 and 58.1 kb (Rouleau 2007). Six different exon 1-2 deletions have been reported, 16 times, in a number of different populations (Sluiter 2010). The rearrangement reported here has been described three times with an identical size (36 934bp). The hotspot for recombination is explained by the presence of ψ*BRCA1.* Molecular combing proved capable of characterizing events even in this highly homologous region.

The results reported herein disclose and exemplify the development of a novel genetic test based on Molecular Combing for the detection of large rearrangements in the *BRCA1* and *BRCA2* genes. Large rearrangements represent 10-15% of deleterious germline mutations in the *BRCA1* gene and 1-7% in the *BRCA2* gene (Mazoyer, 2005). Specific high-resolution GMC were designed and were tested on a series of 16 biological samples; the robustness of the associated measurement strategy was statistically validated on 10 control samples, and 6 different large rearrangements were detected and characterized in samples from patients with a severe family history of breast cancer. The robustness of the newly designed GMC, devoid of repetitive sequences, is endorsed by the fact that our Molecular Combing method confirmed the results obtained with high-resolution zoom-in aCGH (11 k) on the same samples (Rouleau et al., 2007), with a resolution in the 1-2 kb range.

Tandem repeat duplications are the most difficult large rearrangements to detect. Contrary to other techniques, such as aCGH and MLPA, the capacity of Molecular Combing to visualize hybridized DNA probes at high resolution permits precise mapping and characterization of tandem repeat duplications, as shown here in case 1 (*BRCA1* Dup Ex 13). aCGH can be used to determine the presence and size of duplications, but not the exact location and orientation of tandem repeat duplications. In PCR-based techniques such as MLPA, duplications are considered to be present when the ratio between the number of duplicated exons in the sample carrying a mutation and the number of exons in the control sample is at least 1.5, reflecting the presence of 3 copies of a specific exon in the mutated sample and 2 copies in the wild-type sample. The ratio of 1.5 is difficult to demonstrate unambiguously by MLPA, which often gives false-positive signals, as observed in case 1 (*BRCA1* Dup Ex 13). The limits of MLPA have been underlined in several recent studies (Cavalieri et al., 2008), (Staaf et al., 2008). MLPA is limited to coding sequences and can also give false-negative scores, due to the restricted coverage of the 21 probes (Cavalieri et al., 2008). In addition, MLPA provides only limited information on the location of deletion or duplication breakpoints in the usually very large intronic or affected flanking regions, thus necessitating laborious mapping for sequence characterization of the rearrangements. Staaf et al recently suggested that MLPA should be regarded as a screening tool that needs to be complemented by other means of mutation characterization, such as a CGH (Staaf et al., 2008). We propose Molecular Combing as such a replacement technology for MLPA or aCGH, as it unambiguously identifies and visualizes duplications.

Another advantage of Molecular Combing as disclosed herein was its capacity to cover non-coding regions, including the 5' region of the *BRCA1* gene and the genomic region upstream of *BRCA1* that comprises the *NBR2*gene, the ψ*BRCA1* pseudogene and the *NBR1* gene. Recent studies show that it is very difficult to design exploitable PCR or aCGH probes in this rearrangement-prone genomic region (Rouleau et al., 2007), (Staaf et al., 2008), because of the presence of duplicated regions and the high density of Alu repeats. Genomic rearrangements typically arise from unequal homologous recombination between short interspersed nuclear elements (SINEs), including Alu repeats, long interspersed nuclear elements (LINEs), or simple repeat sequences.

Molecular Combing permits precise physical mapping within this difficult regions, as shown here in cases three and two (*BRCA1* Del Ex 2), where we measured mutation sizes of 38.5 kb and 37.1 kb, respectively. As cases 3 and 2 belong to the same family, the detected mutation was the same in both cases, as confirmed by aCGH (Rouleau et al., 2007). The measurement difference of 1.4 kb between these two cases is acceptable, being within the 1-2 kb definition range of the molecular combing assay. The mutation was originally described by Puget et al, who determined the mutation size (37 kb) with a first-generation molecular combing "color bar coding" screening method (Puget et al., 2002). Size estimated with aCGH was in the 40.4 - 58.1 kb range, because of the low density of exploitable oligonucleotide sequences in this genomic region and the reduced sensitivity of 22 some oligonucleotides due to sequence homology (Rouleau et al., 2007). Molecular combing can therefore be used for the analysis of hard-to-sequence genomic regions that contain large numbers of repetitive elements. Here we demonstrate that the high concentration of Alu sequences in *BRCA1* does not represent an obstacle for molecular combing.

### Detection of previously uncharacterized BRCA1 large rearrangements in breast cancer patients

Further samples were tested, and we characterized by Molecular Combing rearrangements which other techniques had failed to accurately describe. One such example is detailed below.

### Triplication of exons 1a, 1b and 2 of BRCA1 and a portion of NBR2.

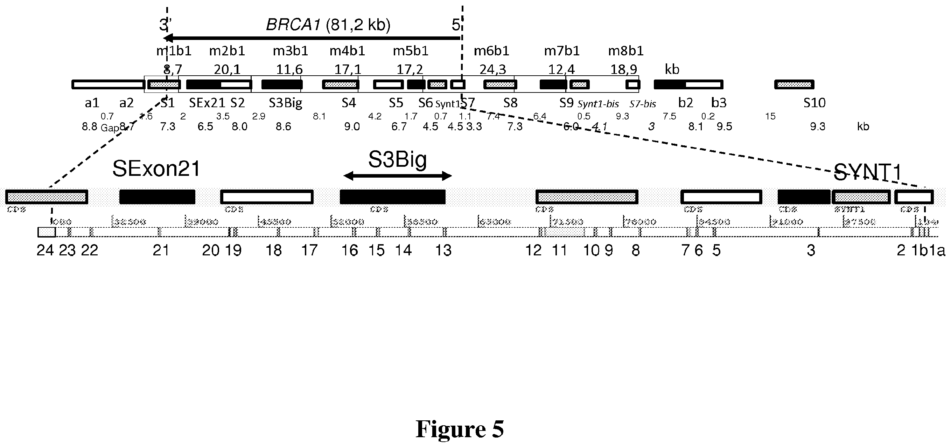

We analyzed sample #7 (provided by the Institut Claudius Régaud, Toulouse, France) by Molecular Combing, using the set of probes described in Figure 5. By visual inspection, two alleles of the BRCA1 gene were identified, differing in the length of the motif g7b1 which extends from the end of the S9B1 probe to the opposite end of the S11B1 probe. The mutation appears to be a triplication involving portions of the SYNT1 probe (SEQ ID 133) and the S10B1 probe, as was confirmed in probe color swapping experiments. This triplication of a DNA segment with a size comprised between 5 and 10 kb involves exons 1a, 1b and 2 of the BRCA1 gene and possibly part of the 5' extremity of the NBR2 gene.

Such a triplication has not been reported in this genomic region yet. This may be due to the previous lack of relevant technologies to detect the mutation. Therefore, we designed tests specific to this mutation. These tests may be used to screen for this triplication or to confirm this triplication in samples where a rearrangement is suspected in this region. There are several types of possible tests, such as PCR, quantitative PCR (qPCR), MLPA, aCGH, sequencing...

Results of quantification techniques, which provide a number of copies of a given sequence (qPCR, MLPA, aCGH, ...) will not provide direct assessment of the tandem nature of the additional copies of the sequence. The triplication reported here may be suspected when sequences within exons 1a, 1b and/or 2 of BRCA1 and/or the sequences between these exons are present in multiple (more than two per diploid genome) copies. Generally speaking, when these results are above the threshold determined for duplicated sequence (which have three copies in total of the duplicated sequence), the sample should be suspected to bear a triplication on a single allele (rather than duplications of the sequence in two separate alleles. Confirmation of the triplication and its tandem nature may be obtained either through a PCR test or through a Molecular Combing test as described in this and the examples section.

As this is a more direct method, we detail some PCR designs here, in the example sections. The man skilled in the art may adapt these tests through common, generally known, molecular biology methods, e.g. by modifying primer locations within the sequence ranges mentioned, and / or modifying experimental conditions (annealing temperature, elongation time, ... for PCR). Also, these tests may be included in "multiplex" tests where other mutations are also sought. For example, one or several pair(s) of primers designed to detect the triplication and described below may be used simultaneously with one or several other pair(s) of primers targeting distinct amplicons. In addition to these adaptations, several common variants exist for the molecular tests described. Nevertheless, these variants remain functionally identical to the described tests and the adaptation of our designs to these variants is easily achievable by the man skilled in the art. For example, sequencing may be replaced by targeted resequencing, where the region of interest is isolated for other genomic regions before the sequencing step, so as to increase coverage in the region of interest. As another example, semi-quantitative PCR, where DNA is quantity after amplification is assessed by common agarose electrophoresis, may replace QMPSF.

These results demonstrate that the developed Molecular Combing platform is a valuable tool for genetic screening of tandem repeat duplications, CNVs, and other complex rearrangements in *BRCA1* and *BRCA2,* such as translocations and inversions, particularly in high-risk breast cancer families.

A prominent application of the developed molecular diagnostic tool is as a predictive genetic test. However, the methods and tools disclosed herein may be applied as or in a companion diagnostic test, for instance, for the screening of BRCA-mutated cells in the context of the development of PARP inhibitors. Such a genetic test can be applied not only to clinical blood samples, but also to circulating cells and heterogeneous cell populations, such as tumor tissues.

### EXAMPLES

### Example 1

### Materials and methods

### Preliminary patient screening

The Genomic Morse Code was validated on 10 samples from patients with no deleterious mutations detected in *BRCA1* or *BRCA2* (control patients). The genetic test was validated on 6 samples from patients with positive family history of breast cancer and known to bear large rearrangements affecting either *BRCA1* or *BRCA2.* Total human genomic DNA was obtained from EBV-immortalized lymphoblastoid cell lines. Preliminary screening for large rearrangements was performed with the QMPSF assay (Quantitative Multiplex PCR of Short Fluorescent Fragments) in the conditions described by Casilli et al and Tournier et al (Casilli et al., 2002) or by means of MLPA (Multiplex Ligation-Dependent Probe Amplification) using the SALSA MLPA kits P002 (MRC Holland, Amsterdam, The Netherlands) for *BRCA1* and P045 (MRC-Holland) for *BRCA2.* All 16 patients gave their written consent for *BRCA1* and *BRCA2* analysis.

### Molecular Combing

### Sample preparation

Total human genomic DNA was obtained from EBV-immortalized lymphoblastoid cell lines. A 45-µL suspension of 10⁶ cells in PBS was mixed with an equal volume of 1.2% Nusieve GTG agarose (Lonza, Basel, Switzerland) prepared in 1x PBS, previously equilibrated at 50°C. The plugs were left to solidify for 30 min at 4°C, then cell membranes are solubilised and proteins digested by an overnight incubation at 50°C in 250 µL of 0.5 M EDTA pH 8.0, 1% Sarkosyl (Sigma-Aldrich, Saint Louis, MO, USA) and 2mg/mL proteinase K (Eurobio, Les Ulis, France), and the plugs were washed three times at room temperature in 10 mM Tris, 1 mM EDTA pH 8.0. The plugs were then either stored at 4°C in 0.5 M7EDTA pH 8.0 or used immediately. Stored plugs were washed three times for 30 minutes in 10 mM Tris, 1 mM EDTA pH 8.0 prior to use.

### Probe preparation

All *BRCA1* and *BRCA2* probes were cloned into pCR2.1-Topo or pCR-XL-Topo (Invitrogen) plasmids by TOPO cloning, using PCR amplicons as inserts. Amplicons were obtained using bacterial artificial chromosomes (BACs) as template DNA. The following BACs were used: for *BRCA1,* the 207-kb BACRP11-831F13 (ch17: 41172482-41379594, InVitrogen, USA); and for *BRCA2,* the 172-kb BAC RP11-486O17 (ch13: 32858070-33030569, InVitrogen, USA). See Tables 1 and 2 for primer sequences and probe coordinates. Primer sequences are referenced as SEQ ID 1 to SEQ ID 130. In some cases (as detailed in table 1), additional artificial sequences were added to the 5' end of the primer for ease of cloning. These artificial sequences are SEQ ID 134 (ForwardPrimerPrefix) for forward primers and SEQ ID 135 (ReversePrimerPrefix) for forward primers, both containing a poly-A and a restriction site for, respectively, AscI and PacI.
SEQ ID 131 (BRCA1-1A), SEQ ID 132 (BRCA1-1B) and SEQ ID 133 (BRCA1 - SYNT1) are are examples of probe sequences.

Whole plasmids were used as templates for probe labeling by random priming. Briefly, for biotin (Biota) labeling, 200 ng of template was labeled with the DNA Bioprime kit (Invitrogen) following the manufacturers instructions, in an overnight labeling reaction. For Alexa-488 (A488) or digoxigenin (Dig) labeling, the same kit and protocol were used, but the dNTP mixture was modified to include the relevant labeled dNTP, namely Dig-11-dUTP (Roche Diagnostics, Meylan, France) or A488-7-OBEA dCTP (Invitrogen) and its unlabelled equivalent, both at 100 µM, and all other dNTPs at 200 µM. Labeled probes were stored at -20°C. For each coverslip, 5 µL of each labeled probe (1/10th of a labeling reaction product) was mixed with 10 µg of human Cot-1 and 10 µg of herring sperm DNA (both from Invitrogen) and precipitated in ethanol. The pellet was then resuspended in 22 µL of 50% formamide, 30% Blocking Aid (Invitrogen), 1X SSC, 2.5% Sarkosyl, 0.25% SDS, and 5 mM NaCl.

### Genomic DNA combing and probe hybridization

Genomic DNA was stained by 1h incubation in 40 mM Tris, 2 mM EDTA containing 3 µM Yoyo-1(Invitrogen, Carlsbad, CA, USA) in the dark at room temperature. The plug was then transferred to 1 mL of 0.5 M MES pH 5.5, incubated at 68°C for 20 min to melt the agarose, and then incubated at 42°C overnight with 1.5 U beta agarase I (New England Biolabs, Ipswich, MA, USA). The solution was transferred to a combing vessel already containing 1ml of 0.5 M MES pH 5.5, and DNA combing was performed with the Molecular Combing System on dedicated coverslips (Combicoverslips) (both from Genomic Vision, Paris, France).

Combicoverslips with combed DNA are then baked for 4 h at 60°C.The coverslips were either stored at -20°C or used immediately for hybridisation. The quality of combing (linearity and density of DNA molecules) was estimated under an epi-fluorescence microscope equipped with an FITC filter set and a 40 x air objective. A freshly combed coverslip is mounted in20µL of a 1ml ProLong-gold solution containing 1µL of Yoyo-1 solution (both from Invitrogen). Prior to hybridisation, the coverslips were dehydrated by successive 3 minutes incubations in 70%, 90% and 100% ethanol baths and then air-dried for 10 min at room temperature. The probe mix (20 µL; see Probe Preparation) was spread on the coverslip, and then left to denature for 5 min at 90°C and to hybridise overnight at 37°C in a hybridizer (Dako). The coverslip was washed three times for 5 min in 50%formamide, 1X SSC, then 3 x 3 min in 2X SSC.

Detection was performed with two or three successive layers of flurophore or streptavidin -conjugated antibodies, depending on the modified nucleotide employed in the random priming reaction (see above). For the detection of biotin labeled probes the antibodies used were Streptavidin-A594 (InVitrogen, Molecular Probes) for the 1st and 3rd layer, biotinylated goat anti-Streptavidin (Vector Laboratories) for the 2nd layer; For the detection of A488-labelled probes the antibodies used were rabbit anti-A488 (InVitrogen, Molecular Probes) for the 1st and goat anti-rabbit A488 (InVitrogen, Molecular Probes) for the 2nd layer; For the detection of digoxygenin labeled probes the antibodies used were mouse anti-Dig (Jackson Immunoresearch) for the 1st layer, ratanti-mouse AMCA (Jackson Immunoresearch) for the 2nd layer and goat anti-mouse A350 (InVitrogen, Molecular Probes) for the 3rd Layer.

A 20 minute incubation step was performed at 37°C in a humid chamber for each layer, and three successive 3 minutes washes in 2X SSC, 0.1% Tween at room temperature between layers. Three additional 3 minutes washes in PBS and dehydration by successive 3 minutes washes in 70%, 90% and 100% ethanol were performed before mounting the coverslip.

### Image acquisition

Image acquisition was performed with a customized automated fluorescence microscope (Image Xpress Micro, Molecular Devices, Sunnyvale, CA, USA) at 40x magnification, and image analysis and signal measurement were performed with the software ImageJ (http://_rsbweb.nih.gov/ij) and JMeasure (Genomic Vision, Paris, France). Hybridisation signals corresponding to the *BRCA1* and *BRCA2* probes were selected by an operator on the basis of specific patterns made by the succession of probes. For all motifs signals belonging to the same DNA fibre, the operator set the ends of the segment and determined its identity and length (kb), on a 1:1 scale image. The data were then output as a spreadsheet. In the final analysis, only intact motif signals were considered, confirming that no fibre breakage had occurred within the *BRCA1* or *BRCA2* motifs.

### Statistical analysis

Molecular Combing allows DNA molecules to be stretched uniformly with a physical distance to contour length correlation of 1 µm, equivalent to 2 kb (Michalet et al., 1997). As a consequence, in the absence of large rearrangements, the derived stretching factor (*SF*) has a value close to 2 kb / µm (±0.2).

All 7 *BRCA1* motifs (g1b1-g7b1) and all 5 *BRCA2* motifs (glb2-g5b2) were measured in all 20 biological samples. The mean value size of all motifs measured in the 10 healthy controls, including the associated statistical analysis, is reported in Table S1. The size of all motifs measured in the 6 breast cancer patients, including the associated statistical analysis, is reported in Table S2. For each motif, the following values were determined: the number of measured images (*n*), the theoretical calculated length (*calculated* (kb)), the mean measured length (*µ*(kb)), the standard deviation (*SD*(kb)), the coefficient of variation (*CV*(%)), the difference between *µ* and *calculated* (*delta*), and the stretching factor (*SF*=*(calculated*/*µ) x 2*) (Michalet et al., 1997). In the absence of mutations, *delta* values are comprised between -1.9 kb and 1.9 kb, and *SF* values are comprised between 1.8 and 2.2. The presence of a large rearrangement on *BRCA1* or *BRCA2* was first identified by visual inspection of the corresponding GMC. From numerous datasets, we established that in the presence of large rearrangements in both *BRCA1* and *BRCA2, delta* ≥ 2 kb (for duplications) or *delta* ≤ -2 kb (for deletions), and the corresponding *SF* ≥ 2.3 kb / µm (for deletions) or *SF* ≤ 1.7 kb / µm (for duplications). To confirm the presence of a large rearrangement, the motif (-s) of interest was (were) first measured on a total population of images (typically between 20 and 40), comprising wild-type (wt) and mutated (*mt*) alleles. In presence of large rearrangements, and aiming to measure the mutation size, the images were then divided in two groups, corresponding to the *wt* and the *mt* alleles. Within each of the two groups of *n* images, following values were calculated: *µ*(kb), *SD* (kb), *CV*(%). The *µ* value of the wild-type allele was then compared with the *µ* value of the mutated allele. To this aim, we calculated the standard error of the mean (*SEM* = *SD*/√*n*) and the 95% confidence interval (*95% CI* = *µ* ± *2x SEM*)*.* The mutation size was then calculated as a difference between the mean size of the two alleles: *mutation size* = *µ*(*BRCA1^{mt}*) - *µ*(*BRCA1^{wt}*)*.* The related *error* was calculated according to following formula: $e r r o r = \left(\left(\left({μ}^{m t} + 2 x S E {M}^{m t}\right) - \left({μ}^{w t} - 2 x S E {M}^{w t}\right)\right) - \left(\left({μ}^{m t} - 2 x S E {M}^{m t}\right) - \left({μ}^{w t} + 2 x S E {M}^{w t}\right)\right)\right) / 2 .$

### Example 2: Comparison of Genetic Morse Code and Molecular Combing of the Invention to Prior Color Bar Code Procedure.

### Part 1. Previous application of Molecular Combing on characterization of BRCA1 and BRCA2 large rearrangements: design of low resolution Color Bar Codes (CBCs)

Molecular Combing has already been used by Gad et al. (Gad GenChrCan 2001, Gad JMG 2002) to detect large rearrangements in the *BRCA1* and *BRCA2* genes. The hybridization DNA probes originally used were part of a low resolution "color bar coding" screening approach composed of cosmids, PACs and long-range PCR products. Some probes were small and ranged from 6 to 10 kb, covering a small fraction the *BRCA1* and *BRCA2* loci. Other probes were very big (PAC 103014 measuring 120 kb for *BRCA1* and BAC 486017 measuring 180 kb for *BRCA2*) and were covering the whole loci, including all the repetitive sequences. Thus, no bioinformatic analysis to identify potentially disturbing repetitive sequences has been even performed. More importantly, no repetitive sequence has been ever excluded from the design of the CBCs. This often resulted in incomplete characterizations of the screened mutations (see Part 3). As a consequence, detection of the probes often resulted in the superposition of individual colored signals (*e.g*., yellow/white spots resulting from superposition of different colored signals) and in strong background noise, undermining the quality of the images and preventing the development of a robust strategy to measure the signals length. In addition, no DNA probe was r isolated and cloned in an insert vector. The *BRCA1* Color Bare Code (CBC) was composed of only 7 DNA probes ((Gad, et al, Genes Chromosomes and cancer 31:75-84 (2001))), whereas the *BRCA2* CBC was composed of only 8 DNA probes (Gad, et al, J Med Genet (2002)). This low number of DNA probes did not allow high resolution physical mapping.().

Importantly, such a low resolution screening approach did not allow the unambiguous visualization of complex mutations, such as tandem repeat duplications or triplications. In contrast, full characterization of tandem repeat duplications and triplications is possible with the high-resolution GMC (see Example 1). Moreover, the accurate physical mapping of all the mutated exons was often problematic, requiring additional laborious sequencing experiments. This often resulted in incomplete characterizations of the screened mutations (see Chapter 3).

### Part 2. New application of Molecular Combing on characterization of BRCA1 and BRCA2 large rearrangements: design of high resolution Genomic Morse Codes (GMCs) and development of a genetic test.

An important point of novelty for the present invention is the design and cloning of high-resolution Genomic Morse Codes (GMC) for both *BRCA1* and *BRCA2* genomic regions. The *BRCA1* GMC is composed of 35 DNA probes (Figure 1), whereas the *BRCA2* GMC is composed of 27 DNA probes (Figure 2).
Comparative Figure 1: in-silico generated (top) and microscopy observed (bottom) high resolution *BRCA1* GMC.
Comparative Figure 2: in-silico generated (top) and microscopy observed (bottom) high resolution GMC of *BRCA2.*

35 genomic regions in *BRCA1* and 27 regions in *BRCA2* devoid of repetitive sequences were identified, and were used to design and clone the corresponding DNA hybridization probes. All the details of the employed DNA hybridization probes (name, size, coordinates, color and the nature of the covered exons) are listed above. The cloned DNA probes allow the accurate physical mapping of deleted exons and permit the simultaneous detection of large rearrangements in *BRCA1* and *BRCA2.* The above described improvement in resolution, permitted the inventors to translate their observations into the development of a robust predictive genetic test for breast and ovarian cancer (see example 1).

### Part 3: High resolution GMCs allow the unambiguous detection and visualization of complex mutation (e.g.: tandem repeat duplications and triplications) that can't be characterized by low resolution CBCs

The following are selected examples of complex mutations that could not be characterized (or only partially) by low resolution CBC, but could be precisely and unambiguously characterized by high resolution GMC:

### 3.1 BRCA1 Dup ex 18-20

### CBC:

The image generated by Gad et al (case IC171712 in Figure 1 of Gad et al, Oncogene 2001) has a low resolution and the nature and particularly the identity of the deleted exons cannot be defined by visual inspection. As a consequence, the size of the mutation has not been determined, confirming that the generated images were problematic for measurements.

### GMC : (see Table S2 of Example 1)

By visual inspection, this mutation appears as a tandem duplication of the red signal S5B1. After measurement, the mutation was estimated to have a size of 6.7 ± 1.2 kb, restricted to a portion of the genome that encodes for exons 18 to 20. The estimated mutation size is fully in line with the 8.7 kb reported in the literature (Staaf, 2008). Details on the measurement and statistical analysis can be found in Example 1.
Comparative Figure 3: characterization of the *BRCA1* mutation Dup ex 18-20 via CBC (top) and GMC (bottom).

### 3.2 BRCA1 Del ex 8-13

### CBC:

The image generated by Gad et al (case IC657 in Figure 1 of Gad et al, Oncogene 2001) has a low resolution and the nature of the deleted exons cannot be unambiguously defined by visual inspection. The size of the mutation after measurement was 20.0 ± 9.6 kb, having an important standard deviation.
GMC: (see Fig. 4B, Example 1)

By visual inspection, the mutation clearly appeared as a deletion of the blue signal S7B1, including a large genomic portion between signals S7B1 and S8B1. After measurement, the mutation was estimated to have a size of 20 ± 28 kb, having a smaller error.

### 3.3 BRCA1 Dup ex 13 (6.1 kb)

### CBC:

No microscopy image related to mutation has been ever provided. The estimated mutation size was 5.8 ± 1.8 kb (case IARC3653 in Figure 3 of Gad et al, Oncogene 2001), but is not supported by visual inspection.
GMC: (see Fig. 4A, Example 1)

By visual inspection via Molecular Combing, this mutation appears as a partial tandem duplication of the blue signal S7B1. After measurement, the mutation was estimated to have a size of 6.1 ± 1.6 kb, restricted to a portion of the DNA probe BRCA1-8 that encodes exon 13. The estimated mutation size is fully in line with the 6.1 kb reported in the literature (Puget, 1999), and according to the Breast Cancer Information Core database, this mutation belongs to the 10 most frequent mutations in BRCA1 (Szabo, 2000). Therefore, there is perfect correlation between the images and the measurements, and correlation with values present in literature.

### 3.4 Tandem repeat triplication of exons 1a, 1b and 2 of BRCA1 and a portion of NBR2.

### CBC:

No tandem triplication has been ever reported using the CBC.

### GMC:

By visual inspection via Molecular Combing, two alleles of the BRCA1 gene were identified in a sample provided by the Institut Claudius Regaud, Toulouse, France, differing in the length of the motif g7b1 which extends from the end of the S9B1 probe to the opposite end of the S11B1 probe. The mutation appeared to be a triplication involving portions of the SYNT1 and the S10B1 probe, as confirmed in probe color swapping experiments. This triplication of a DNA segment with a size comprised between 5 and 10 kb, and probably between 6 and 8 kb, involves exons 1a, 1b and 2 of the BRCA1 gene and possibly part of the 5' extremity of the NBR2 gene.

The CBC would have at best detected this mutation as an increase of the length of a single probe, and thus would not have been able to characterize the mutation as a tandem triplication. Contrarily to Molecular Combing, none of the current molecular diagnostics technology, such as MLPA or aCGH, could assess whether the duplication or triplication is in tandem (within *BRCA1*) or dispersed (out of *BRCA1*). This observation makes a clear difference in terms of risk evaluation, since there is no evidence that repeated genomic portions out of the *BRCA1* locus are clinically significant. Molecular Combing highlights that the mutation occurs within the *BRCA1* gene, thus being of clinical significance.

The following important advantages of GMC compared to CBC are evident from the examples above:
- high resolution visual inspection
- precise mapping of mutated exons
- precise measurement of mutation size with robust statistics
- simultaneous detection of *BRCA1* and *BRCA2*
- detection of inversions and translocation
- absence of disturbing repetitive sequence (Alu sequences) for GMCs *BRCA1* and *BRCA2.*

### Tests specific to detect a triplication in the 5' region of BRCA1

PCR tests to detect unambiguously the triplication described above or a close triplication may distinguish non triplicated from triplicated alleles through either one of two ways:
a- appearance of PCR fragments with the triplicated allele that do not appear with a non-triplicated allele or;
b- change of size of a PCR fragment.

The organization of the sequences in a triplication may be used to design primer pairs such that the PCR amplification is only possible in a tandem repeat. If one of the primers is located in the amplified sequence and is in the same orientation as the BRCA gene (5' to 3') and the other is the reverse complementary of a sequence within the amplified sequence located upstream of the first primer (i.e. the direction from the location of the first to the second primer is the same as the direction from the 3' to the 5' end of the BRCA gene), the PCR in a non-mutated sample will not be possible as the orientation of the primers do not allow it. Conversely, in a triplicated sample, the first primer hybridizing on a repeat unit is oriented correctly relative to the second primer hybridizing in the repeat unit immediately downstream of the first primer's repeat unit. Thus, the PCR is possible. In a triplicated sample, two PCR fragments should be obtained using a pair of primers designed this way. In a sample with a duplication, only one fragment would appear. The size of the smaller PCR fragment(or the only fragment in the case of a duplication), s, is the sum of the following distances:
D, measured from the first (downstream) primer to the downstream (3' direction relative to the BRCA1 gene) breakpoint, and
U, measured from the second (upstream) primer to the upstream (5' direction relative to the BRCA1 gene) breakpoint.

This measurement thus provides a location range for both breakpoints, the downstream breakpoint being at a distance smaller than or equal to s from the location of the downstream primer (in the downstream direction) and the upstream breakpoint at a distance smaller than or equal to s from the location of the upstream primer (in the upstream direction). Besides, since the size of the triplicated sequence (L) is the sum of U+D and the distance between the two primers, L may be readily deduced from the size of the PCR fragment.

The size of the larger fragment is the sum of L and the size of the smaller fragment. Thus, by substracting the size of the smaller fragment from the size of the larger one, the size of the triplicated sequence is readily assessable in a second, independent assessment. This reduces the uncertainty on the location of the breakpoints. Thus, a test designed this way will allow a precise characterization of the triplication. Given the location of the triplication identified here, primer pairs used to detect the triplication could include combinations of one or several of the following downstream and upstream primers (the primer designed as the downstream primer is in the direct orientation relative to the BRCA1 gene and while the upstream primer is reverse complementary to the first strand of the BRCA1 gene). In choosing a combination of primers, in addition to the prescriptions below, one must choose the primer locations so the downstream primer is located downstream of the upstream primer:
A downstream primer may be located :
   i) in the region between exons 2 and 3 of BRCA1, preferably at a distance from 2-4 kb from the 3' end of exon 2, more preferably at a distance from 2.5-3 kb from the 3' end of exon 2
   ii) in the region between exons 2 and 3 of BRCA1, within 2 kb from the 3' end of exon 2, preferably within 1.5 kb and more preferably within 1 kb from the 3' end of exon 2
An upstream primer may be located:
   i) in the region between the BRCA1 gene and the NBR2 gene, within 2 kb from exon 1a of BRCA1, preferably within 1.5 kb and more preferably within 1 kb of exon 1a of BRCA1;
   ii) within exon 1a of BRCA 1 or within exon 1b or in the region between exons 1a and 1b;
   iii) in the region between exons 1b and 2, or in exon 2, or in the region between exons 2 and 3.
   An example of such a combination is the primer pair consisting of primers BRCA1-Synt1-R (SEQ ID 126) and BRCA1-A3A-F (SEQ ID 25);
The combinations above are not meant to be exhaustive and the man skilled in the art may well choose other location for the upstream and downstream primers, provided the orientation and relative location of the primers is chosen as described. Several combinations of primers may be used in separate experiments or in a single experiment (in which case all of the "upstream" primers must be located upstream of all of the "downstream" primers. If more than three primers are used simultaneously (multiplex PCR°, the number of PCR fragments obtained will vary depending on the exact location of the breakpoint (no PCR fragment at all will appear in non mutated samples) and the characterization of the mutation will be difficult. Therefore, it is advisable to perform additional experiments with separate primer pairs if at least one fragment is observed in the multiplex PCR.

Importantly, with the design described in the preceeding paragraphs, the orientation of the triplicated sequence is of minor importance: indeed, in a triplication, at least two of the repeat units will share the same orientation and at least one PCR fragments should be amplified. This holds true for a duplication, as in the case of an inverted repeat, a PCR fragment would be obtained from a one of the primers hybridizing in two separate locations with reverse (facing) orientations, while a direct tandem repeat would generate a PCR fragment from the two primers as described above.

Another type of PCR test to reveal the triplication and its tandem nature requires the amplification of a fraction of or of the entire repeat array, using primer pairs spanning the repeated sequence (both primers remaining outside the amplified sequence), or spanning a breakpoint (one primer is within and the other outside the amplified sequence) or entirely included in the amplified sequence. These tests will generate a PCR fragment of given size in a normal sample, while in a sample with a triplication on one allele, one or more additional PCR fragment will appear, including one the size of the "normal" fragment plus twice the size of the repeat sequence. If a mutation is present, these tests will often lead to results than can have several interpretations. If a single experiment is performed and reveals a mutation, a (series of) complementary test(s) may be performed following the designs presented herein to confirm the correct interpretation. Given the location of the triplication identified here, primer pairs used to detect the triplication could include a combination of one or several of of the following primers, with at least one down stream and one upstream primer. The primer designed as the downstream primer is reverse complementary relative to the BRCA1 gene sequence and while the upstream primer is in direct orientation relative to the BRCA1 gene. In choosing a combination of primers, in addition to the prescriptions below, one must choose the primer locations so the downstream primer is located downstream of the upstream primer:
A downstream primer may be located :
   i) in exon 3 of the BRCA1 gene; or
   ii) in the region between exons 2 and 3 of BRCA1, preferably more than 2 kb and less than 10 kb from the 3' end of exon 2, more preferably more than 3 kb and less than 8 kb and even more preferably more than 4 kb and less than 6 kb from the 3' end of exon 2.
An upstream primer may be located:
   i) in the region between the BRCA1 gene and the NBR2 gene, less than 10 kb from exon 1a of BRCA1 and more than 1 kb from exon 1a of BRCA1, preferably more less than 8 kb than 2 kb and more preferably less than 6 and more than 4 kb of exon 1a of BRCA1; or
   ii) in exon 1a, exon 1b or in the region between exons 1a and 1b of BRCA1; or
   iii) in exon 2 or in the region between exons 1b and 2 of BRCA1 or in the region between exons 2 and 3.
   iii)
   iv) Examples of such combinations are the primer pairs consisting of primers BRCA1-A3A-F (SEQ ID 25) and BRCA1-A3A-R (SEQ ID 26) and of primers BRCA1-Synt1-F (SEQ ID 125) and BRCA1-Synt1-R (SEQ ID 126)
   v) a downstream primer as described in i) and an upstream primer as described in ii)
   vi) a dowstream primer as described in i) and an upstream primer as described in iii)
   vii) a dowstream primer as described in ii) and an upstream primer as described in i)

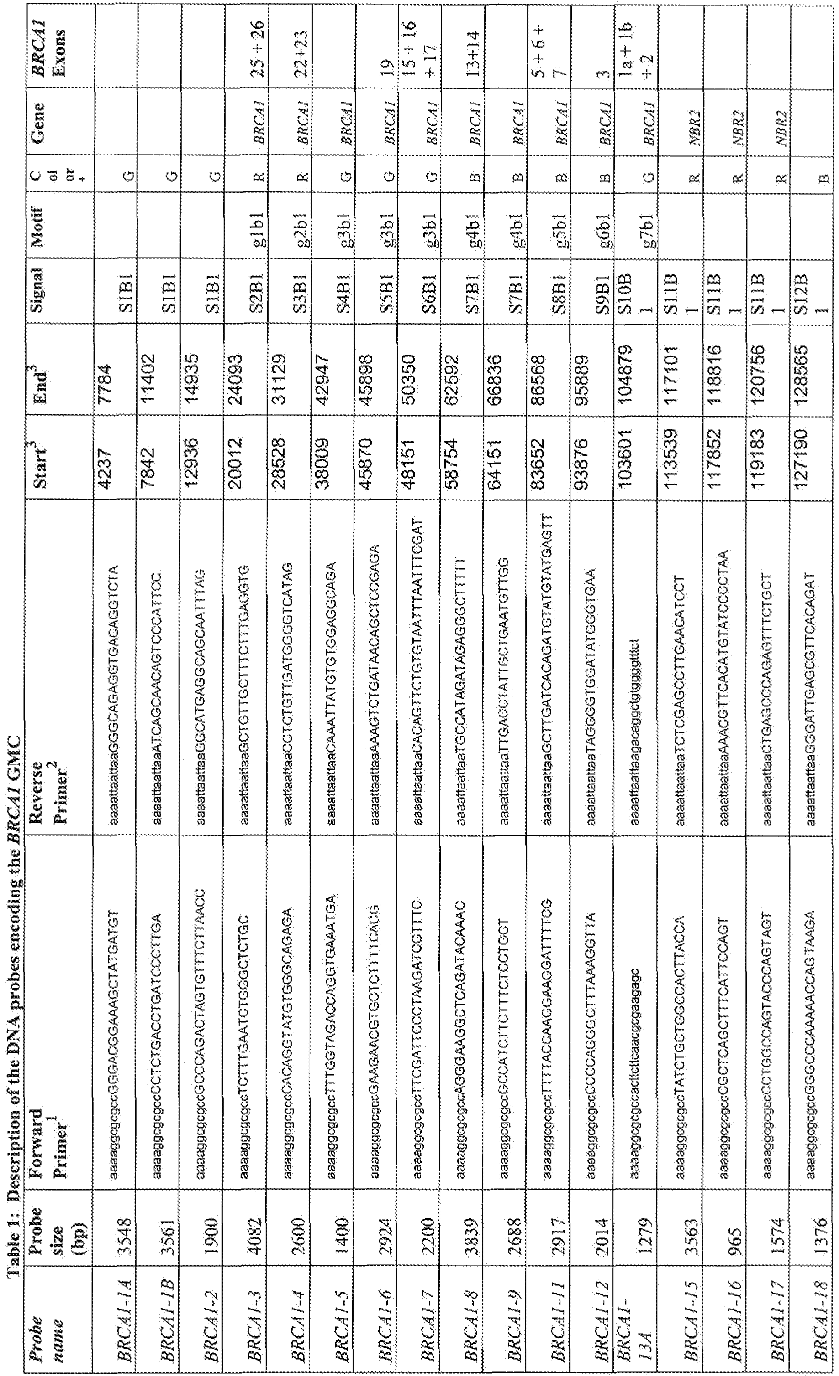

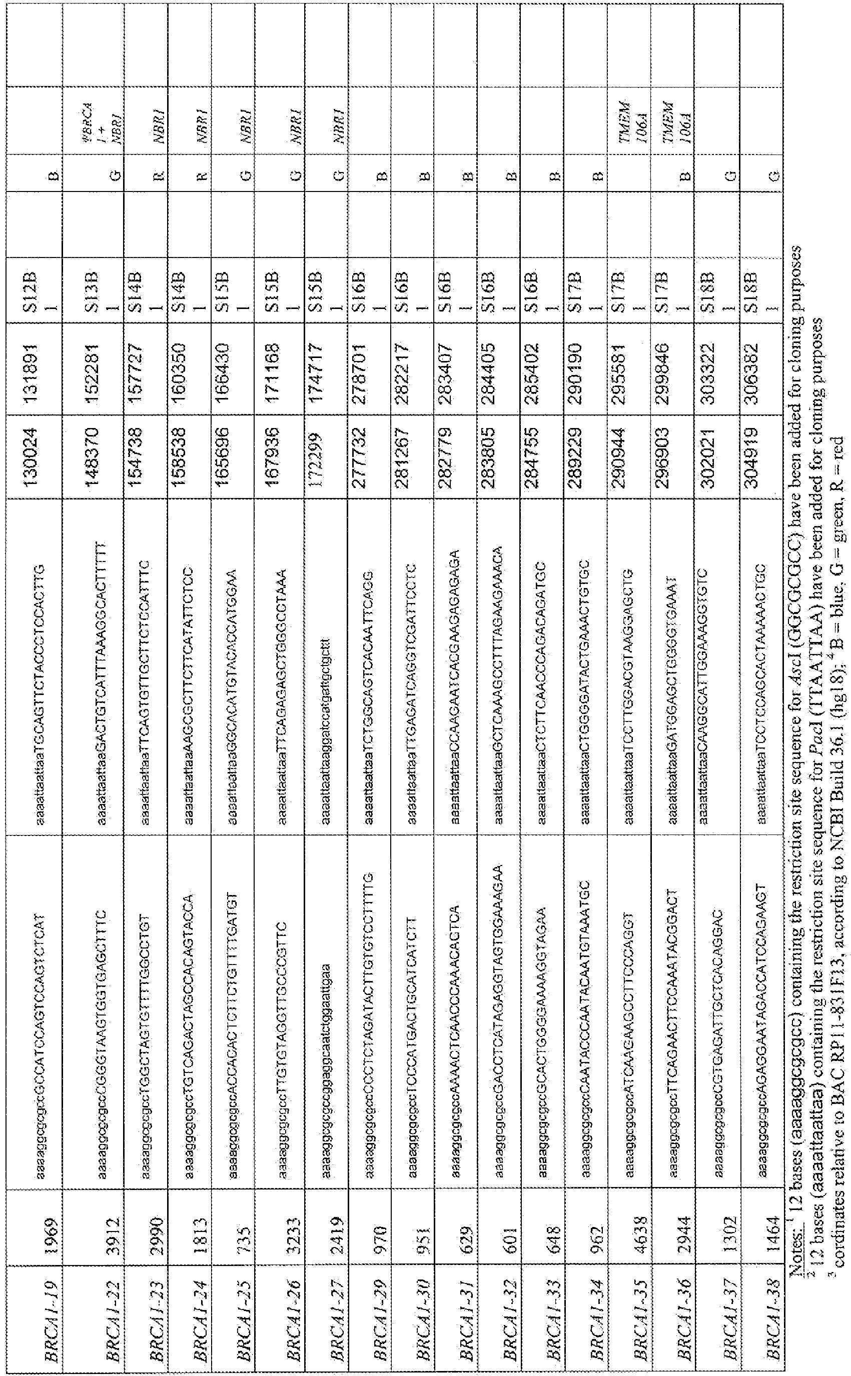

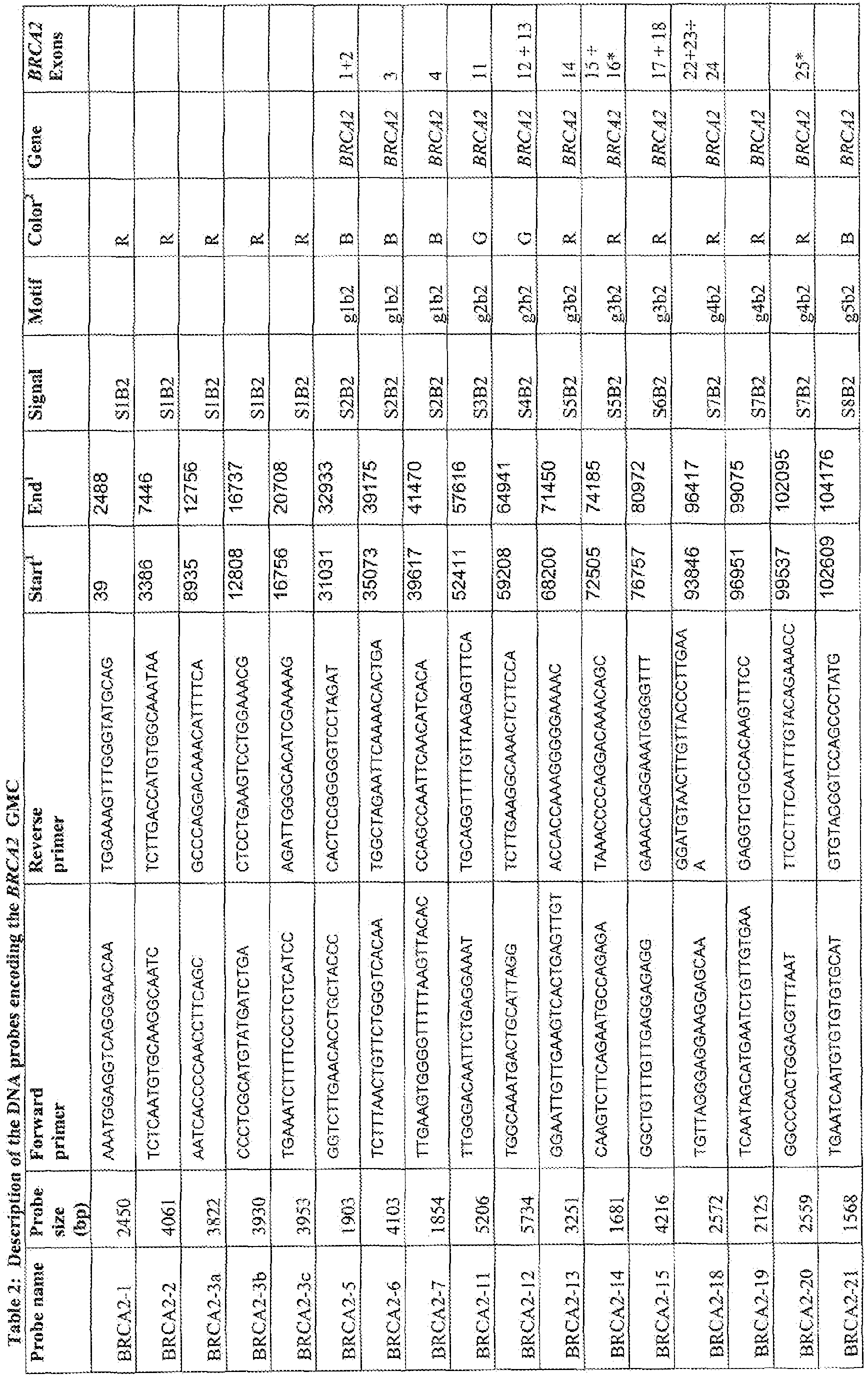

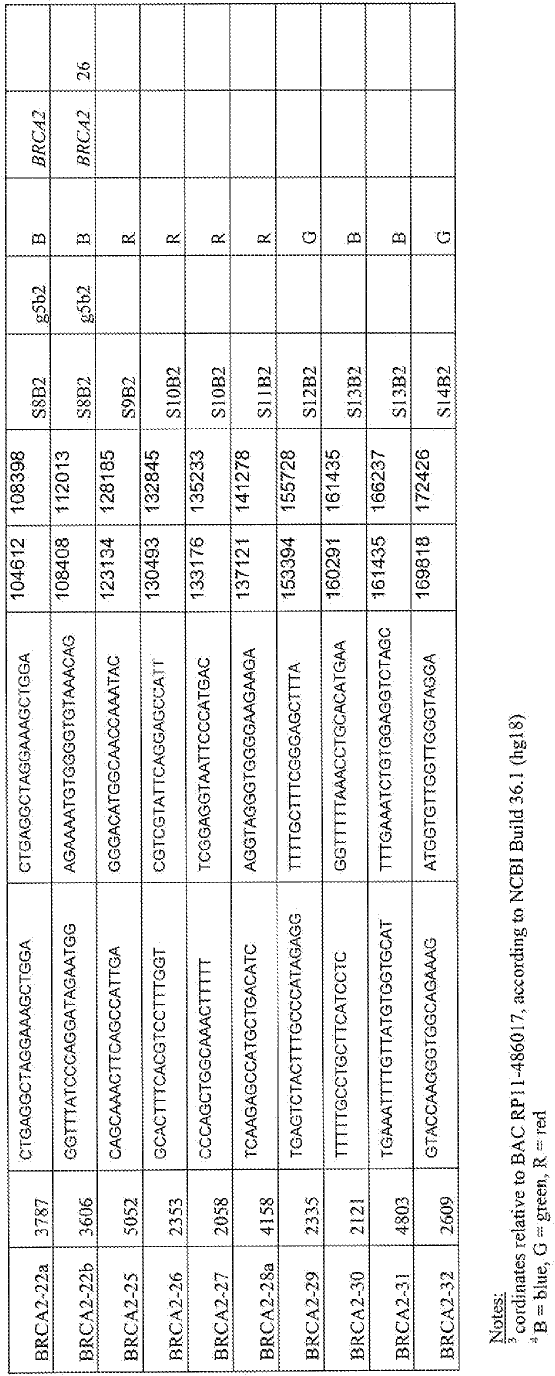

**Table 3**

| | | | | | | | | | | | | | | | |
|---|---|---|---|---|---|---|---|---|---|---|---|---|---|---|---|
| Total Alu sequences in probes | | | | | 30 | (10 %) | | | | | | | | | |
| Total Alu sequences in exlcuded regions | | | | | 270 | (90 %) | | | | | | | | | |

| | **score** | % **div.** | % **del.** | % **ins.** | **position in query sequence (hg18)** | | | **+** | **matching repeat** | **repeat class/family** | **position in repeat** | | | **linkage id** | **Alu seq** (count) |
|---|---|---|---|---|---|---|---|---|---|---|---|---|---|---|---|
| | | | | | | | | | | | (left) begin | end end | begin (left) | | |
| | | | | | begin | end | (left) | | | | | | | | |
| excluded region **1** | 2519 | 7.1 | 1.0 | 0.0 | 132 | 441 | 308672 | + | AluSp | SINE/Alu | 1 | 313 | 0 | 1 | 7 |
| | 25 | 72.0 | 0.0 | 0.0 | 1136 | 1160 | 307953 | + | AT_rich | Low_Cplxty | 1 | 25 | 0 | 2 | |
| | 22 | 58.3 | 0.0 | 0.0 | 1627 | 1662 | 307451 | + | GC_rich | Low_Cplxty | 1 | 36 | 0 | 3 | |
| | 223 | 19.3 | 3.5 | 0.0 | 1708 | 1764 | - 307349 | + | (CGG)n | Simple | 2 | 60 | 0 | 4 | |
| | 21 | 57.1 | 0.0 | 0.0 | 1959 | 1986 | 307127 | + | GC_rich | Low_Cplxty | 1 | 28 | 0 | 5 | |
| | 2280 | 7.5 | 2.7 | 0.7 | 2142 | 2434 | 306679 | + | AluSz | SINE/Alu | 1 | 299 | -13 | 6 | |
| | 2216 | 10.4 | 0.0 | 1.4 | 2436 | 2733 | 306380 | + | AluSx1 | SINE/Alu | 1 | 294 | -18 | 7 | |
| | 2480 | 4.4 | 2.0 | 0.3 | 2734 | 3026 | 306087 | + | AluY | SINE/Alu | 1 | 298 | -13 | 8 | |
| | 1117 | 15.8 | 0.6 | 0.0 | 3305 | 3475 | 305638 | C | AluJr | SINE/Alu | -11 | 301 | 130 | 9 | |
| | 364 | 13.5 | 0.0 | 0.0 | 3482 | 3533 | 305580 | C | MER66A | LTR/ERV1 | -140 | 338 | 287 | 10 | |
| | 749 | 11.9 | 5.9 | 0.8 | 3557 | 3674 | 305439 | C | AluJr | SINE/Alu | -187 | 125 | 2 | 9 | |
| | 1741 | 6.0 | 17. 9 | 1.0 | 3746 | 3996 | 305117 | C | AluY | SINE/Alu | -18 | 293 | 1 | 11 | |
| **probe 1A** | 273 | 26.3 | 2.9 | 0.8 | 4677 | 4880 | 304233 | + | G-rich | Low_Cplxty | 1 | 208 | 0 | 12 | 1 |
| | 22 | 40.9 | 0.0 | 0.0 | 5327 | 5348 | 303765 | + | GC_rich | Low_Cplxty | 1 | 22 | 0 | 13 | |
| | 2331 | 9.6 | 0.7 | 0.3 | 5904 | 6205 | 302908 | + | AluSx | SINE/Alu | 1 | 303 | -9 | 14 | |
| **excluded region 2** | | | | | | | | | | | | | | | 0 |
| **probe 1B** | 2512 | 6.3 | 0.3 | 3.2 | 9150 | 9467 | 299646 | + | AluY | SINE/Alu | 1 | 309 | -2 | 15 | 2 |
| | 313 | 24.8 | 17.9 | 0.0 | 9930 | 10046 | 299067 | C | L2b | LINE/L2 | 0 | 337 5 | 3238 | 16 | |
| | 374 | 31.1 | 1.9 | 6.6 | 10058 | 10260 | 298853 | C | L2b | LINE/L2 | -179 | 3208 | 3005 | 16 | |
| | 958 | 15.6 | 0.0 | 7.1 | 10508 | 10687 | 298426 | + | FRAM | SINE/Alu | 8 | 175 | -1 | 17 | |
| **excluded region 3** | 1420 | 7.5 | 0.0 | 0.6 | 11598 | 11771 | 297342 | C | AluSc | SINE/Alu | -2 | 307 | 135 | 18 | 7 |
| | 2332 | 8.4 | 0.7 | 0.3 | 11783 | 12078 | 297035 | C | AluSp | SINE/Alu | -16 | 297 | 1 | 19 | |
| | 486 | 10.1 | 0.0 | 15. 1 | 12079 | 12129 | 296984 | C | AluSc | SINE/Alu | -218 | 91 | 47 | 18 | |
| | 1515 | 13.5 | 0.9 | 0.5 | 12130 | 12344 | 296769 | C | AluSx | SINE/Alu | -94 | 218 | 3 | 20 | |
| | 2169 | 8.4 | 1.4 | 1.7 | 12353 | 12507 | 296606 | C | AluY | SINE/Alu | -20 | 291 | 133 | 21 | |
| | 2672 | 4.7 | 0.0 | 0.0 | 12508 | 12807 | -296306 | C | AluY | SINE/Alu | -11 | 300 | 1 | 22 | |
| | 2169 | 8.4 | 1.4 | 1.7 | 12808 | 12941 | 296172 | C | AluY | SINE/Alu | -179 | 132 | 3 | 21 | |
| **probe 2** | 2169 | 8.4 | 1.4 | 1.7 | 12808 | 12941 | 296172 | C | AluY | SINE/Alu | -179 | 132 | 3 | 21 | 2 |
| | 486 | 10.1 | 0.0 | 15.1 | 12942 | 12979 | 296134 | C | AluSc | SINE/Alu | -177 | 132 | 99 | 18 | |
| | 381 | 34.8 | 4.9 | 0.6 | 13095 | 13256 | 295857 | + | MIRc | SINE/MIR | 18 | 186 | -82 | 23 | |
| | 219 | 29.5 | 2.8 | 2.8 | 13304 | 13411 | 295702 | C | L2c | LINE/L2 | -202 | 318 5 | 3078 | 24 | |
| | 449 | 3.2 | 0.0 | 0.0 | 13485 | 13546 | 295567 | + | SVA_E | Other | 1318 | 137 9 | -3 | 25 | |
| | 601 | 28. | 18.6 | 0.0 | 14578 | 14771 | 294342 | + | MIRb | SINE/MIR | 24 | 253 | -15 | 26 | |
| **excluded region 4** | 1845 | 17.3 | 1.6 | 2.3 | 15074 | 15380 | 293733 | + | AluJr | SINE/Alu | 1 | 305 | -7 | 27 | 6 |
| | 1568 | 15.0 | 10.5 | 1.0 | 15388 | 15653 | 293460 | + | AluJb | SINE/Alu | 1 | 291 | -21 | 28 | |
| | 352 | 26.1 | 6.5 | 2.0 | 15654 | 15791 | 293322 | + | MIR3 | SINE/MIR | 35 | 178 | -30 | 29 | |
| | 689 | 11.4 | 0.0 | 0.0 | 16242 | 16346 | 292767 | C | L1MB5 | LINE/L1 | 0 | 617 4 | 6070 | 30 | |
| | 2643 | 5.6 | 0.0 | 0.0 | 16374 | 16678 | 292435 | C | AluY | SINE/Alu | -6 | 305 | 1 | 31 | |
| | 2125 | 10.7 | 3.8 | 0.3 | 16912 | 17200 | 291913 | C | AluSq2 | SINE/Alu | -13 | 299 | 1 | 32 | |
| | 381 | 2.2 | 0.0 | 0.0 | 17660 | 17705 | 291408 | + | (CA)n | Simple | 2 | 47 | 0 | 33 | |
| | 280 | 25.0 | 14.8 | 3.4 | 17883 | 17993 | 291120 | + | MIR3 | SINE/MIR | 44 | 166 | -102 | 34 | |
| | 2337 | 11.2 | 0.0 | 0.3 | 18230 | 18541 | 290572 | + | AluSq2 | SINE/Alu | 1 | 311 | -1 | 35 | |
| | 201 | 35.9 | 0.0 | 11.3 | 18752 | 18908 | 290205 | C | L2c | LINE/L2 | -1 | 338 6 | 3246 | 36 | |
| | 254 | 32.5 | 5.9 | 2.6 | 19294 | 19505 | 289608 | + | L2b | LINE/L2 | 3073 | 328 6 | -89 | 37 | |
| | 217 | 21.9 | 0.0 | 0.0 | 19530 | 19570 | 289543 | + | (CA)n | Simple | 2 | 42 | 0 | 38 | |
| | 2506 | 8.1 | 0.0 | 0.0 | 19616 | 19923 | 289190 | C | AluY | SINE/Alu | -3 | 308 | 1 | 39 | |
| | 639 | 21.8 | 3.1 | 2.2 | 19966 | 20118 | 288995 | + | MIRb | SINE/MIR | 6 | 162 | -106 | 40 | |
| **probe 3** | 639 | 21.8 | 3.1 | 2.2 | 19966 | 20118 | 288995 | + | MIRb | SINE/MIR | 6 | 162 | -106 | 40 | 0 |
| | 1555 | 15.4 | 8.4 | 2.6 | 20654 | 20974 | 288139 | C | MER44A | DNA/TcMT | 0 | 339 | 1 | 41 | |
| | 381 | 16.3 | 15.1 | 7.4 | 21186 | 21311 | - 287802 | C | MER5A | DNA/hAT-Charlie | -54 | 135 | 1 | 42 | |
| | 229 | 22.5 | 6.5 | 4.2 | 21507 | 21599 | 287514 | C | X8_LINE | LINE/CR1 | -29 | 267 | 173 | 43 | |
| | 200 | 38.8 | 3.6 | 2.9 | 22836 | 22973 | 286140 | + | MIR | SINE/MIR | 49 | 187 | -75 | 44 | |
| | 1354 | 22.8 | 13.0 | 2.1 | 23166 | 23655 | 285458 | + | MLT1E2 | LTR/ERVL-MaLR | 2 | 541 | -86 | 45 | |
| | 399 | 20.9 | 0.0 | 6.0 | 23697 | 23808 | 285305 | C | MIR | SINE/MIR | -75 | 193 | 97 | 46 | |
| **excluded region 5** | 2288 | 12.0 | 0.7 | 0.0 | 24330 | 24637 | 284476 | C | AluSxl | SINE/Alu | 0 | 312 | 3 | 47 | 11 |
| | 2339 | 9.7 | 0.3 | 0.3 | 25459 | 25758 | 283355 | C | AluSx | SINE/Alu | -12 | 300 | 1 | 48 | |
| | 1409 | 9.1 | 0.0 | 0.0 | 25759 | 25933 | 283180 | C | AluSq2 | SINE/Alu | -4 | 308 | 134 | 49 | |
| | 1785 | 12.8 | 0.0 | 1.6 | 25934 | 26184 | 282929 | C | AluSx | SINE/Alu | -12 | 300 | 54 | 50 | |
| | 916 | 10.5 | 0.0 | 2.5 | 26186 | 26309 | 282804 | + | AluSx | SINE/Alu | 178 | 298 | -14 | 51 | |
| | 1897 | 16.1 | 0.7 | 1.0 | 26638 | 26936 | 282177 | C | AluJr | SINE/Alu | -14 | 298 | 1 | 52 | |
| | 189 | 21.1 | 13.8 | 7.6 | 27056 | 27142 | 281971 | C | L2a | LINE/L2 | -3 | 342 3 | 3332 | 53 | |
| | 713 | 22.6 | 2.4 | 3.6 | 27280 | 27307 | 281806 | C | AluJb | SINE/Alu | -144 | 168 | 141 | 54 | |
| | 1795 | 13.9 | 7.9 | 0.7 | 27308 | 27587 | 281526 | C | AluJb | SINE/Alu | -12 | 300 | 1 | 55 | |
| | 713 | 22.6 | 2.4 | 3.6 | 27588 | 27728 | 281385 | C | AluJb | SINE/Alu | -172 | 140 | 1 | 54 | |
| | 2417 | 7.8 | 0.0 | 1.7 | 27734 | 28039 | 281074 | C | AluSc | SINE/Alu | -7 | 302 | 2 | 56 | |
| | 2080 | 14.0 | 1.0 | 1.9 | 28040 | 28353 | 280760 | C | AluSz | SINE/Alu | -1 | 311 | 1 | 57 | |
| **probe 4** | 200 | 17.6 | 0.0 | 0.0 | 29069 | 29102 | 280011 | + | C-rich | Low_Cplxty | 146 | 179 | 0 | 58 | 1 |
| | 2386 | 8.5 | 1.3 | 1.6 | 29863 | 30169 | 278944 | + | AluSc8 | SINE/Alu | 1 | 306 | -6 | 59 | |
| **excluded region 6** | 2494 | 7.4 | 0.0 | 0.0 | 31175 | 31470 | 277643 | C | AluSg | SINE/Alu | -14 | 296 | 1 | 60 | 16 |
| | 886 | 20.8 | 3.0 | 0.5 | 31677 | 31814 | 277299 | + | MER3 | DNA/hAT-Charlie | 1 | 142 | -67 | 61 | |
| | 1112 | 16.3 | 0.0 | 1.8 | 31815 | 31980 | 277133 | C | AluJo | SINE/Alu | -13 | 299 | 137 | 62 | |
| | 886 | 20.8 | 3.0 | 0.5 | 31981 | 32044 | 277069 | + | MER3 | DNA/hAT-Charlie | 143 | 207 | -2 | 61 | |
| | 396 | 0.0 | 0.0 | 0.0 | 32317 | 32360 | 276753 | + | (CA)n | Simple | 2 | 45 | 0 | 63 | |
| | 2102 | 9.2 | 0.0 | 0.0 | 32415 | 32675 | 276438 | C | AluSx3 | SINE/Alu | -15 | 297 | 37 | 64 | |
| | 2319 | 9.0 | 0.0 | 1.7 | 32917 | 33217 | 275896 | + | AluY | SINE/Alu | 1 | 296 | -15 | 65 | |
| | 2269 | 10.2 | 2.4 | 0.0 | 33230 | 33524 | 275589 | + | AluSp | SINE/Alu | 1 | 302 | -11 | 66 | |
| | 1969 | 16.6 | 0.0 | 0.3 | 33980 | 34275 | 274838 | C | AluJb | SINE/Alu | -16 | 296 | 2 | 67 | |
| | 2311 | 8.8 | 0.3 | 2.3 | 34281 | 34585 | 274528 | C | AluSq2 | SINE/Alu | -13 | 299 | 1 | 68 | |
| | 199 | 36.4 | 1.5 | 0.0 | 34736 | 34801 | 274312 | + | MIRc | SINE/MIR | 60 | 126 | -142 | 69 | |
| | 809 | 26.0 | 0.7 | 9.3 | 34870 | 34901 | 274212 | + | MIR | SINE/MIR | 5 | 33 | -229 | 70 | |
| | 1727 | 18.2 | 0.0 | 5.9 | 34902 | 35038 | 274075 | + | AluSx | SINE/Alu | 1 | 136 | -176 | 71 | |
| | 1897 | 14.9 | 0.0 | 0.4 | 35039 | 35313 | 273800 | + | AluSx | SINE/Alu | 1 | 274 | -38 | 72 | |
| | 1727 | 18.2 | 0.0 | 5.9 | 35314 | 35496 | 273617 | + | AluSx | SINE/Alu | 137 | 303 | -9 | 71 | |
| | 809 | 26.0 | 0.7 | 9.3 | 35497 | 35710 | 273403 | + | MIR | SINE/MIR | 34 | 230 | -32 | 70 | |
| | 1810 | 17.4 | 1.3 | 1.6 | 35711 | 36014 | 273099 | C | AluJb | SINE/Alu | -9 | 303 | 1 | 73 | |
| | 809 | 26.0 | 0.7 | 9.3 | 36015 | 36046 | 273067 | + | MIR | SINE/MIR | 231 | 262 | 0 | 70 | |
| | 670 | 20.9 | 3.3 | 12.7 | 36048 | 36228 | 272885 | + | FRAM | SINE/Alu | 1 | 166 | 0 | 74 | |
| | 437 | 34.5 | 4.7 | 6.3 | 36250 | 36506 | 272607 | + | MIRb | SINE/MIR | 2 | 254 | -14 | 75 | |
| | 2289 | 9.9 | 0.0 | 3.9 | 36764 | 37086 | 272027 | + | AluSx1 | SINE/Alu | 1 | 311 | -1 | 76 | |
| | 2440 | 4.5 | 0.0 | 1.1 | 37090 | 37406 | 271707 | + | AluY | SINE/Alu | 1 | 311 | 0 | 77 | |
| | 1364 | 10.9 | 0.0 | 0.0 | 37407 | 37581 | 271532 | + | AluSc8 | SINE/Alu | 133 | 307 | -5 | 78 | |
| | 1601 | 18.5 | 0.3 | 4.8 | 37615 | 37916 | 271197 | + | AluJr | SINE/Alu | 2 | 290 | -22 | 79 | |
| **probe 5** | 325 | 27.1 | 8.8 | 10.6 | 38602 | 38717 | 270396 | + | L2c | LINE/L2 | 2331 | 244 6 | -973 | 80 | 1 |
| | 2107 | 10.4 | 0.3 | 3.2 | 38718 | 39005 | 270108 | + | AluSx1 | SINE/Alu | 1 | 280 | -32 | 81 | |
| | 414 | 0.0 | 0.0 | 0.0 | 39006 | 39051 | 270062 | + | (CAA)n | Simple | 3 | 48 | 0 | 82 | |
| | 325 | 27.1 | 8.8 | 10.6 | 39052 | 39115 | 269998 | + | L2c | LINE/L2 | 2447 | 2509 | -910 | 80 | |
| | 218 | 28.1 | 9.7 | 3.2 | 39093 | 39298 | 269815 | + | L2c | LINE/L2 | 2464 | 2682 | -737 | 80 | |
| **excluded region 7** | 218 | 28.1 | 9.7 | 3.2 | 39093 | 39298 | 269815 | + | L2c | LINE/L2 | 2464 | 2682 | -737 | 80 | 9 |
| | 198 | 0.0 | 0.0 | 0.0 | 39435 | 39456 | 269657 | + | (TTA)n | Simple | 2 | 23 | 0 | 83 | |
| | 1165 | 10.7 | 0.0 | 0.0 | 39457 | 39605 | 269508 | C | AluSx | SINE/Alu | -27 | 285 | 137 | 84 | |
| | 1808 | 10.0 | 11. 9 | 1.0 | 39609 | 39877 | 269236 | C | AluSp | SINE/Alu | -15 | 298 | 1 | 85 | |
| | 984 | 11.4 | 0.0 | 0.8 | 39890 | 40020 | 269093 | C | AluSx | SINE/Alu | -179 | 133 | 4 | 84 | |
| | 1982 | 13.2 | 0.3 | 5.6 | 40025 | 40342 | 268771 | C | AluSz | SINE/Alu | -10 | 302 | 1 | 86 | |
| | 2106 | 14.2 | 0.6 | 0.6 | 40380 | 40690 | 268423 | + | AluSz | SINE/Alu | 1 | 311 | -1 | 87 | |
| | 460 | 35.3 | 7.3 | 3.8 | 40691 | 41046 | 268067 | + | L2c | LINE/L2 | 3015 | 3382 | -5 | 80 | |
| | 2297 | 10.7 | 0.0 | 0.7 | 41122 | 41420 | 267693 | C | AluSz | SINE/Alu | -15 | 297 | 1 | 88 | |
| | 205 | 30.4 | 0.0 | 0.0 | 41578 | 41633 | 267480 | + | (TA)n | Simple | 1 | 56 | 0 | 89 | |
| | 1733 | 20.1 | 0.3 | 0.3 | 41635 | 41928 | 267185 | C | AluJr4 | SINE/Alu | -16 | 296 | 3 | 90 | |
| | 2129 | 12.4 | 0.7 | 0.0 | 42139 | 42429 | 266684 | C | AluSx | SINE/Alu | -16 | 296 | 4 | 91 | |
| | 2203 | 10.4 | 1.0 | 0.0 | 42431 | 42719 | 266394 | C | AluSp | SINE/Alu | -15 | 298 | 7 | 92 | |
| **probe 6** | 189 | 0.0 | 0.0 | 0.0 | 44176 | 44196 | 264917 | + | (CAG)n | Simple | 2 | 22 | 0 | 93 | 2 |
| | 2434 | 8.6 | 0.0 | 0.0 | 44364 | 44664 | 264449 | C | AluY | SINE/Alu | -9 | 302 | 2 | 94 | |
| | 2200 | 10.7 | 1.6 | 1.6 | 44923 | 45230 | 263883 | + | AluSp | SINE/Alu | 1 | 308 | -5 | 95 | |
| | 804 | 27.1 | 11.1 | 9.7 | 45271 | 45749 | 263364 | C | L3 | LINE/CR1 | -188 | 3911 | 3427 | 96 | |
| **excluded region 8** | 2148 | 13.0 | 0.3 | 0.0 | 45943 | 46243 | 262870 | C | AluSg | SINE/Alu | -7 | 303 | 2 | 97 | 6 |
| | 2489 | 7.2 | 0.3 | 0.3 | 46349 | 46653 | 262460 | C | AluSq2 | SINE/Alu | -7 | 305 | 1 | 98 | |
| | 2380 | 8.9 | 0.0 | 1.6 | 46776 | 47089 | 262024 | C | AluSc | SINE/Alu | 0 | 309 | 1 | 99 | |
| | 413 | 12.9 | 2.7 | 4.2 | 47300 | 47372 | 261741 | + | L1PA8 | LINE/L1 | 6086 | 615 7 | -15 | 100 | |
| | 436 | 5.8 | 0.0 | 0.0 | 47373 | 47424 | 261689 | C | AluSz6 | SINE/Alu | -12 | 300 | 249 | 101 | |
| | 198 | 0.0 | 0.0 | 0.0 | 47427 | 47448 | 261665 | + | (A)n | Simple | 1 | 22 | 0 | 102 | |
| | 2545 | 6.1 | 0.0 | 0.0 | 47532 | 47826 | -261287 | + | AluY | SINE/Alu | 1 | 295 | -16 | 103 | |
| | 827 | 16.6 | 0.0 | 6.1 | 47965 | 48103 | 261010 | + | FLAM_C | SINE/Alu | 1 | 131 | -12 | 104 | |
| **probe 7** | 2366 | 9.4 | 0.3 | 0.0 | 49470 | 49768 | 259345 | C | AluSp | SINE/Alu | -13 | 300 | 1 | 105 | 1 |
| | 21 | 42.9 | 0.0 | 0.0 | 50235 | 50255 | 258858 | + | AT_rich | Low_Cplxty | 1 | 21 | 0 | 106 | |
| **excluded region 9** | 352 | 36.9 | 5.3 | 1.6 | 50840 | 51026 | 258087 | + | L1M5 | UNE/L1 | 5465 | 565 8 | -584 | 107 | 16 |
| | 307 | 30.7 | 16. 0 | 0.6 | 51006 | 51149 | 257964 | + | L1MC | UNE/L1 | 5649 | 581 4 | 2068 | 108 | |
| | 2314 | 7.3 | 0.0 | 1.8 | 51258 | 51580 | 257533 | + | AluY | SINE/Alu | 1 | 311 | 0 | 109 | |
| | 2432 | 6.5 | 0.0 | 0.3 | 51642 | 51931 | 257182 | + | AluSp | SINE/Alu | 1 | 289 | -24 | 110 | |
| | 1598 | 17.3 | 0.3 | 5.7 | 51946 | 52103 | 257010 | C | AluJb | SINE/Alu | -19 | 293 | 142 | 111 | |
| | 2332 | 9.0 | 0.3 | 1.4 | 52104 | 52403 | 256710 | C | AluSp | SINE/Alu | -16 | 297 | 1 | 112 | |
| | 1569 | 17.0 | 0.3 | 5.7 | 52404 | 52538 | 256575 | C | AluJb | SINE/Alu | -171 | 141 | 15 | 111 | |
| | 754 | 14.3 | 0.9 | 0.0 | 52591 | 52702 | 256411 | + | AluJr | SINE/Alu | 6 | 118 | -194 | 113 | |
| | 198 | 10.3 | 0.0 | 0.0 | 53274 | 53302 | 255811 | + | (TA)n | Simple | 1 | 29 | 0 | 114 | |
| | 2130 | 12.4 | 0.0 | 0.7 | 53303 | 53592 | 255521 | C | AluSx | SINE/Alu | -24 | 288 | 1 | 115 | |
| | 1263 | 13.1 | 1.1 | 0.0 | 54309 | 54483 | 254630 | + | AluSx1 | SINE/Alu | 135 | 311 | -1 | 116 | |
| | 514 | 11.2 | 1.6 | 5.1 | 54497 | 54618 | 254495 | + | GA-rich | Low_Cplxty | 63 | 180 | 0 | 117 | |
| | 210 | 15.2 | 0.0 | 0.0 | 54620 | 54652 | 254461 | + | A-rich | Low_Cplxty | 1 | 33 | 0 | 118 | |
| | 190 | 27.9 | 0.0 | 0.0 | 55008 | 55050 | 254063 | C | L2c | LINE/L2 | -15 | 337 2 | 3330 | 119 | |
| | 1334 | 8.6 | 0.0 | 0.0 | 55101 | 55262 | 253851 | C | AluSxl | SINE/Alu | -14 | 298 | 137 | 120 | |
| | 1447 | 17.3 | 2.4 | 0.8 | 55382 | 55629 | 253484 | + | AluJb | SINE/Alu | 37 | 288 | -24 | 121 | |
| | 21 | 39.3 | 0.0 | 0.0 | 56454 | 56481 | 252632 | + | AT_rich | Low_Cplxty | 1 | 28 | 0 | 122 | |
| | 2264 | 11.3 | 0.0 | 1.0 | 56869 | 57169 | 251944 | C | AluSxl | SINE/Alu | -14 | 298 | 1 | 123 | |
| | 2295 | 9.9 | 0.6 | 0.6 | 57258 | 57570 | 251543 | C | AluSp | SINE/Alu | 0 | 313 | 1 | 124 | |
| | 660 | 16.5 | 0.0 | 12.2 | 57575 | 57624 | 251489 | C | FLAM_C | SINE/Alu | -10 | 123 | 81 | 125 | |
| | 2194 | 11.5 | 0.3 | 0.3 | 57625 | 57920 | 251193 | C | AluSxl | SINE/Alu | -16 | 296 | 1 | 126 | |
| | 660 | 16.5 | 0.0 | 12.2 | 57921 | 58007 | 251106 | C | FLAM_C | SINE/Alu | -53 | 80 | 1 | 125 | |
| | 1846 | 11.2 | 10. 0 | 0.0 | 58454 | 58743 | 250370 | + | AluSq2 | SINE/Alu | 1 | 312 | 0 | 127 | |
| **probe 8** | 211 | 30.5 | 3.4 | 0.0 | 59728 | 59786 | 249327 | C | L2b | LINE/L2 | -7 | 336 8 | 3308 | 128 | 3 |
| | 1431 | 8.3 | 0.0 | 0.6 | 59852 | 60031 | 249082 | C | AluSp | SINE/Alu | -133 | 180 | 2 | 129 | |
| | 1870 | 13.5 | 1.8 | 2.1 | 60059 | 60340 | 248773 | + | AluJo | SINE/Alu | 1 | 281 | -31 | 130 | |
| | 398 | 16.9 | 2.2 | 5.8 | 60348 | 60436 | 248677 | + | FLAM_A | SINE/Alu | 42 | 127 | -15 | 131 | |
| **excluded region 10** | 1908 | 14.1 | 5.0 | 0.0 | 62695 | 62991 | - 246122 | C | AluSz | SINE/Alu | 0 | 312 | 1 | 132 | 4 |
| | 219 | 26.6 | 7.8 | 0.0 | 63055 | 63118 | 245995 | C | L2a | LINE/L2 | -5 | 342 1 | 3353 | 133 | |
| | 2274 | 8.9 | 0.7 | 2.0 | 63394 | 63567 | 245546 | C | AluSx | SINE/Alu | -5 | 307 | 134 | 134 | |
| | 2444 | 8.1 | 0.0 | 0.0 | 63568 | 63865 | - 245248 | C | AluY | SINE/Alu | -13 | 298 | 1 | 135 | |
| | 2274 | 8.9 | 0.7 | 2.0 | 63866 | 64000 | 245113 | C | AluSx | SINE/Alu | -179 | 133 | 2 | 134 | |
| **probe 9** | 951 | 10.3 | 0.8 | 0.0 | 64794 | 64919 | 244194 | + | AluSx4 | SINE/Alu | 179 | 305 | -7 | 136 | 1 |
| | 447 | 25. | 3.4 | 0.0 | 65518 | 65636 | 243477 | C | LlME2z | LINE/L1 | -3 | 644 1 | 6319 | 137 | |
| | 390 | 4.2 | 0.0 | 0.0 | 65637 | 65684 | 243429 | + | (CA)n | Simple | 1 | 48 | 0 | 138 | |
| | 319 | 27.9 | 1.2 | 0.0 | 65785 | 65870 | 243243 | + | L2c | LINE/L2 | 3295 | 338 1 | -6 | 139 | |
| | 468 | 29.4 | 4.9 | 2.4 | 66559 | 66913 | 242200 | + | LlME4a | UNE/L1 | 5471 | 584 9 | -275 | 140 | |
| **excluded region 11** | 468 | 29.4 | 4.9 | 2.4 | 66559 | 66913 | 242200 | + | L1ME4a | UNE/L1 | 5471 | 584 9 | -275 | 140 | 29 |
| | 2423 | 10.3 | 0.3 | 0.0 | 66917 | 67227 | 241886 | + | AluSp | SINE/Alu | 1 | 312 | -1 | 141 | |
| | 1271 | 20.6 | 1.3 | 7.2 | 67277 | 67586 | 241527 | C | AluJb | SINE/Alu | -18 | 294 | 2 | 142 | |
| | 1136 | 14.8 | 3.9 | 1.1 | 67686 | 67910 | 241203 | C | L1MB3 | UNE/L1 | -142 | 614 9 | 5936 | 143 | |
| | 319 | 20.7 | 0.0 | 1.7 | 67920 | 67978 | 241135 | C | MER66C | LTR/ERV1 | -133 | 422 | 365 | 144 | |
| | 637 | 14.4 | 0.0 | 0.0 | 67980 | 68076 | 241037 | C | L1MB3 | LINE/L1 | -239 | 594 1 | 5845 | 143 | |
| | 2023 | 12.9 | 0.0 | 3.4 | 68567 | 68869 | 240244 | + | AluSx1 | SINE/Alu | 1 | 293 | -19 | 145 | |
| | 1001 | 10.2 | 0.0 | 0.0 | 69082 | 69208 | 239905 | C | AluSq | SINE/Alu | -11 | 302 | 176 | 146 | |
| | 1879 | 16.8 | 1.0 | 0.7 | 69264 | 69566 | 239547 | + | AluJb | SINE/Alu | 1 | 304 | -8 | 147 | |
| | 233 | 30.9 | 0.6 | 0.0 | 69730 | 69811 | 239302 | + | MIRb | SINE/MIR | 64 | 155 | -113 | 148 | |
| | 2043 | 11.6 | 0.0 | 0.4 | 69909 | 70185 | 238928 | C | AluSxl | SINE/Alu | -11 | 301 | 26 | 149 | |
| | 2040 | 15.7 | 0.3 | 0.3 | 74836 | 75147 | 233966 | + | AluJb | SINE/Alu | 1 | 312 | 0 | 150 | |
| | 2323 | 11.2 | 0.0 | 0.0 | 75632 | 75942 | 233171 | + | AluSz | SINE/Alu | 2 | 312 | 0 | 151 | |
| | 1259 | 12.3 | 0.0 | 0.0 | 75957 | 76126 | 232987 | + | AluSc5 | SINE/Alu | 130 | 299 | -13 | 152 | |
| | 317 | 18.6 | 11. 4 | 0.0 | 76427 | 76496 | 232617 | + | MIR3 | SINE/MIR | 125 | 202 | -6 | 153 | |
| | 818 | 16.1 | 2.8 | 6.4 | 76513 | 76691 | 232422 | + | L1PREC2 | LINE/L1 | 5984 | 615 6 | -4 | 154 | |
| | 213 | 14.6 | 3.9 | 6.0 | 76911 | 76961 | 232152 | C | L2b | LINE/L2 | -8 | 336 7 | 3318 | 155 | |
| | 859 | 14.5 | 1.5 | 0.8 | 77008 | 77138 | 231975 | + | AluSz | SINE/Alu | 2 | 133 | -179 | 156 | |
| | 792 | 26.0 | 4.7 | 0.4 | 77151 | 77382 | 231731 | + | MIR | SINE/MIR | 20 | 261 | -1 | 157 | |
| | 1679 | 14.3 | 6.3 | 2.0 | 77567 | 77852 | 231261 | C | AluJr | SINE/Alu | -14 | 298 | 1 | 158 | |
| | 39 | 73.2 | 0.0 | 1.8 | 77874 | 77905 | 231208 | + | AT_rich | Low_Cplxty | 1 | 32 | 0 | 159 | |
| | 2010 | 11.5 | 1.0 | 3.5 | 77906 | 78201 | 230912 | C | AluSx | SINE/Alu | -23 | 289 | 1 | 160 | |
| | 39 | 73.2 | 0.0 | 1.8 | 78202 | 78225 | 230888 | + | AT_rich | Low_Cplxty | 1 | 24 | 0 | 161 | |
| | 719 | 20.3 | 0.0 | 0.0 | 78226 | 78343 | 230770 | C | AluJo | SINE/Alu | -194 | 118 | 1 | 162 | |
| | 2399 | 7.0 | 0.3 | 2.0 | 78356 | 78657 | 230456 | C | AluSp | SINE/Alu | -15 | 298 | 2 | 163 | |
| | 2302 | 11.2 | 0.3 | 0.3 | 78796 | 79106 | 230007 | C | AluSp | SINE/Alu | -2 | 311 | 1 | 164 | |
| | 813 | 14.2 | 2.5 | 0.0 | 79584 | 79703 | 229410 | + | AluJr | SINE/Alu | 1 | 123 | -189 | 165 | |
| | 1195 | 11.6 | 0.0 | 3.6 | 79875 | 80047 | 229066 | C | AluSc8 | SINE/Alu | -16 | 296 | 130 | 166 | |
| | 891 | 8.6 | 2.8 | 2.2 | 80061 | 80238 | 228875 | + | (TA)n | Simple | 2 | 180 | 0 | 167 | |
| | 2249 | 9.9 | 0.7 | 0.0 | 80275 | 80566 | 228547 | C | AluSx | SINE/Alu | -18 | 294 | 1 | 168 | |
| | 2011 | 15.6 | 0.0 | 0.0 | 80729 | 81029 | 228084 | C | AluSg | SINE/Alu | -8 | 302 | 2 | 169 | |
| | 2222 | 11.8 | 0.3 | 0.0 | 81042 | 81337 | 227776 | C | AluSz | SINE/Alu | -15 | 297 | 1 | 170 | |
| | 1207 | 21.6 | 6.4 | 5.7 | 81444 | 81606 | 227507 | C | AluJb | SINE/Alu | -4 | 298 | 134 | 171 | |
| | 2190 | 9.2 | 0.0 | 0.3 | 81607 | 81890 | 227223 | C | AluY | SINE/Alu | -12 | 299 | 17 | 172 | |
| | 2382 | 8.4 | 0.0 | 0.0 | 81894 | 82190 | 226923 | C | AluSc5 | SINE/Alu | -15 | 297 | 1 | 173 | |
| | 1612 | 18.7 | 2.8 | 0.7 | 82193 | 82481 | 226632 | C | AluJo | SINE/Alu | -16 | 296 | 2 | 174 | |
| | 1207 | 21.6 | 6.4 | 5.7 | 82482 | 82605 | 226508 | C | AluJb | SINE/Alu | -169 | 133 | 2 | 171 | |
| | 2381 | 9.5 | 0.0 | 0.0 | 82721 | 83024 | 226089 | + | AluSx | SINE/Alu | 1 | 304 | -8 | 175 | |
| | 629 | 20.6 | 2.8 | 0.0 | 83049 | 83155 | 225958 | C | FLAM_A | SINE/Alu | -32 | 110 | 1 | 176 | |
| | 1596 | 9.9 | 0.0 | 0.0 | 83361 | 83561 | 225552 | + | AluSx | SINE/Alu | 1 | 201 | -111 | 177 | |
| | 402 | 9.6 | 0.0 | 0.0 | 83562 | 83613 | 225500 | + | AluSx | SINE/Alu | 251 | 302 | -10 | 177 | |
| | 207 | 0.0 | 0.0 | 0.0 | 83620 | 83642 | 225471 | + | (GAA)n | Simple | 2 | 24 | 0 | 178 | |
| **probe 11** | 23 | 56.7 | 0.0 | 0.0 | 83927 | 83956 | 225157 | + | AT_rich | Low_Cplxty | 1 | 30 | 0 | 179 | 2 |
| | 756 | 19.5 | 4.0 | 0.6 | 84063 | 84237 | 224876 | C | MER104 | DNA/TcMar-Tc2 | 0 | 181 | 1 | 180 | |
| | 1710 | 19.9 | 0.0 | 1.0 | 84774 | 85075 | 224038 | C | AluJr | SINE/Alu | -12 | 300 | 2 | 181 | |
| | 298 | 26.3 | 15.7 | 0.7 | 85233 | 85366 | 223747 | C | L2a | LINE/L2 | 0 | 342 6 | 3273 | 182 | |
| | 1918 | 12.8 | 4.3 | 0.3 | 85401 | 85681 | 223432 | + | AluJb | SINE/Alu | 18 | 309 | -3 | 183 | |
| | 700 | 18.2 | 0.0 | 6.0 | 86439 | 86596 | 222517 | + | L1M4 | LINE/L1 | 4729 | 487 7 | 1269 | 184 | |
| **excluded region 12** | 700 | 18.2 | 0.0 | 6.0 | 86439 | 86596 | - 222517 | + | L1M4 | LINE/L1 | 4729 | 487 7 | - 1269 | 184 | 18 |
| | 2561 | 5.3 | 0.3 | 0.0 | 86599 | 86898 | 222215 | C | AluY | SINE/Alu | -10 | 301 | 1 | 185 | |
| | 1921 | 12.4 | 6.0 | 1.6 | 86905 | 87203 | 221910 | C | AluSz6 | SINE/Alu | 0 | 312 | 1 | 186 | |
| | 645 | 18.4 | 0.0 | 5.2 | 87205 | 87347 | 221766 | + | L1M4 | LINE/L1 | 4873 | 5008 | 1138 | 184 | |
| | 1844 | 13.9 | 3.5 | 0.3 | 87599 | 87885 | 221228 | + | AluSz | SINE/Alu | 1 | 296 | -16 | 187 | |
| | 2072 | 10.9 | 3.0 | 1.6 | 87965 | 88268 | 220845 | + | AluSz6 | SINE/Alu | 1 | 308 | -4 | 188 | |
| | 2020 | 8.0 | 8.4 | 0.0 | 88269 | 88554 | 220559 | + | AluSp | SINE/Alu | 1 | 313 | 0 | 189 | |
| | 249 | 11.9 | 0.0 | 0.0 | 88567 | 88608 | 220505 | + | (TCTA)n | Simple | 1 | 42 | 0 | 190 | |
| | 1260 | 19.2 | 0.5 | 1.4 | 88609 | 88832 | 220281 | C | AluJr | SINE/Alu | -90 | 222 | 1 | 191 | |
| | 2443 | 7.5 | 0.0 | 0.0 | 89435 | 89729 | 219384 | C | AluY | SINE/Alu | -16 | 295 | 1 | 192 | |
| | 231 | 23.6 | 6.4 | 2.6 | 89730 | 89827 | 219286 | + | Tigger10 | DNA/TcMT | 101 | 204 | 1639 | 193 | |
| | 1848 | 18.3 | 0.3 | 0.7 | 89841 | 90140 | 218973 | + | AluJb | SINE/Alu | 1 | 299 | -13 | 194 | |
| | 836 | 13.2 | 2.5 | 0.0 | 90229 | 90349 | 218764 | + | AluSz | SINE/Alu | 1 | 124 | -188 | 195 | |
| | 2379 | 9.7 | 0.0 | 0.0 | 90355 | 90652 | - 218461 | + | AluSx | SINE/Alu | 1 | 298 | -14 | 196 | |
| | 771 | 27.4 | 5.0 | 8.2 | 90653 | 90773 | 218340 | + | Tigger10 | DNA/TcMT | 841 | 948 | -895 | 197 | |
| | 2275 | 11.6 | 0.0 | 0.0 | 90774 | 91074 | 218039 | + | AluSx | SINE/Alu | 1 | 301 | -11 | 198 | |
| | 2415 | 7.0 | 0.0 | 0.3 | 91077 | 91407 | 217706 | + | AluY | SINE/Alu | 2 | 311 | 0 | 199 | |
| | 771 | 27.4 | 5.0 | 8.2 | 91408 | 91630 | 217483 | + | Tigger10 | DNA/TcMT | 949 | 1180 | -663 | 197 | |
| | 2276 | 9.3 | 1.0 | 0.0 | 91631 | 91920 | 217193 | C | AluSx4 | SINE/Alu | -18 | 294 | 2 | 200 | |
| | 771 | 27.4 | 5.0 | 8.2 | 91921 | 91972 | 217141 | + | Tigger10 | DNA/TcMT | 1181 | 1229 | -614 | 197 | |
| | 1010 | 20.2 | 1.6 | 0.0 | 91975 | 92162 | 216951 | + | AluJr4 | SINE/Alu | 109 | 299 | -13 | 201 | |
| | 217 | 26.7 | 1.6 | 1.6 | 92163 | 92223 | 216890 | + | (CATATA)n | Simple | 5 | 65 | 0 | 202 | |
| | 2319 | 9.6 | 0.7 | 0.0 | 92336 | 92638 | - 216475 | C | AluSp | SINE/Alu | -8 | 305 | 1 | 203 | |
| | 1942 | 13.2 | 0.4 | 0.4 | 92899 | 93202 | 215911 | C | AluSc8 | SINE/Alu | 0 | 312 | 1 | 204 | |
| | 2094 | 11.2 | 3.1 | 0.3 | 93338 | 93623 | 215490 | + | AluSx1 | SINE/Alu | 2 | 295 | -17 | 205 | |
| | 887 | 20.1 | 0.0 | 0.0 | 93624 | 93767 | 215346 | C | AluJo | SINE/Alu | -32 | 280 | 137 | 206 | |
| | 252 | 33.6 | 6.9 | 0.0 | 93795 | 93910 | 215203 | + | Tigger15a | DNA/TcMT | 530 | 653 | -62 | 207 | |
| **probe 12** | 252 | 33.6 | 6.9 | 0.0 | 93795 | 93910 | 215203 | + | Tigger15a | DNA/TcMT | 530 | 653 | -62 | 207 | 2 |
| | 468 | 11.4 | 8.6 | 0.0 | 93927 | 93996 | 215117 | C | AluSq2 | SINE/Alu | -13 | 299 | 224 | 208 | |
| | 395 | 24.4 | 2.5 | 2.5 | 93999 | 94116 | 214997 | C | Charlie4z | DNA/hAT-Charlie | -46 | 121 | 4 | 209 | |
| | 2373 | 8.8 | 0.3 | 0.0 | 94759 | 95052 | 214061 | + | AluSx4 | SINE/Alu | 2 | 296 | -16 | 210 | |
| | 23 | 43.5 | 0.0 | 0.0 | 95358 | 95380 | 213733 | + | AT_rich | Low_Cplxty | 1 | 23 | 0 | 211 | |
| | 258 | 25.6 | 10.1 | 1.2 | 95449 | 95527 | 213586 | C | L2c | LINE/L2 | -16 | 337 1 | 3286 | 212 | |
| | 377 | 18.3 | 9.1 | 7.7 | 95752 | 95905 | 213208 | C | L1MC5 | LINE/L1 | -36 | 792 5 | 7770 | 213 | |
| **excluded region 13** | 377 | 18.3 | 9.1 | 7.7 | 95752 | 95905 | 213208 | C | L1MC5 | LINE/L1 | -36 | 792 5 | 7770 | 213 | 15 |
| | 728 | 16.7 | 11. 4 | 0.0 | 95916 | 96047 | 213066 | C | AluJo | SINE/Alu | -26 | 286 | 140 | 214 | |
| | 2235 | 10.5 | 0.3 | 0.3 | 96061 | 96354 | 212759 | C | AluSq2 | SINE/Alu | -18 | 294 | 1 | 215 | |
| | 823 | 23.1 | 9.4 | 1.1 | 96357 | 96637 | 212476 | C | L1MC5 | LINE/L1 | -444 | 751 7 | 7255 | 213 | |
| | 2036 | 13.5 | 0.0 | 1.0 | 96696 | 96992 | 212121 | + | AluSx4 | SINE/Alu | 1 | 294 | -18 | 216 | |
| | 2148 | 11.7 | 0.3 | 1.3 | 96996 | 97302 | 211811 | + | AluSg | SINE/Alu | 1 | 304 | -6 | 217 | |
| | 738 | 27.7 | 8.5 | 2.2 | 97396 | 97904 | 211209 | C | L2a | LINE/L2 | -12 | 341 4 | 2870 | 218 | |
| | 1585 | 12.8 | 0.0 | 20.1 | 97915 | 98272 | 210841 | C | AluJr4 | SINE/Alu | -14 | 298 | 1 | 219 | |
| | 1845 | 13.4 | 4.1 | 2.4 | 98298 | 98588 | 210525 | C | AluSx4 | SINE/Alu | -15 | 297 | 2 | 220 | |
| | 497 | 11.0 | 33.0 | 0.0 | 98722 | 98821 | 210292 | + | FLAM_C | SINE/Alu | 1 | 133 | -10 | 221 | |
| | 237 | 31.1 | 10.1 | 0.0 | 98916 | 99034 | 210079 | + | MIR3 | SINE/MIR | 5 | 135 | -73 | 222 | |
| | 2590 | 5.3 | 0.0 | 0.0 | 100020 | 100320 | - 208793 | + | AluYk4 | SINE/Alu | 1 | 301 | -11 | 223 | |
| | 1949 | 8.9 | 3.7 | 2.2 | 100331 | 100600 | 208513 | + | AluSg | SINE/Alu | 2 | 275 | -35 | 224 | |
| | 2347 | 7.8 | 0.0 | 0.0 | 100630 | 100937 | 208176 | + | AluY | SINE/Alu | 1 | 311 | 0 | 225 | |
| | 2326 | 10.1 | 0.7 | 0.0 | 100941 | 101248 | 207865 | + | AluSp | SINE/Alu | 3 | 312 | -1 | 226 | |
| | 590 | 26.8 | 13.0 | 0.5 | 101876 | 102152 | - 206961 | C | L2a | LINE/L2 | -2 | 342 4 | 3117 | 227 | |
| | 1614 | 16.1 | 1.7 | 2.8 | 102162 | 102300 | 206813 | + | AluJb | SINE/Alu | 1 | 134 | -168 | 228 | |
| | 2330 | 9.8 | 0.0 | 3.6 | 102301 | 102617 | 206496 | + | AluY | SINE/Alu | 1 | 306 | -5 | 229 | |
| | 1614 | 16.1 | 1.7 | 2.8 | 102618 | 102771 | 206342 | + | AluJb | SINE/Alu | 135 | 291 | -11 | 228 | |
| | 2237 | 9.1 | 2.0 | 0.0 | 102886 | 103183 | 205930 | C | AluSc5 | SINE/Alu | -8 | 304 | 1 | 230 | |
| **probe 13A** | 270 | 0.0 | 0.0 | 0.0 | 104284 | 104313 | 204800 | + | (TTTTG)n | Simple | 1 | 30 | 0 | 231 | 1 |
| | 1650 | 4.5 | 5.5 | 0.0 | 104318 | 104516 | 204597 | C | AluSx | SINE/Alu | -37 | 275 | 66 | 232 | |
| **excluded region 14** | 8064 | 14.0 | 7.8 | 5.5 | 106203 | 107278 | - 201835 | + | LTR12C | LTR/ERV1 | 3 | 114 0 | -439 | 233 | 10 |
| | 2324 | 10.1 | 0.0 | 0.3 | 107279 | 107586 | 201527 | + | AluY | SINE/Alu | 2 | 308 | -3 | 234 | |
| | 8064 | 14.0 | 7.8 | 5.5 | 107587 | 108052 | - 201061 | + | LTR12C | LTR/ERV1 | 1141 | 157 9 | 0 | 233 | |
| | 939 | 10.0 | 0.0 | 6.1 | 108354 | 108493 | 200620 | C | FLAM_C | SINE/Alu | -11 | 132 | 1 | 235 | |
| | 2397 | 8.1 | 0.0 | 1.6 | 109001 | 109308 | - 199805 | C | AluY | SINE/Alu | -7 | 304 | 2 | 236 | |
| | 790 | 13.7 | 1.6 | 1.6 | 109726 | 109849 | - 199264 | C | FLAM_C | SINE/Alu | -19 | 124 | 1 | 237 | |
| | 2100 | 13.8 | 0.3 | 0.0 | 109852 | 110149 | - 198964 | C | AluSz | SINE/Alu | -13 | 299 | 1 | 238 | |
| | 696 | 27.4 | 7.1 | 0.9 | 110153 | 110362 | - 198751 | C | MIRc | SINE/MIR | -1 | 267 | 45 | 239 | |
| | 248 | 31.0 | 6.2 | 0.0 | 110411 | 110523 | 198590 | C | L1M5 | LINE/L1 | -747 | 544 7 | 5328 | 240 | |
| | 189 | 7.4 | 0.0 | 0.0 | 110917 | 110943 | - 198170 | + | (TAA)n | Simple | 2 | 28 | 0 | 241 | |
| | 1606 | 7.3 | 0.0 | 0.0 | 111079 | 111269 | - 197844 | + | AluY | SINE/Alu | 104 | 294 | -17 | 242 | |
| | 2148 | 15.1 | 0.0 | 0.0 | 111309 | 111619 | - 197494 | C | AluSz6 | SINE/Alu | -1 | 311 | 1 | 243 | |
| | 431 | 16.2 | 14.1 | 0.0 | 111625 | 111723 | - 197390 | C | MIRb | SINE/MIR | -67 | 201 | 89 | 244 | |
| | 327 | 26.0 | 0.0 | 12.2 | 112010 | 112101 | - 197012 | + | MIRc | SINE/MIR | 37 | 118 | -150 | 245 | |
| | 1373 | 9.8 | 0.6 | 0.6 | 112104 | 112286 | - 196827 | C | AluSc | SINE/Alu | 0 | 309 | 127 | 246 | |
| | 2444 | 7.5 | 0.0 | 2.9 | 112288 | 112607 | - 196506 | C | AluY | SINE/Alu | 0 | 311 | 1 | 247 | |
| | 251 | 22.8 | 3.5 | 1.7 | 112610 | 112667 | - 196446 | + | MIR | SINE/MIR | 104 | 162 | -100 | 245 | |
| | 180 | 29.8 | 18.2 | 1.0 | 112901 | 112988 | 196125 | + | MER5A | DNA/hAT-Charlie | 68 | 170 | -19 | 248 | |
| | 2303 | 12.0 | 0.0 | 0.0 | 113162 | 113470 | 195643 | C | AluSz | SINE/Alu | -3 | 309 | 1 | 249 | |
| **probe 15** | 804 | 14.4 | 1.6 | 0.0 | 115549 | 115673 | 193440 | + | FLAM_C | SINE/Alu | 2 | 128 | -15 | 250 | 1 |
| | 7181 | 6.4 | 0.7 | 0.1 | 115705 | 116977 | - 192136 | + | L1PA5 | LINE/L1 | 4875 | 615 4 | 0 | 251 | |
| **excluded region 15** | 1884 | 13.3 | 1.9 | 0.4 | 117135 | 117404 | - 191709 | + | AluSz | SINE/Alu | 1 | 274 | -38 | 252 | 2 |
| | 180 | 0.0 | 0.0 | 0.0 | 117411 | 117430 | 191683 | + | (CAAAA)n | Simple | 1 | 20 | 0 | 253 | |
| | 2240 | 12.3 | 1.0 | 0.0 | 117441 | 117749 | 191364 | + | AluSq2 | SINE/Alu | 1 | 312 | 0 | 254 | |
| | 224 | 37.7 | 0.0 | 0.0 | 117758 | 117834 | - 191279 | + | L2 | LINE/L2 | 458 | 534 | - 2885 | 255 | |
| **probe 16** | 652 | 29.2 | 9.5 | 7.2 | 118175 | 118595 | 190518 | + | LTR33B | LTR/ERVL | 53 | 482 | -21 | 256 | 0 |
| | 722 | 16.5 | 0.0 | 2.5 | 118599 | 118722 | - 190391 | + | MER21C | LTR/ERVL | 1 | 121 | -817 | 257 | |
| | 2342 | 12.3 | 0.0 | 2.8 | 118771 | 118897 | 190216 | C | L1PREC2 | LINE/L1 | 0 | 616 0 | 6034 | 258 | |
| **excluded region 16** | 2262 | 9.2 | 2.7 | 0.0 | 118898 | 119189 | 189924 | C | AluSg4 | SINE/Alu | -12 | 300 | 1 | 259 | 1 |
| **probe 17** | 2262 | 9.2 | 2.7 | 0.0 | 118898 | 119189 | - 189924 | C | AluSg4 | SINE/Alu | -12 | 300 | 1 | 259 | 1 |
| | 2342 | 12.3 | 0.0 | 2.8 | 119190 | 119429 | 189684 | C | L1PREC2 | LINE/L1 | -127 | 603 3 | 5803 | 258 | |
| | 1975 | 21.0 | 10.4 | 1.1 | 119430 | 120051 | - 189062 | + | MER21C | LTR/ERVL | 111 | 790 | -148 | 257 | |
| | 279 | 35.6 | 6.5 | 1.6 | 120054 | 120343 | 188770 | + | L2c | LINE/L2 | 3030 | 334 9 | -38 | 260 | |
| | 440 | 17.1 | 4.2 | 6.9 | 120617 | 120735 | - 188378 | + | MLT1M | LTR/ERVL-MaLR | 83 | 198 | -474 | 261 | |
| **excluded region 17** | 1069 | 13.8 | 0.0 | 1.3 | 120857 | 121016 | - 188097 | + | AluJo | SINE/Alu | 135 | 292 | -20 | 262 | 12 |
| | 28 | 62.9 | 0.0 | 0.0 | 121035 | 121069 | - 188044 | + | AT_rich | Low_Cplxty | 1 | 35 | 0 | 263 | |
| | 2240 | 6.4 | 1.1 | 0.0 | 121072 | 121338 | - 187775 | + | AluY | SINE/Alu | 3 | 272 | -39 | 264 | |
| | 2197 | 11.4 | 0.0 | 0.7 | 121453 | 121749 | - 187364 | C | AluSx | SINE/Alu | -17 | 295 | 1 | 265 | |
| | 265 | 28.2 | 1.4 | 1.4 | 121841 | 121912 | 187201 | + | MIRb | SINE/MIR | 197 | 268 | 0 | 266 | |
| | 503 | 30.5 | 4.4 | 5.3 | 121998 | 122246 | - 186867 | + | MIRb | SINE/MIR | 19 | 265 | -3 | 267 | |
| | 1266 | 11.9 | 0.0 | 1.1 | 122278 | 122453 | - 186660 | C | AluSp | SINE/Alu | -13 | 300 | 127 | 268 | |
| | 726 | 22.5 | 0.0 | 0.0 | 122457 | 122629 | - 186484 | + | (TATATG) n | Simple | 4 | 176 | 0 | 269 | |
| | 23 | 34.8 | 0.0 | 0.0 | 122630 | 122652 | - 186461 | + | AT_rich | Low_Cplxty | 1 | 23 | 0 | 270 | |
| | 940 | 11.3 | 0.8 | 0.0 | 122653 | 122776 | - 186337 | C | AluSp | SINE/Alu | -188 | 125 | 1 | 268 | |
| | 26 | 60.6 | 0.0 | 0.0 | 123439 | 123471 | - 185642 | + | AT_rich | Low_Cplxty | 1 | 33 | 0 | 271 | |
| | 2378 | 7.4 | 0.0 | 1.0 | 123475 | 123773 | - 185340 | + | AluY | SINE/Alu | 1 | 296 | -15 | 272 | |
| | 784 | 13.1 | 0.0 | 0.0 | 124275 | 124381 | - 184732 | + | AluSx | SINE/Alu | 1 | 107 | -205 | 273 | |
| | 2735 | 4.2 | 0.0 | 0.0 | 124853 | 125161 | - 183952 | C | AluY | SINE/Alu | -2 | 309 | 1 | 274 | |
| | 2424 | 8.1 | 0.0 | 0.0 | 125836 | 126131 | 182982 | C | AluY | SINE/Alu | -3 | 308 | 13 | 275 | |
| | 1876 | 10.7 | 1.6 | 5.1 | 126545 | 126728 | 182385 | C | AluSx | SINE/Alu | -17 | 295 | 108 | 276 | |
| | 2573 | 5.1 | 0.0 | 0.0 | 126729 | 127023 | - 182090 | C | AluY | SINE/Alu | -15 | 296 | 2 | 277 | |
| | 1876 | 10.7 | 1.6 | 5.1 | 127024 | 127143 | 181970 | C | AluSx | SINE/Alu | -205 | 107 | 1 | 276 | |
| **probe 18** | 25 | 72.0 | 0.0 | 0.0 | 127246 | 127270 | - 181843 | + | AT_rich | Low_Cplxty | 1 | 25 | 0 | 278 | 1 |
| | 240 | 21.1 | 16. 9 | 4.0 | 127577 | 127665 | 181448 | + | MIR3 | SINE/MIR | 94 | 193 | -15 | 279 | |
| | 1262 | 8.1 | 1.7 | 1.1 | 127666 | 127838 | 181275 | + | AluSp | SINE/Alu | 124 | 297 | -16 | 280 | |
| | 2123 | 13.3 | 16. 2 | 0.4 | 127864 | 128270 | - 180843 | C | LTR7C | LTR/ERV1 | 0 | 471 | 1 | 281 | |
| | 576 | 20.3 | 3.1 | 3.9 | 128487 | 128614 | - 180499 | C | MER2B | DNA/TcMT | 0 | 336 | 210 | 282 | |
| **excluded region 18** | 576 | 20.3 | 3.1 | 3.9 | 128487 | 128614 | - 180499 | C | MER2B | DNA/TcMT | 0 | 336 | 210 | 282 | 4 |
| | 1973 | 10.5 | 4.9 | 5.6 | 128631 | 128935 | - 180178 | C | AluY | SINE/Alu | -8 | 303 | 1 | 283 | |
| | 1150 | 5.9 | 0.0 | 0.0 | 128936 | 129070 | - 180043 | C | AluSz | SINE/Alu | -177 | 135 | 1 | 284 | |
| | 187 | 33. 4 | 7.1 | 9.9 | 129286 | 129324 | 179789 | + | L2 | LINE/L2 | 2142 | 218 1 | - 1238 | 285 | |
| | 2251 | 10. 0 | 0.0 | 1.0 | 129325 | 129624 | 179489 | C | AluSg4 | SINE/Alu | -14 | 298 | 2 | 286 | |
| | 187 | 33. 4 | 7.1 | 9.9 | 129625 | 129648 | - 179465 | + | L2 | LINE/L2 | 2182 | 219 2 | - 1227 | 285 | |
| | 1745 | 16. 7 | 3.5 | 0.0 | 129649 | 129935 | 179178 | C | AluJb | SINE/Alu | -15 | 297 | 1 | 287 | |
| | 187 | 33. 4 | 7.1 | 9.9 | 129936 | 130109 | 179004 | + | L2 | LINE/L2 | 2193 | 237 4 | - 1045 | 285 | |
| **probe 19** | 187 | 33. 4 | 7.1 | 9.9 | 129936 | 130109 | 179004 | + | L2 | LINE/L2 | 2193 | 237 4 | - 1045 | 285 | 2 |
| | 548 | 25.0 | 0.0 | 0.0 | 130353 | 130464 | 178649 | + | MER81 | DNA/hAT-Bkjk | 2 | 113 | -1 | 288 | |
| | 397 | 20.0 | 3.0 | 1.0 | 130604 | 130704 | - 178409 | + | LTR88b | LTR/Gypsy? | 722 | 824 | -13 | 289 | |
| | 1038 | 18.1 | 0.0 | 0.6 | 130839 | 131004 | 178109 | + | AluSz6 | SINE/Alu | 7 | 171 | -141 | 290 | |
| | 207 | 0.0 | 0.0 | 0.0 | 131023 | 131045 | 178068 | + | (CAAAAA)n | Simple | 2 | 24 | 0 | 291 | |
| | 1739 | 17.6 | 0.0 | 2.7 | 131144 | 131445 | 177668 | + | AluJr | SINE/Alu | 1 | 294 | -18 | 292 | |
| **excluded region 19** | 1739 | 17.6 | 0.0 | 2.7 | 131144 | 131445 | - 177668 | + | AluJr | SINE/Alu | 1 | 294 | -18 | 292 | 18 |
| | 683 | 21. 3 | 8.9 | 2.2 | 131485 | 131652 | 177461 | C | MIRb | SINE/MIR | -35 | 233 | 55 | 293 | |
| | 290 | 24.9 | 15.2 | 3.1 | 131818 | 131962 | - 177151 | + | L2c | LINE/L2 | 3225 | 338 6 | -1 | 294 | |
| | 2015 | 12.0 | 0.6 | 1.3 | 131975 | 132108 | 177005 | + | AluSx | SINE/Alu | 1 | 135 | -177 | 295 | |
| | 2358 | 8.6 | 0.0 | 3.0 | 132109 | 132421 | 176692 | + | AluY | SINE/Alu | 1 | 304 | -7 | 296 | |
| | 2015 | 12.0 | 0.6 | 1.3 | 132422 | 132598 | 176515 | + | AluSx | SINE/Alu | 136 | 310 | -2 | 295 | |
| | 369 | 16.2 | 0.0 | 2.9 | 132682 | 132751 | 176362 | C | L1MC5 | LINE/L1 | -523 | 7438 | 7371 | 297 | |
| | 3496 | 8.6 | 2.0 | 1.4 | 13275 2 | 133237 | 175876 | + | LTR15 | LTR/ERV1 | 1 | 671 | -4 | 298 | |
| | 378 | 23.8 | 13. 4 | 0.5 | 133242 | 133382 | - 175731 | C | L1MC5 | LINE/L1 | -547 | 749 5 | 7255 | 297 | |
| | 2042 | 13.2 | 0.3 | 0.7 | 133441 | 133736 | 175377 | + | AluSx | SINE/Alu | 1 | 295 | -17 | 299 | |
| | 2238 | 9.5 | 0.0 | 0.0 | 133740 | 134023 | 175090 | + | AluSg | SINE/Alu | 1 | 284 | -26 | 300 | |
| | 371 | 4.7 | 0.0 | 0.0 | 134037 | 134079 | 175034 | + | AluSz6 | SINE/Alu | 244 | 286 | -26 | 301 | |
| | 694 | 29.0 | 9.4 | 4.0 | 134183 | 134701 | 174412 | C | L2a | LINE/L2 | 0 | 337 5 | 2870 | 302 | |
| | 1211 | 10.9 | 39. 0 | 1.0 | 134705 | 134933 | - 174180 | C | AluSx3 | SINE/Alu | -14 | 298 | 1 | 303 | |
| | 651 | 22.9 | 0.8 | 0.0 | 134943 | 135064 | 174049 | C | AluSz | SINE/Alu | -187 | 125 | 3 | 303 | |
| | 1658 | 16.3 | 4.3 | 2.1 | 135083 | 135358 | 173755 | C | AluSz | SINE/Alu | -30 | 282 | 1 | 304 | |
| | 2301 | 11.2 | 0.3 | 0.0 | 135492 | 135794 | 173319 | + | AluSx | SINE/Alu | 1 | 304 | -8 | 305 | |
| | 375 | 28.3 | 11. 6 | 1.6 | 135871 | 136110 | 173003 | + | MIRc | SINE/MIR | 2 | 268 | 0 | 306 | |
| | 2136 | 11.4 | 1.0 | 0.7 | 136954 | 137251 | 171862 | + | AluSc8 | SINE/Alu | 1 | 299 | -13 | 307 | |
| | 2368 | 7.1 | 1.0 | 0.3 | 137253 | 137549 | 171564 | + | AluSp | SINE/Alu | 3 | 301 | -12 | 308 | |
| | 801 | 26.6 | 8.3 | 0.7 | 138199 | 138452 | 170661 | C | L2a | LINE/L2 | -1 | 342 5 | 3153 | 309 | |
| | 1432 | 15.2 | 6.6 | 0.3 | 138490 | 138606 | 170507 | + | AluJb | SINE/Alu | 1 | 117 | -195 | 310 | |
| | 195 | 6.9 | 0.0 | 0.0 | 138607 | 138635 | 170478 | + | (CA)n | Simple | 2 | 30 | 0 | 311 | |
| | 1432 | 15.2 | 6.6 | 0.3 | 138636 | 138788 | 170325 | + | AluJb | SINE/Alu | 118 | 287 | -25 | 310 | |
| | 254 | 12.8 | 0.0 | 0.0 | 138793 | 138831 | 170282 | + | L1ME3 | LINE/L1 | 6124 | 6162 | 0 | 312 | |
| | 1283 | 15.2 | 0.6 | 4.5 | 138839 | 139162 | - 169951 | C | SVA_F | Other | -615 | 760 | 449 | 313 | |
| | 2029 | 2.1 | 0.0 | 0.0 | 139163 | 139395 | - 169718 | + | SVA_C | Other | 1152 | 138 4 | 0 | 314 | |
| | 1528 | 7.5 | 0.0 | 1.5 | 13957 9 | 139781 | - 169332 | C | AluY | SINE/Alu | -13 | 298 | 99 | 315 | |
| | 3520 | 7.6 | 0.2 | 2.8 | 139782 | 140256 | - 168857 | C | LTR2 | LTR/ERV1 | 0 | 463 | 1 | 316 | |
| | 7381 | 7.3 | 2.1 | 0.0 | 140257 | 141186 | - 167927 | C | Harleq-int | LTR/ERV1 | 0 | 784 7 | 6898 | 316 | |
| | 3412 0 | 6.3 | 0.8 | 0.3 | 141187 | 145402 | 163711 | C | Harleq-int | LTR/ERV1 | -996 | 590 0 | 1666 | 316 | |
| | 384 | 4.2 | 0.0 | 0.0 | 145423 | 145470 | - 163643 | + | L1PA3 | LINE/L1 | 6103 | 615 0 | -5 | 317 | |
| | 637 | 8.0 | 4.9 | 1.9 | 145480 | 145581 | - 163532 | C | Harleq-int | LTR/ERV1 | - 5222 | 167 4 | 1570 | 316 | |
| | 5813 | 9.7 | 2.9 | 2.2 | 145595 | 146781 | - 162332 | C | Harleq-int | LTR/ERV1 | - 5816 | 108 0 | 1 | 316 | |
| | 3514 | 7.8 | 0.4 | 0.2 | 146783 | 147234 | - 161879 | C | LTR2 | LTR/ERV1 | -10 | 453 | 1 | 316 | |
| | 775 | 7.8 | 0.0 | 0.0 | 147235 | 147336 | - 161777 | C | AluY | SINE/Alu | -209 | 102 | 1 | 315 | |
| | 2256 | 9.6 | 0.3 | 0.7 | 147892 | 148194 | - 160919 | + | AluSp | SINE/Alu | 1 | 302 | -11 | 318 | |
| **probe 22** | 2246 | 7.9 | 3.5 | 0.0 | 148712 | 149001 | - 160112 | C | AluSg | SINE/Alu | -9 | 301 | 2 | 319 | 2 |
| | 21 | 42.9 | 0.0 | 0.0 | 150814 | 150834 | - 158279 | + | GC_rich | Low_Cplxty | 1 | 21 | 0 | 320 | |
| | 740 | 14.6 | 0.0 | 6.6 | 151349 | 151478 | 157635 | C | FLAM_C | SINE/Alu | -21 | 122 | 1 | 321 | |
| **excluded region 20** | 2502 | 6.8 | 0.0 | 0.3 | 152355 | 152661 | - 156452 | C | AluY | SINE/Alu | -5 | 306 | 1 | 322 | 5 |
| | 794 | 13.7 | 1.6 | 1.6 | 152695 | 152818 | 156295 | C | FLAM_C | SINE/Alu | -19 | 124 | 1 | 323 | |
| | 2085 | 13.3 | 1.3 | 0.0 | 152821 | 153120 | 155993 | C | AluSz | SINE/Alu | -8 | 304 | 1 | 324 | |
| | 563 | 32.8 | 6.6 | 1.5 | 153132 | 153370 | 155743 | C | MIRc | SINE/MIR | -10 | 258 | 3 | 325 | |
| | 791 | 18. 7 | 9.2 | 4.2 | 153566 | 153838 | - 155275 | + | L1MC5 | LINE/L1 | 7642 | 792 7 | -34 | 326 | |
| | 2240 | 9.6 | 0.0 | 0.7 | 153853 | 154145 | 154968 | + | AluSc8 | SINE/Alu | 3 | 293 | -19 | 327 | |
| | 28 | 67.9 | 0.0 | 0.0 | 154149 | 154176 | 154937 | + | AT_rich | Low_Cplxty | 1 | 28 | 0 | 328 | |
| | 2160 | 9.6 | 2.2 | 3.9 | 154350 | 154662 | 154451 | + | AluY | SINE/Alu | 1 | 308 | -3 | 329 | |
| **probe 23** | 216 | 27.8 | 3.8 | 1.2 | 154848 | 154927 | 154186 | + | L2a | LINE/L2 | 3302 | 338 3 | -43 | 330 | 1 |
| | 298 | 25.0 | 4.6 | 4.6 | 155156 | 155264 | - 153849 | + | L2b | LINE/L2 | 3256 | 336 4 | -11 | 331 | |
| | 1947 | 15.3 | 0.3 | 0.7 | 156525 | 156824 | 152289 | + | AluJb | SINE/Alu | 1 | 299 | -13 | 332 | |
| | 252 | 27.7 | 8.2 | 5.8 | 156901 | 157034 | - 152079 | C | L1MC | LINE/L1 | - 2228 | 565 4 | 5518 | 333 | |
| | 441 | 0.0 | 0.0 | 0.0 | 157109 | 157157 | 151956 | + | (CA)n | Simple | 2 | 50 | 0 | 334 | |
| | 315 | 28.3 | 5.2 | 0.0 | 157159 | 157290 | - 151823 | C | L1M5 | LINE/L1 | -655 | 546 8 | 5326 | 335 | |
| **excluded region 21** | 813 | 14.2 | 0.0 | 3.5 | 157768 | 157887 | 151226 | C | AluJo | SINE/Alu | -196 | 116 | 1 | 336 | 3 |
| | 2245 | 13.2 | 0.0 | 0.0 | 157903 | 158212 | 150901 | C | AluSz | SINE/Alu | -2 | 310 | 1 | 337 | |
| | 958 | 19.8 | 6.9 | 0.9 | 158305 | 158506 | 150607 | C | AluJr | SINE/Alu | -12 | 300 | 87 | 338 | |
| **probe24** | 515 | 29.2 | 0.6 | 1.3 | 158572 | 158727 | - 150386 | C | MIR | SINE/MIR | -106 | 156 | 2 | 339 | 0 |
| | 559 | 23.7 | 7.7 | 1.8 | 159274 | 159428 | 149685 | C | Tiggerl6b | DNA/TcMT | -16 | 321 | 158 | 340 | |
| | 276 | 19.7 | 0.0 | 0.0 | 159632 | 159697 | 149416 | C | L1MA9 | LINE/L1 | -19 | 629 3 | 6228 | 341 | |
| | 1903 | 14.2 | 6.8 | 0.3 | 159698 | 160008 | 149105 | C | Tigger3a | DNA/TcMT | 0 | 348 | 18 | 342 | |
| | 304 | 29.1 | 1.7 | 10.2 | 160014 | 160193 | - 148920 | C | L1MA9 | LINE/L1 | -93 | 621 9 | 6054 | 341 | |
| | 26 | 69.2 | 0.0 | 0.0 | 160250 | 160275 | 148838 | + | AT_rich | Low_Cplxty | 1 | 26 | 0 | 343 | |
| **excluded region 22** | 30 | 60.0 | 0.0 | 0.0 | 160373 | 160402 | 148711 | + | AT_rich | Low_Cplxty | 1 | 30 | 0 | 344 | 16 |
| | 1901 | 16.8 | 0.3 | 0.3 | 160410 | 160707 | 148406 | C | AluJb | SINE/Alu | -14 | 298 | 1 | 345 | |
| | 2429 | 6.6 | 2.3 | 0.0 | 160926 | 161228 | 147885 | + | AluY | SINE/Alu | 1 | 310 | -1 | 346 | |
| | 2151 | 12.8 | 0.3 | 1.0 | 161239 | 161543 | 147570 | + | AluSq2 | SINE/Alu | 1 | 303 | -9 | 347 | |
| | 812 | 17.1 | 0.0 | 1.6 | 161559 | 161687 | 147426 | C | FLAM A | SINE/Alu | -13 | 129 | 3 | 348 | |
| | 2239 | 11.0 | 0.3 | 1.3 | 161748 | 162056 | 147057 | C | AluSz6 | SINE/Alu | -6 | 306 | 1 | 349 | |
| | 637 | 9.0 | 0.8 | 11.5 | 162165 | 162289 | - 146824 | C | L1MA9 | LINE/L1 | -33 | 627 9 | 6167 | 350 | |
| | 2152 | 13.0 | 0.0 | 0.0 | 162300 | 162598 | - 146515 | C | AluSx | SINE/Alu | -12 | 300 | 2 | 351 | |
| | 853 | 17.8 | 0.0 | 0.0 | 162600 | 162728 | 146385 | C | FLAM_C | SINE/Alu | -14 | 129 | 1 | 352 | |
| | 2348 | 9.8 | 0.0 | 0.0 | 162759 | 163053 | 146060 | C | AluSc | SINE/Alu | -13 | 296 | 2 | 353 | |
| | 753 | 24.7 | 0.0 | 0.7 | 163054 | 163199 | 145914 | C | AluJb | SINE/Alu | -32 | 280 | 136 | 354 | |
| | 1899 | 16.7 | 2.0 | 0.0 | 163202 | 163494 | - 145619 | C | AluSz6 | SINE/Alu | -12 | 300 | 2 | 355 | |
| | 21 | 67.9 | 0.0 | 0.0 | 163511 | 163538 | 145575 | + | AT_rich | Low_Cplxty | 1 | 28 | 0 | 356 | |
| | 1411 | 15. | 1.9 | 12. 5 | 163577 | 163884 | 145229 | C | AluJo | SINE/Alu | -23 | 289 | 11 | 357 | |
| | 2314 | 10.8 | 0.0 | 0.0 | 16390 | 164201 | 144912 | C | AluSx | SINE/Alu | -16 | 296 | 1 | 358 | |
| | 2470 | 9.1 | 0.3 | 0.0 | 164346 | 164653 | 144460 | + | AluSc | SINE/Alu | 1 | 309 | 0 | 359 | |
| | 629 | 21. | 7.3 | 0.0 | 164831 | 164954 | 144159 | + | AluJb | SINE/Alu | 4 | 136 | -176 | 360 | |
| | 1493 | 17. | 4.8 | 2.0 | 164955 | 165244 | 143869 | + | AluJo | SINE/Alu | 2 | 299 | -13 | 361 | |
| | 2231 | 9.3 | 0.0 | 1.4 | 165251 | 165587 | 143526 | + | AluSq2 | SINE/Alu | 1 | 312 | 0 | 362 | |
| **probe25** | 5877 | 8.3 | 2.5 | 6.2 | 166057 | 166719 | - 142394 | C | L1PA7 | UNE/L1 | -1 | 615 3 | 5491 | 363 | 0 |
| **excluded region 23** | 5877 | 8.3 | 2.5 | 6.2 | 166057 | 166719 | - 142394 | C | L1PA7 | LINE/L1 | -1 | 615 3 | 5491 | 363 | 3 |
| | 2432 | 7.4 | 0.0 | 0.7 | 166720 | 167015 | 142098 | C | AluY | SINE/Alu | -17 | 294 | 1 | 364 | |
| | 5877 | 8.3 | 2.5 | 6.2 | 167016 | 167038 | - 142075 | C | L1PA7 | LINE/L1 | -664 | 549 0 | 5490 | 363 | |
| | 2296 | 11.5 | 0.0 | 0.0 | 167039 | 167343 | - 141770 | C | AluSx3 | SINE/Alu | -7 | 305 | 1 | 365 | |
| | 5877 | 8.3 | 2.5 | 6.2 | 167344 | 167416 | - 141697 | C | L1PA7 | LINE/L1 | -664 | 549 0 | 5420 | 363 | |
| | 2527 | 8.4 | 0.0 | 0.0 | 167417 | 167725 | 141388 | C | AluY | SINE/Alu | -2 | 309 | 1 | 366 | |
| | 5877 | 7.4 | 1.0 | 0.3 | 167726 | 168279 | - 140834 | C | L1PA7 | LINE/L1 | -735 | 541 9 | 4870 | 363 | |
| **probe26** | 5877 | 7.4 | 1.0 | 0.3 | 167726 | 168279 | 140834 | C | L1PA7 | LINE/L1 | -735 | 541 9 | 4870 | 363 | 2 |
| | 1566 | 16. 2 | 8.3 | 0.3 | 169630 | 169907 | - 139206 | C | AluJb | SINE/Alu | -12 | 300 | 1 | 367 | |
| | 266 | 33.0 | 2.3 | 1.4 | 169960 | 170120 | - 138993 | C | MIRb | SINE/MIR | -96 | 172 | 5 | 368 | |
| | 1633 | 22.3 | 0.0 | 0.7 | 170506 | 170806 | - 138307 | + | AluJr | SINE/Alu | 1 | 299 | -13 | 369 | |
| **excluded region 24** | 2359 | 8.0 | 0.3 | 0.7 | 171255 | 171556 | - 137557 | C | AluY | SINE/Alu | -9 | 302 | 2 | 370 | 3 |
| | 2345 | 8.4 | 0.0 | 1.0 | 171557 | 171854 | - 137259 | C | AluSg | SINE/Alu | -12 | 298 | 4 | 371 | |
| | 2440 | 6.5 | 0.0 | 2.6 | 171895 | 172204 | - 136909 | C | AluY | SINE/Alu | -9 | 302 | 1 | 372 | |
| **probe27** | 500 | 17.8 | 10.2 | 1.4 | 173641 | 173784 | - 135329 | + | L1MC4a | LINE/L1 | 7729 | 799 4 | -1 | 373 | 0 |
| **excluded region 25** | 1743 | 15.8 | 0.3 | 6.0 | 174758 | 174905 | - 134208 | + | AluJb | SINE/Alu | 2 | 145 | -167 | 374 | 8 |
| | 2453 | 8.3 | 0.3 | 0.0 | 174906 | 175207 | - 133906 | + | AluSp | SINE/Alu | 1 | 303 | -10 | 375 | |
| | 1743 | 15.8 | 0.3 | 6.0 | 175208 | 175375 | - 133738 | + | AluJb | SINE/Alu | 146 | 301 | -11 | 374 | |
| | 2487 | 8.2 | 0.0 | 0.0 | 175378 | 175681 | - 133432 | + | AluSg7 | SINE/Alu | 1 | 304 | -8 | 376 | |
| | 1773 | 15.8 | 0.3 | 6.0 | 276759 | 276906 | -32207 | + | AluJb | SINE/Alu | 2 | 145 | -167 | 377 | |
| | 2466 | 8.3 | 0.3 | 0.0 | 276907 | 277207 | -31906 | + | AluSp | SINE/Alu | 1 | 302 | -11 | 378 | |
| | 1773 | 15.8 | 0.3 | 6.0 | 277208 | 277375 | -31738 | + | AluJb | SINE/Alu | 146 | 301 | -11 | 377 | |
| | 2510 | 8.5 | 0.0 | 0.0 | 277378 | 277684 | -31429 | + | AluSg7 | SINE/Alu | 1 | 307 | -5 | 379 | |
| **probe29** | | | | | | | | | | | | | | | 0 |
| **excluded region 26** | 2477 | 7.4 | 0.0 | 0.0 | 278774 | 279071 | -30042 | + | AluY | SINE/Alu | 1 | 298 | -13 | 380 | 6 |
| | 2212 | 9.4 | 0.3 | 5.3 | 279406 | 279724 | -29389 | + | AluSp | SINE/Alu | 1 | 304 | -9 | 381 | |
| | 2283 | 10.4 | 0.3 | 0.0 | 279909 | 280205 | -28908 | + | AluSg | SINE/Alu | 1 | 298 | -12 | 382 | |
| | 2288 | 9.1 | 0.0 | 0.7 | 280216 | 280501 | -28612 | + | AluY | SINE/Alu | 1 | 284 | -27 | 383 | |
| | 235 | 22.6 | 7.0 | 2.2 | 280538 | 280623 | -28490 | + | L1ME4a | LINE/L1 | 5948 | 603 7 | -87 | 384 | |
| | 1552 | 21.2 | 4.2 | 0.3 | 280624 | 280910 | -28203 | C | AluJb | SINE/Alu | -14 | 298 | 1 | 385 | |
| | 2217 | 8.9 | 1.4 | 0.7 | 280919 | 281210 | -27903 | C | AluY | SINE/Alu | -17 | 294 | 1 | 386 | |
| **probe30** | 288 | 7.0 | 0.0 | 0.0 | 281782 | 281824 | -27289 | + | (GGA)n | Simple | 1 | 43 | 0 | 387 | 0 |
| **excluded region 27** | 2005 | 17.0 | 0.0 | 0.0 | 282404 | 282703 | -26410 | C | AluSz6 | SINE/Alu | -11 | 301 | 2 | 388 | 1 |
| **probe31** | | | | | | | | | | | | | | | 0 |
| **excluded region 28** | 2341 | 8.6 | 0.7 | 0.7 | 283434 | 283734 | -25379 | + | AluSx1 | SINE/Alu | 1 | 301 | -11 | 389 | 1 |
| **probe32** | | | | | | | | | | | | | | | 0 |
| **excluded region 29** | 331 | 28.5 | 9.8 | 2.3 | 283817 | 283938 | -25175 | + | MIRb | SINE/MIR | 18 | 148 | -120 | 390 | 0 |
| **probe33** | 328 | 29.2 | 3.2 | 14.3 | 285397 | 285474 | -23639 | + | MIRb | SINE/MIR | 3 | 70 | -198 | 392 | 0 |
| **excluded region 30** | 328 | 29.2 | 3.2 | 14.3 | 285397 | 285474 | -23639 | + | MIRb | SINE/MIR | 3 | 70 | -198 | 392 | 10 |
| | 2457 | 7.7 | 0.0 | 0.3 | 285475 | 285773 | -23340 | C | AluY | SINE/Alu | -13 | 298 | 1 | 393 | |
| | 328 | 29.2 | 3.2 | 14.3 | 285774 | 285818 | -23295 | + | MIRb | SINE/MIR | 71 | 114 | -154 | 392 | |
| | 408 | 34.7 | 8.7 | 2.2 | 285879 | 285923 | -23190 | C | L2c | LINE/L2 | -38 | 334 9 | 3305 | 394 | |
| | 1815 | 17.3 | 0.0 | 3.3 | 285924 | 286070 | -23043 | + | AluJb | SINE/Alu | 1 | 145 | -167 | 395 | |
| | 2404 | 7.7 | 0.3 | 0.3 | 286071 | 286369 | -22744 | + | AluSc5 | SINE/Alu | 1 | 299 | -13 | 396 | |
| | 1815 | 17.3 | 0.0 | 3.3 | 286370 | 286532 | -22581 | + | AluJb | SINE/Alu | 146 | 301 | -11 | 395 | |
| | 408 | 34.7 | 8.7 | 2.2 | 286533 | 286611 | -22502 | C | L2c | LINE/L2 | -83 | 330 4 | 3221 | 394 | |
| | 2426 | 8.9 | 0.0 | 0.0 | 286612 | 286903 | -22210 | + | AluSg | SINE/Alu | 1 | 292 | -18 | 397 | |
| | 408 | 31.6 | 7.5 | 2.4 | 286904 | 287093 | -22020 | C | L2c | LINE/L2 | -167 | 322 0 | 3009 | 394 | |
| | 1897 | 18.1 | 0.0 | 0.3 | 287133 | 287435 | -21678 | + | AluSz6 | SINE/Alu | 1 | 302 | -10 | 398 | |
| | 2477 | 8.5 | 0.7 | 0.0 | 287436 | 287740 | -21373 | + | AluSg | SINE/Alu | 1 | 307 | -3 | 399 | |
| | 236 | 28.4 | 6.8 | 6.1 | 287743 | 287888 | -21225 | C | L2c | LINE/L2 | -495 | 292 4 | 2778 | 394 | |
| | 2425 | 7.2 | 0.7 | 0.0 | 287918 | 288210 | -20903 | + | AluSx4 | SINE/Alu | 5 | 299 | -13 | 400 | |
| | 1966 | 14.8 | 0.0 | 0.7 | 288319 | 288601 | -20512 | + | AluJb | SINE/Alu | 1 | 281 | -31 | 401 | |
| | 198 | 19.2 | 9.4 | 1.8 | 288602 | 288648 | -20465 | C | L2c | LINE/L2 | -823 | 259 6 | 2545 | 394 | |
| | 370 | 33.9 | 7.3 | 3.9 | 288662 | 288761 | -20352 | C | L2c | LINE/L2 | -927 | 249 2 | 2386 | 394 | |
| | 1455 | 18.4 | 8.1 | 5.3 | 288762 | 288900 | -20213 | C | MER2 | DNA/TcMT | -1 | 344 | 212 | 402 | |
| | 1649 | 18.9 | 1.0 | 1.7 | 288901 | 289197 | -19916 | C | AluJr | SINE/Alu | -17 | 295 | 1 | 403 | |
| | 1455 | 18.4 | 8.1 | 5.3 | 289198 | 289390 | -19723 | C | MER2 | DNA/TcMT | -134 | 211 | 3 | 402 | |
| **probe34** | 1455 | 18.4 | 8.1 | 5.3 | 289198 | 289390 | -19723 | C | MER2 | DNA/TcMT | -134 | 211 | 3 | 402 | 0 |
| | 370 | 31.2 | 4.9 | 4.4 | 289391 | 289699 | -19414 | C | L2c | LINE/L2 | - 1034 | 238 5 | 2033 | 394 | |
| | 274 | 29.6 | 20. 4 | 8.6 | 289992 | 290173 | -18940 | C | MIRb | SINE/MIR | -48 | 220 | 16 | 404 | |
| | 254 | 16.1 | 1.4 | 10. 9 | 290149 | 290218 | -18895 | + | MIR | SINE/MIR | 96 | 159 | -103 | 405 | |
| **excluded region 31** | 254 | 16.1 | 1.4 | 10. 9 | 290149 | 290218 | -18895 | + | MIR | SINE/MIR | 96 | 159 | -103 | 405* | 2 |
| | 1998 | 16.9 | 0.0 | 0.3 | 290222 | 290534 | -18579 | + | AluJb | SINE/Alu | 1 | 312 | 0 | 406 | |
| | 2584 | 6.3 | 0.0 | 0.0 | 290614 | 290913 | -18200 | C | AluY | SINE/Alu | -11 | 300 | 1 | 407 | |
| **probe35** | 25 | 76.1 | 0.0 | 0.0 | 291372 | 291417 | -17696 | + | AT_rich | Low_Cplxty | 1 | 46 | 0 | 408 | 0 |
| | 21 | 38.1 | 0.0 | 0.0 | 291399 | 291419 | -17694 | + | AT_rich | Low_Cplxty | 1 | 21 | 0 | 409 | |
| | 228 | 6.7 | 0.0 | 0.0 | 293811 | 293840 | -15273 | + | (CAGCC)n | Simple | 3 | 32 | 0 | 410 | |
| **excluded region 32** | 1075 | 11.7 | 0.0 | 1.4 | 295607 | 295751 | -13362 | + | FLAM_C | SINE/Alu | 1 | 143 | 0 | 411 | 3 |
| | 2297 | 12.3 | 0.0 | 0.3 | 296215 | 296522 | -12591 | + | AluSx1 | SINE/Alu | 1 | 307 | -5 | 412 | |
| | 2261 | 8.2 | 0.7 | 0.0 | 296524 | 296803 | -12310 | + | AluSg | SINE/Alu | 22 | 303 | -7 | 413 | |
| **probe36** | 611 | 31.6 | 6.1 | 1.2 | 296940 | 297170 | -11943 | C | MIRb | SINE/MIR | -1 | 267 | 26 | 414 | 1 |
| | 796 | 17.6 | 2.3 | 0.0 | 299588 | 299718 | -9395 | C | FLAM_C | SINE/Alu | -8 | 135 | 2 | 415 | |
| **excluded region 33** | 2282 | 9.0 | 0.3 | 0.3 | 299917 | 300205 | -8908 | + | AluSq4 | SINE/Alu | 1 | 289 | -23 | 416 | 3 |
| | 1752 | 16.3 | 2.0 | 1.7 | 300991 | 301290 | -7823 | + | AluSz6 | SINE/Alu | 2 | 302 | -10 | 417 | |
| | 2156 | 13.3 | 0.7 | 0.3 | 301631 | 301930 | -7183 | C | AluSz6 | SINE/Alu | -10 | 302 | 2 | 418 | |
| **probe37** | | | | | | | | | | | | | | | 0 |
| **excluded region 34** | 1844 | 12.7 | 7.6 | 0.0 | 303366 | 303641 | -5472 | + | AluSz6 | SINE/Alu | 1 | 297 | -15 | 419 | 6 |
| | 186 | 4.3 | 0.0 | 0.0 | 303712 | 303734 | -5379 | + | (TCTG)n | Simple | 2 | 24 | 0 | 420 | |
| | 1799 | 15.9 | 0.0 | 0.7 | 303735 | 304005 | -5108 | C | AluSx3 | SINE/Alu | -43 | 269 | 1 | 421 | |
| | 1627 | 16. | 0.6 | 8.1 | 304121 | 304299 | -4814 | C | AluJb | SINE/Alu | -3 | 309 | 129 | 422 | |
| | 2369 | 10.8 | 0.3 | 0.0 | 304300 | 304604 | -4509 | C | AluSc | SINE/Alu | -2 | 307 | 2 | 423 | |
| | 1627 | 16.8 | 0.6 | 8.1 | 304605 | 304742 | -4371 | C | AluJb | SINE/Alu | -184 | 128 | 14 | 422 | |
| | 365 | 16.1 | 8.5 | 0.0 | 304786 | 304873 | -4240 | C | FRAM | SINE/Alu | 0 | 133 | 24 | 424 | |
| **probe38** | 219 | 3.6 | 0.0 | 0.0 | 305000 | 305027 | -4086 | + | (CA)n | Simple | 2 | 29 | 0 | 425 | 0 |
| | 201 | 7.4 | 0.0 | 0.0 | 305028 | 305054 | -4059 | + | (TC)n | Simple | 2 | 28 | 0 | 426 | |
| | 262 | 36.0 | 0.0 | 0.0 | 305840 | 305978 | -3135 | + | (TGG)n | Simple | 1 | 139 | 0 | 427 | |
| **aduded region 35** | 980 | 19.5 | 0.0 | 1.2 | 306413 | 306573 | -2540 | C | AluJb | SINE/Alu | -18 | 294 | 134 | 428 | 9 |
| | 1683 | 16.0 | 0.0 | 1.5 | 306574 | 306841 | -2272 | C | AluJr | SINE/Alu | -14 | 298 | 35 | 429 | |
| | 1081 | 16.8 | 6.0 | 8.0 | 306893 | 306924 | -2189 | C | Charlie5 | DNA/hAT-Charlie | -1 | 262 3 | 2600 | 430 | |
| | 2498 | 7.1 | 0.0 | 0.0 | 306925 | 307220 | -1893 | + | AluSg | SINE/Alu | 1 | 296 | -14 | 431 | |
| | 351 | 0.0 | 0.0 | 0.0 | 307222 | 307260 | -1853 | + | (TA)n | Simple | 2 | 40 | 0 | 432 | |
| | 1081 | 16.8 | 6.0 | 8.0 | 307261 | 307290 | -1823 | C | Charlie5 | DNA/hAT-Charlie | -25 | 259 9 | 2574 | 430 | |
| | 2429 | 10.1 | 0.0 | 0.0 | 307291 | 307597 | -1516 | C | AluSg | SINE/Alu | -3 | 307 | 1 | 433 | |
| | 1081 | 16.8 | 6.0 | 8.0 | 307598 | 307634 | -1479 | C | Charlie5 | DNA/hAT-Charlie | -51 | 257 3 | 2537 | 430 | |
| | 1814 | 18.1 | 3.4 | 0.0 | 307635 | 307932 | -1181 | + | AluJr | SINE/Alu | 1 | 308 | -4 | 434 | |
| | 1081 | 16.8 | 6.0 | 8.0 | 307933 | 307957 | -1156 | C | Charlie5 | DNA/hAT-Charlie | -88 | 253 6 | 2509 | 430 | |
| | 1804 | 16.6 | 1.0 | 1.0 | 307958 | 308258 | -855 | C | AluJb | SINE/Alu | -11 | 301 | 1 | 435 | |
| | 1081 | 16.8 | 6.0 | 8.0 | 308259 | 308509 | -604 | C | Charlie5 | DNA/hAT-Charlie | -116 | 250 8 | 2251 | 430 | |
| | 180 | 0.0 | 0.0 | 0.0 | 308538 | 308557 | -556 | + | (TTG)n | Simple | 2 | 21 | 0 | 436 | |
| | 2319 | 9.2 | 0.0 | 0.3 | 308558 | 308843 | -270 | C | AluSx | SINE/Alu | -25 | 287 | 3 | 437 | |
| | 26 | 80.0 | 0.0 | 0.0 | 308875 | 308914 | -199 | + | AT_rich | Low_Cplxty | 1 | 40 | 0 | 438 | |
| | 765 | 15.0 | 4.4 | 0.0 | 308915 | 309027 | -86 | + | AluJo | SINE/Alu | 1 | 118 | -194 | 439 | |
| | 435 | 14.5 | 0.0 | 0.0 | 30905 | 309113 | 0 | C | AluSz6 | SINE/Alu | -13 | 299 | 238 | 440 | |

**Table 4**

| Total Alu sequences in probes | | | | | 11 | (10,5 %) | | | | | | | | | |
|---|---|---|---|---|---|---|---|---|---|---|---|---|---|---|---|
| Total Alu sequences in exlcuded regions | | | | | 93 | (89,4 %) | | | | | | | | | |
| | | | | | **position in query sequence (hg18)** | | | | | | **position in repeat** | | | | |
| | **score** | **%** | **%** | **%** | | | | | **matching** | **repeat** | (left) | end | begi n | **linkag e** | **Alu seq** |
| | | **div.** | **del.** | **ins.** | begin | end | (left) | + | **repeat** | **class/family** | begin | end | (left) | **id** | (count ) |
| **Excluded region 1** | | | | | | | | | | | | | | | 0 |
| **Probe1** | 398 | 34.5 | 9.7 | 1.3 | 240 | 456 | -172044 | C | L3 | LINE/CR1 | -715 | 338 4 | 3150 | 1 | 0 |
| **Excluded region 2** | 2477 | 7.0 | 0.6 | 1.0 | 2534 | 2845 | -169655 | + | AluY | SINE/Alu | 1 | 311 | 0 | 2 | 2 |
| | 2391 | 8.5 | 0.0 | 2.3 | 2948 | 3254 | -169246 | + | AluSg | SINE/Alu | 3 | 302 | -8 | 3 | |
| **Probe2** | 21 | 42.9 | 0.0 | 0.0 | 4058 | 4078 | -168422 | + | AT_rich | Low_complexity | 1 | 21 | 0 | 4 | 0 |
| | 181 | 13.3 | 0.0 | 0.0 | 5187 | 5216 | -167284 | C | L2b | LINE/L2 | -2 | 337 3 | 3344 | 5 | |
| | 21 | 53.6 | 0.0 | 0.0 | 5344 | 5371 | -167129 | + | AT_rich | Low_complexity | 1 | 28 | 0 | 6 | |
| | 25 | 44.0 | 0.0 | 0.0 | 6259 | 6283 | -166217 | + | AT_rich | Low_complexity | 1 | 25 | 0 | 7 | |
| | 36 | 69.4 | 0.0 | 0.0 | 6261 | 6296 | -166204 | + | AT_rich | Low_complexity | 1 | 36 | 0 | 8 | |
| | 300 | 32.4 | 7.6 | 6.2 | 6346 | 6569 | -165931 | C | L2c | LINE/L2 | -139 | 324 8 | 3022 | 9 | |
| **Excluded region 3** | 2134 | 12.3 | 3.6 | 0.3 | 7463 | 7763 | -164737 | C | AluSp | SINE/Alu | -2 | 311 | 1 | 10 | 3 |
| | 4581 | 12.2 | 3.9 | 2.7 | 7764 | 8038 | -164462 | + | Tigger1 | DNA/TcMar-Tigger | 1552 | 182 9 | -589 | 11 | |
| | 2268 | 12.5 | 0.0 | 0.0 | 8039 | 8350 | -164150 | C | AluSz | SINE/Alu | 0 | 312 | 1 | 12 | |
| | 4581 | 12.2 | 3.9 | 2.7 | 8351 | 8579 | -163921 | + | Tigger1 | DNA/TcMar-Tigger | 1830 | 205 2 | -366 | 11 | |
| | 2110 | 12.2 | 0.4 | 0.4 | 8580 | 8896 | -163604 | + | AluSc | SINE/Alu | 1 | 309 | 0 | 13 | |
| | 4581 | 12.6 | 5.9 | 2.5 | 8897 | 9223 | -163277 | + | Tigger1 | DNA/TcMar-Tigger | 2053 | 241 8 | 0 | 11 | |
| **Probe3a** | 4581 | 12.6 | 5.9 | 2.5 | 8897 | 9223 | -163277 | + | Tigger1 | DNA/TcMar-Tigger | 2053 | 241 8 | 0 | 11 | 0 |
| | 722 | 28.2 | 6.0 | 0.9 | 9919 | 10136 | -162364 | C | MIRb | SINE/MIR | -14 | 254 | 26 | 14 | |
| | 566 | 16.8 | 1.6 | 2.4 | 11054 | 11181 | -161319 | + | L1MB8 | UNE/L1 | 6051 | 617 7 | -1 | 15 | |
| | 216 | 15.8 | 0.0 | 0.0 | 11954 | 11991 | -160509 | + | T-rich | Low_complexity | 143 | 180 | 0 | 16 | |
| **Excluded region 4** | | | | | | | | | | | | | | | 0 |
| **Probe3b** | 1039 | 34.0 | 8.2 | 3.8 | 14509 | 15076 | -157424 | C | L2b | LINE/L2 | 0 | 337 5 | 2752 | 17 | 0 |
| | 580 | 10.9 | 8.9 | 0.0 | 15077 | 15177 | -157323 | + | L1MB4 | LINE/L1 | 6070 | 617 9 | -1 | 18 | |
| | 1039 | 29.2 | 11. 7 | 4.9 | 15178 | 15625 | -156875 | C | L2b | LINE/L2 | -668 | 275 1 | 2301 | 17 | |
| | 392 | 34.2 | 7.0 | 0.0 | 15699 | 15856 | -156644 | + | MER5B | DNA/hAT-Charlie | 5 | 173 | -5 | 19 | |
| | 260 | 27.0 | 2.2 | 1.1 | 16498 | 16587 | -155913 | + | MER5B | DNA/hAT-Charlie | 1 | 91 | -87 | 20 | |
| | 356 | 35.0 | 9.7 | 1.8 | 16639 | 17148 | -155352 | + | L2b | LINE/L2 | 687 | 1265 | 2154 | 21 | |
| **Excluded region 5** | 356 | 35.0 | 9.7 | 1.8 | 16639 | 17148 | -155352 | + | L2b | LINE/L2 | 687 | 1265 | 2154 | 21 | 0 |
| **Probe3c** | 582 | 29.9 | 8.9 | 3.0 | 17310 | 18031 | -154469 | + | L2b | LINE/L2 | 1332 | 2163 | 1256 | 21 | 0 |
| | 570 | 21.9 | 5.8 | 0.6 | 18054 | 18209 | -154291 | + | MER5A1 | DNA/hAT-Charlie | 2 | 165 | -1 | 22 | |
| | 615 | 26.7 | 6.3 | 7.5 | 18211 | 18297 | -154203 | + | L2b | LINE/L2 | 2215 | 228 5 | 1134 | 21 | |
| | 463 | 12.4 | 0.0 | 0.0 | 18298 | 18386 | -154114 | C | L1PB1 | LINE/L1 | 0 | 615 1 | 6063 | 23 | |
| | 615 | 26.7 | 6.3 | 7.5 | 18387 | 18553 | -153947 | + | L2b | LINE/L2 | 2286 | 2466 | -953 | 21 | |
| | 616 | 28.0 | 8.3 | 2.9 | 18583 | 18810 | -153690 | C | MIR | SINE/MIR | 0 | 262 | 23 | 24 | |
| | 251 | 27.6 | 7.8 | 4.5 | 18895 | 19023 | -153477 | + | L2b | LINE/L2 | 2618 | 275 0 | -669 | 21 | |
| | 180 | 24.4 | 18.9 | 0.9 | 19184 | 19278 | -153222 | + | L2b | LINE/L2 | 3029 | 3 | -235 | 21 | |
| | 288 | 25.5 | 5.2 | 0.0 | 19430 | 19517 | -152983 | + | MIR | SINE/MIR | 108 | 206 | -62 | 25 | |
| | 409 | 20.3 | 0.9 | 13. 5 | 20554 | 20661 | -151839 | + | MER20 | DNA/hAT-Charlie | 6 | 101 | -118 | 26 | |
| **Excluded region 6** | 2283 | 10.6 | 0.0 | 0.7 | 20878 | 21178 | -151322 | C | AluSx1 | SINE/Alu | -13 | 299 | 1 | 27 | 9 |
| | 2650 | 5.7 | 0.0 | 0.0 | 21294 | 21593 | -150907 | C | AluYk4 | SINE/Alu | -12 | 300 | 1 | 28 | |
| | 411 | 30.1 | 0.0 | 0.0 | 21609 | 21711 | -150789 | C | MIR | SINE/MIR | -2 | 260 | 158 | 29 | |
| | 271 | 27.3 | 6.5 | 0.0 | 21747 | 21823 | -150677 | + | L1MEg | LINE/L1 | 117 | 198 | -6002 | 30 | |
| | 1322 | 24.0 | 7.1 | 2.2 | 21910 | 22707 | -149793 | + | L1MEg | LINE/L1 | 667 | 148 1 | 4719 | 30 | |
| | 2394 | 10.8 | 0.0 | 0.0 | 22717 | 23021 | -149479 | + | AluSx | SINE/Alu | 1 | 305 | -7 | 31 | |
| | 367 | 22.0 | 15.0 | 5.0 | 23105 | 23289 | -149211 | + | L1MEg | LINE/L1 | 1665 | 1878 | 4246 | 30 | |
| | 2251 | 12.5 | 1.6 | 0.0 | 23290 | 23594 | -148906 | + | AluSx1 | SINE/Alu | 1 | 310 | -2 | 32 | |
| | 367 | 23.5 | 14.9 | 3.8 | 23595 | 23754 | -148746 | + | L1MEg | LINE/L1 | 1858 | 203 5 | - 4165 | 30 | |
| | 21 | 66.7 | 0.0 | 0.0 | 23863 | 23883 | -1486177 | + | AT_rich | Low_complexity | 1 | 21 | 0 | 33 | |
| | 2312 | 9.8 | 0.0 | 0.0 | 23884 | 24168 | -148332 | C | AluSg4 | SINE/Alu | -27 | 285 | 1 | 34 | |
| | 354 | 27.4 | 23.6 | 0.1 | 24296 | 24462 | -148038 | + | MIRb | SINE/MIR | 44 | 240 | -28 | 35 | |
| | 2271 | 11.0 | 0.0 | 0.3 | 25061 | 25359 | -147141 | C | AluSq2 | SINE/Alu | -14 | 298 | 1 | 36 | |
| | 204 | 31.0 | 5.5 | 4.3 | 25745 | 25835 | -146665 | + | L2c | LINE/L2 | 3252 | 334 3 | -44 | 37 | |
| | 189 | 38.0 | 1.8 | 2.7 | 26973 | 27083 | -145417 | + | L2 | LINE/L2 | 2741 | 2850 | -569 | 38 | |
| | 3579 | 15.7 | 3.5 | 1.5 | 28391 | 28663 | -143837 | + | L1MA9 | LINE/L1 | 5556 | 582 3 | -489 | 39 | |
| | 2204 | 10.2 | 0.0 | 1.4 | 28664 | 28973 | -143527 | + | AluSx | SINE/Alu | 1 | 312 | 0 | 40 | |
| | 3579 | 15.7 | 3.5 | 1.5 | 28974 | 29408 | -143092 | + | L1MA9 | LINE/L1 | 5824 | 627 9 | -33 | 39 | |
| | 2260 | 11. 5 | 0.0 | 1.9 | 29420 | 29733 | -142767 | C | AluSx | SINE/Alu | -3 | 309 | 2 | 41 | |
| | 388 | 29.1 | 18.1 | 0.4 | 30060 | 30252 | -142248 | + | MIRb | SINE/MIR | 40 | 266 | -2 | 42 | |
| | 2247 | 9.7 | 0.3 | 0.7 | 30637 | 30936 | -141564 | + | AluSp | SINE/Alu | 1 | 299 | -14 | 43 | |
| **Probe5** | 467 | 24.0 | 10.4 | 0.0 | 32206 | 32359 | -140141 | C | MER3 | DNA/hAT-Charlie | -21 | 188 | 19 | 44 | 0 |
| | 637 | 15.5 | 13.4 | 4.7 | 32864 | 32983 | - 139517 | C | Charliela | DNA/hAT-Charlie | 0 | 145 5 | 1322 | 45 | |
| **Excluded region 7** | 637 | 15.5 | 13.4 | 4.7 | 32864 | 32983 | - 139517 | C | Charliela | DNA/hAT-Charlie | 0 | 145 5 | 1322 | 45 | 2 |
| | 2301 | 10.8 | 0.0 | 0.3 | 32984 | 33289 | - 139211 | + | AluSz | SINE/Alu | 1 | 305 | -7 | 46 | |
| | 637 | 16.9 | 15.4 | 3.0 | 33290 | 33571 | - 138929 | C | Charliela | DNA/hAT-Charlie | -134 | 132 1 | 988 | 45 | |
| | 594 | 21.1 | 7.8 | 0.0 | 33607 | 33772 | - 138728 | C | Charliela | DNA/hAT-Charlie | -590 | 865 | 687 | 45 | |
| | 1745 | 21.7 | 7.6 | 1.8 | 33787 | 34341 | - 138159 | C | Charliela | DNA/hAT-Charlie | -804 | 651 | 67 | 45 | |
| | 2280 | 10.4 | 1.0 | 0.0 | 34508 | 34805 | - 137695 | C | AluSc8 | SINE/Alu | -11 | 301 | 1 | 47 | |
| | 25 | 69.2 | 0.0 | 0.0 | 34861 | 34899 | - 137601 | + | AT_rich | Low_complexity | 1 | 39 | 0 | 48 | |
| **Prabe6** | 551 | 28.8 | 9.0 | 2.0 | 35403 | 35590 | - 136910 | + | MIRb | SINE/MIR | 8 | 208 | -60 | 49 | 0 |
| | 346 | 34.6 | 12.2 | 4.0 | 35890 | 36193 | 136307 | C | L2c | LINE/L2 | -79 | 330 8 | 2981 | 50 | |
| | 243 | 37.6 | 5.5 | 5.5 | 36411 | 36666 | 135834 | + | L2c | LINE/L2 | 2910 | 316 5 | -222 | 51 | |
| | 186 | 15.2 | 15.2 | 0.0 | 36661 | 36706 | - 135794 | C | L2a | LINE/L2 | -98 | 3328 | 3276 | 52 | |
| | 278 | 36.5 | 4.1 | 0.8 | 36911 | 37059 | - 135441 | + | MER5B | DNA/hAT-Charlie | 7 | 153 | -25 | 53 | |
| | 232 | 39.2 | 2.9 | 0.0 | 37056 | 37157 | - 135343 | C | L2c | LINE/L2 | -648 | 2771 | 2667 | 50 | |
| | 293 | 29.1 | 12.7 | 9.0 | 37286 | 37553 | - 134947 | C | L2c | LINE/L2 | -2 | 338 5 | 3109 | 54 | |
| | 22 | 59.1 | 0.0 | 0.0 | 37814 | 37835 | - 134665 | + | AT_rich | Low complexity | 1 | 22 | 0 | 55 | |
| | 1767 | 14.8 | 2.6 | 0.3 | 38038 | 38350 | 134150 | C | L1MC2 | LINE/L1 | -158 | 618 6 | 5867 | 56 | |
| | 2581 | 4.4 | 10.9 | 0.0 | 38351 | 38783 | - 133717 | C | MER9a3 | LTR/ERVK | 0 | 512 | 33 | 57 | |
| | 2503 | 12.5 | 5.4 | 0.2 | 38790 | 39214 | -133286 | C | L1MC2 | LINE/L1 | -471 | 587 3 | 5427 | 56 | |
| **Excluded region 8** | 2503 | 12.5 | 5.4 | 0.2 | 38790 | 39214 | -133286 | C | L1MC2 | LINE/L1 | -471 | 587 3 | 5427 | 56 | 1 |
| | 2575 | 6.6 | 0.0 | 0.3 | 39220 | 39520 | -132980 | C | AluY | SINE/Alu | -11 | 300 | 1 | 58 | |
| **Probe7** | 447 | 30.7 | 12.8 | 1.3 | 40106 | 40462 | -132038 | C | L2a | LINE/L2 | 0 | 342 6 | 2972 | 59 | 1 |
| | 1324 | 19.2 | 10.7 | 1.0 | 40694 | 40974 | -131526 | C | AluJr | SINE/Alu | -2 | 310 | 3 | 60 | |
| **Excluded region 9** | 2608 | 5.3 | 1.3 | 0.0 | 41606 | 41907 | - 130593 | C | AluY | SINE/Alu | -5 | 306 | 1 | 61 | 10 |
| | 1898 | 14.0 | 0.4 | 0.0 | 43234 | 43497 | -129003 | + | AluSx | SINE/Alu | 1 | 265 | -47 | 62 | |
| | 2028 | 8.5 | 0.4 | 1.2 | 43498 | 43755 | -128745 | + | AluY | SINE/Alu | 41 | 296 | -15 | 63 | |
| | 1289 | 15.4 | 0.4 | 8.1 | 43837 | 44089 | -128411 | C | AluJb | SINE/Alu | -14 | 298 | 64 | 64 | |
| | 1897 | 13.9 | 0.0 | 0.0 | 44300 | 44565 | - 127935 | C | AluSx1 | SINE/Alu | -2 | 310 | 45 | 65 | |
| | 311 | 17.9 | 0.0 | 1.5 | 44716 | 44783 | -127717 | + | MER53 | DNA/hAT | 12 | 78 | -115 | 66 | |
| | 491 | 14.9 | 0.0 | 1.1 | 44783 | 44870 | -127630 | + | MER53 | DNA/hAT | 107 | 193 | 0 | 67 | |
| | 480 | 14.4 | 4.8 | 11.0 | 45770 | 45894 | -126606 | C | MER44D | DNA/TcMar-Tigger | -2 | 703 | 586 | 68 | |
| | 1057 | 7.7 | 1.6 | 2.7 | 45879 | 46064 | -126436 | C | MER44D | DNA/TcMar-Tigger | -79 | 626 | 444 | 68 | |
| | 2405 | 12.7 | 5.6 | 1.2 | 46064 | 46728 | -125772 | C | Tigger7 | DNA/TcMar-Tigger | - 1653 | 838 | 145 | 69 | |
| | 919 | 18.1 | 0.0 | 0.0 | 46776 | 46930 | -125570 | C | MER44D | DNA/TcMar-Tigger | -549 | 156 | 2 | 68 | |
| | 1210 | 14.2 | 11.8 | 0.8 | 47131 | 47342 | -125158 | C | AluSx | SINE/Alu | 0 | 312 | 78 | 70 | |
| | 967 | 18.1 | 0.0 | 0.0 | 47500 | 47648 | - 124852 | + | AluJb | SINE/Alu | 152 | 300 | -12 | 71 | |
| | 208 | 22.0 | 1.1 | 6.0 | 47867 | 47953 | - 124547 | + | (TATG)n | Simple_repeat | 3 | 85 | 0 | 72 | |
| | 4691 | 7.6 | 0.2 | 0.6 | 49683 | 50307 | - 122193 | C | L1PA10 | LINE/L1 | -11 | 615 7 | 5536 | 73 | |
| | 1758 | 20.7 | 0.7 | 0.0 | 50462 | 50766 | - 121734 | + | AluJr4 | SINE/Alu | 1 | 307 | -5 | 74 | |
| | 2343 | 10.9 | 0.0 | 0.3 | 51130 | 51431 | - 121069 | + | AluSz | SINE/Alu | 1 | 301 | -11 | 75 | |
| | 1741 | 18.6 | 1.4 | 0.3 | 51949 | 52244 | -120256 | C | AluJo | SINE/Alu | -9 | 303 | 5 | 76 | |
| **Probe11** | | | | | | | | | | | | | | | 0 |
| **Excluded region 10** | 2443 | 0.4 | 0.0 | 0.8 | 57693 | 57950 | 114550 | + | AluYa5 | SINE/Alu | 41 | 296 | -14 | 77 | 3 |
| | 203 | 29.1 | 9.0 | 3.8 | 57957 | 58056 | 114444 | + | MIRc | SINE/MIR | 63 | 167 | -101 | 78 | |
| | 2301 | 9.7 | 1.0 | 0.3 | 58059 | 58356 | 114144 | + | AluSx | SINE/Alu | 1 | 300 | -12 | 79 | |
| | 219 | 18.6 | 3.1 | 15.8 | 58361 | 58424 | 114076 | + | MIR | SINE/MIR | 200 | 256 | -6 | 80 | |
| | 1903 | 12.7 | 4.4 | 9.5 | 58558 | 58831 | 113669 | C | Tigger3a | DNA/TcMar-Tigger | 0 | 348 | 61 | 81 | |
| | 2336 | 9.7 | 0.0 | 1.0 | 58832 | 59130 | 113370 | + | AluSx | SINE/Alu | 1 | 296 | -16 | 82 | |
| | 1903 | 12.7 | 4.4 | 9.5 | 59131 | 59220 | 113280 | C | Tigger3a | DNA/TcMar-Tigger | -288 | 60 | 1 | 81 | |
| **Probe12** | 1903 | 12.7 | 4.4 | 9.5 | 59131 | 59220 | 113280 | C | Tigger3a | DNA/TcMar-Tigger | -288 | 60 | 1 | 81 | 1 |
| | 270 | 39.8 | 0.0 | 0.0 | 60002 | 60119 | - 112381 | + | L4 | LINE/RTE-X | 1467 | 158 4 | -445 | 83 | |
| | 180 | 11.1 | 0.0 | 0.0 | 60235 | 60261 | 112239 | + | (A)n | Simple_repeat | 1 | 27 | 0 | 84 | |
| | 474 | 10.8 | 9.2 | 0.0 | 60778 | 60842 | 111658 | C | AluSq10 | SINE/Alu | -236 | 76 | 6 | 85 | |
| | 612 | 13.2 | 0.9 | 0.0 | 60849 | 60962 | 111538 | C | Charliela | DNA/hAT-Charlie | -26 | 142 9 | 1315 | 86 | |
| | 1915 | 18.2 | 4.9 | 0.7 | 60965 | 61374 | 111126 | C | Charliela | DNA/hAT-Charlie | -617 | 838 | 412 | 86 | |
| | 321 | 29.3 | 5.9 | 2.1 | 61403 | 61538 | 110962 | C | Charliela | DNA/hAT-Charlie | 1314 | 141 | 1 | 86 | |
| | 1905 | 12.3 | 7.7 | 1.4 | 61652 | 61988 | 110512 | C | Tigger4b | DNA/TcMar-Tigger | -1 | 360 | 3 | 87 | |
| | 656 | 22.7 | 6.7 | 8.5 | 62213 | 62511 | - 109989 | C | LlMC4a | LINE/L1 | 1844 | 6038 | 5745 | 88 | |
| | 309 | 32.5 | 6.3 | 3.3 | 63088 | 63262 | 109238 | C | MIRc | SINE/MIR | -19 | 249 | 70 | 89 | |
| | 307 | 26.2 | 21.7 | 1.0 | 63277 | 63442 | 109058 | + | HAL1 | LINE/L1 | 42 | 241 | 2266 | 90 | |
| | 820 | 26.3 | 16.0 | 3.2 | 63465 | 64265 | 108235 | + | HAL1 | LINE/L1 | 271 | 117 2 | 1335 | 90 | |
| | 744 | 23.8 | 8.6 | 6.5 | 64278 | 64682 | 107818 | + | HAL1 | LINE/L1 | 1215 | 162 7 | -880 | 90 | |
| | 646 | 29.9 | 9.2 | 1.7 | 64710 | 64981 | - 107519 | + | HAL1 | LINE/L1 | 1667 | 195 8 | -549 | 90 | |
| **Excluded region 11** | 646 | 29.9 | 9.2 | 1.7 | 64710 | 64981 | 107519 | + | HAL1 | LINE/L1 | 1667 | 195 8 | -549 | 90 | 4 |
| | 2221 | 11.7 | 2.0 | 0.0 | 65009 | 65307 | 107193 | + | AluSz6 | SINE/Alu | 1 | 305 | -7 | 91 | |
| | 741 | 28.5 | 17.7 | 5.0 | 65308 | 65642 | 106858 | + | HAL1 | LINE/L1 | 15 | 396 | 2111 | 92 | |
| | 1932 | 12.4 | 0.4 | 0.0 | 65643 | 65900 | 106600 | + | AluSx | SINE/Alu | 42 | 300 | -12 | 93 | |
| | 741 | 25.5 | 7.2 | 8.2 | 65901 | 66135 | 106365 | + | HAL1 | LINE/L1 | 397 | 625 | 1882 | 92 | |
| | 513 | 26.8 | 6.3 | 2.2 | 66162 | 66382 | 106118 | + | HAL1 | LINE/L1 | 743 | 972 | 1535 | 92 | |
| | 226 | 27.4 | 8.6 | 9.6 | 66385 | 66535 | 105965 | + | HAL1 | LINE/L1 | 1945 | 209 4 | -413 | 92 | |
| | 2516 | 7.3 | 0.0 | 1.3 | 66536 | 66850 | 105650 | + | AluY | SINE/Alu | 1 | 311 | 0 | 94 | |
| | 226 | 27.4 | 8.6 | 9.6 | 66851 | 66926 | 105574 | + | HAL1 | LINE/L1 | 2095 | 216 6 | -341 | 92 | |
| | 4820 | 10.2 | 2.1 | 0.0 | 66927 | 67600 | 104900 | + | LTR12_ | LTR/ERV1 | 1 | 688 | 0 | 95 | |
| | 226 | 27.4 | 8.6 | 9.6 | 67601 | 67698 | 104802 | + | HAL1 | LINE/L1 | 2167 | 2268 | -239 | 92 | |
| | 2139 | 11.2 | 0.0 | 0.0 | 67853 | 68168 | 104332 | C | AluY | SINE/Alu | 0 | 311 | 2 | 96 | |
| **Probe13** | 460 | 25.0 | 6.8 | 1.9 | 69115 | 69261 | 103239 | + | L2a | LINE/L2 | 1657 | 181 0 | 1609 | 97 | 0 |
| | 850 | 28.6 | 3.9 | 2.3 | 69391 | 69648 | 102852 | + | L2a | LINE/L2 | 2735 | 299 6 | -423 | 97 | |
| | 345 | 23.9 | 19.3 | 1.4 | 69670 | 69788 | 102712 | + | L2a | LINE/L2 | 3286 | 342 5 | -1 | 97 | |
| | 327 | 31.5 | 8.0 | 3.0 | 69875 | 70100 | 102400 | C | L2 | LINE/L2 | -923 | 249 6 | 2260 | 98 | |
| **Excluded region 12** | 2153 | 8.9 | 2.0 | 1.0 | 71648 | 71776 | 100724 | + | AluSx | SINE/Alu | 1 | 129 | -183 | 99 | 3 |
| | 225 | 0.0 | 0.0 | 0.0 | 71777 | 71801 | 100699 | + | (TAAA)n | Simple_repeat | 2 | 26 | 0 | 100 | |
| | 2153 | 8.9 | 2.0 | 1.0 | 71802 | 71965 | 100535 | + | AluSx | SINE/Alu | 130 | 296 | -16 | 99 | |
| | 2223 | 8.1 | 0.0 | 9.2 | 72116 | 72437 | 100063 | C | AluSp | SINE/Alu | -18 | 295 | 1 | 101 | |
| **Probe14** | 967 | 25.5 | 2.0 | 3.7 | 73109 | 73356 | -99144 | C | MIR | SINE/MIR | -2 | 260 | 17 | 102 | 0 |
| **Excluded region 13** | 2433 | 9.2 | 0.0 | 0.3 | 74262 | 74565 | -97935 | + | AluSx1 | SINE/Alu | 1 | 303 | -9 | 103 | 5 |
| | 1011 | 11.4 | 0.0 | 0.7 | 74578 | 74717 | -97783 | + | AluJb | SINE/Alu | 1 | 139 | -173 | 104 | |
| | 2204 | 12.2 | 0.0 | 0.3 | 74720 | 75007 | -97493 | + | AluSx | SINE/Alu | 2 | 288 | -24 | 105 | |
| | 2390 | 11.0 | 0.7 | 0.0 | 75008 | 75315 | -97185 | + | AluSx | SINE/Alu | 1 | 310 | -2 | 106 | |
| | 1873 | 27.2 | 6.0 | 3.0 | 75901 | 76439 | -96061 | C | L2a | LINE/L2 | -8 | 341 8 | 2826 | 107 | |
| | 2284 | 9.4 | 1.4 | 0.0 | 76440 | 76725 | -95775 | C | AluSx | SINE/Alu | -22 | 290 | 1 | 108 | |
| | 1873 | 25.9 | 6.3 | 2.2 | 76726 | 77867 | -94633 | C | L2a | LINE/L2 | -594 | 282 5 | 1505 | 107 | |
| **Probe15** | 1873 | 25.9 | 6.3 | 2.2 | 76726 | 77867 | -94633 | C | L2a | LINE/L2 | -594 | 282 5 | 1505 | 107 | 1 |
| | 24 | 54.8 | 0.0 | 0.0 | 77993 | 78023 | -94477 | + | AT_rich | Low_complexity | 1 | 31 | 0 | 109 | |
| | 1987 | 14.5 | 0.7 | 2.3 | 78087 | 78396 | -94104 | C | AluJr | SINE/Alu | -6 | 306 | 2 | 110 | |
| | 654 | 26.9 | 11.1 | 3.8 | 80306 | 80775 | -91725 | C | HAL1 | LINE/L1 | -1 | 250 6 | 2003 | 111 | |
| | 366 | 24.7 | 22.2 | 0.4 | 80915 | 81145 | -91355 | C | HAL1 | LINE/L1 | -698 | 180 9 | 1529 | 111 | |
| **Excluded region 14** | 366 | 24.7 | 22.2 | 0.4 | 80915 | 81145 | -91355 | C | HAL1 | LINE/L1 | -698 | 180 9 | 1529 | 111 | 15 |
| | 362 | 14.3 | 0.0 | 0.0 | 81186 | 81241 | -91259 | C | AluJo | SINE/Alu | -10 | 302 | 247 | 112 | |
| | 810 | 18.7 | 0.0 | 0.0 | 81247 | 81369 | -91131 | C | AluJo | SINE/Alu | -189 | 123 | 1 | 113 | |
| | 2337 | 10.8 | 1.0 | 0.0 | 81439 | 81745 | -90755 | C | AluSq2 | SINE/Alu | -2 | 310 | 1 | 114 | |
| | 222 | 12.8 | 0.0 | 0.0 | 81790 | 81828 | -90672 | + | (T)n | Simple_repeat | 1 | 39 | 0 | 115 | |
| | 645 | 22.8 | 3.0 | 3.0 | 81861 | 82095 | -90405 | C | HAL1 | LINE/L1 | 1173 | 133 4 | 1100 | 111 | |
| | 2246 | 12.8 | 0.0 | 0.0 | 82608 | 82904 | -89596 | + | AluSz | SINE/Alu | 1 | 297 | -15 | 116 | |
| | 870 | 26.0 | 8.8 | 4.5 | 82945 | 83220 | -89280 | + | L1MC5 | LINE/L1 | 6652 | 691 5 | 1046 | 117 | |
| | 2237 | 11.4 | 0.0 | 0.7 | 83221 | 83518 | -88982 | + | AluSx1 | SINE/Alu | 1 | 296 | -16 | 118 | |
| | 870 | 26.0 | 8.8 | 4.5 | 83519 | 83591 | -88909 | + | L1MC5 | LINE/L1 | 6916 | 700 7 | -954 | 117 | |
| | 1689 | 17.8 | 3.1 | 2.0 | 83592 | 83884 | -88616 | + | AluJb | SINE/Alu | 3 | 298 | -14 | 119 | |
| | 870 | 23.0 | 4.9 | 4.9 | 83885 | 84043 | -88457 | + | L1MC5 | LINE/L1 | 7008 | 718 7 | -774 | 117 | |
| | 2385 | 8.7 | 0.0 | 0.3 | 84076 | 84374 | -88126 | C | AluSx3 | SINE/Alu | -1 | 311 | 14 | 120 | |
| | 361 | 24.7 | 11.5 | 6.8 | 84442 | 84667 | -87833 | C | HAL1 | LINE/L1 | 1433 | 107 4 | 839 | 111 | |
| | 2526 | 7.4 | 0.3 | 0.0 | 84867 | 85175 | -87325 | C | AluSg4 | SINE/Alu | -2 | 310 | 1 | 121 | |
| | 524 | 30.4 | 1.8 | 0.6 | 85327 | 85495 | -87005 | C | HAL1 | LINE/L1 | 2066 | 441 | 271 | 111 | |
| | 510 | 25.4 | 7.2 | 6.6 | 85541 | 85640 | -86860 | + | MIR | SINE/MIR | 78 | 186 | -76 | 122 | |
| | 2302 | 10.3 | 0.0 | 0.0 | 85641 | 85941 | -86559 | C | AluSxl | SINE/Alu | -11 | 301 | 1 | 123 | |
| | 510 | 25.4 | 7.2 | 6.6 | 85942 | 86021 | -86479 | + | MIR | SINE/MIR | 187 | 259 | -3 | 122 | |
| | 1959 | 12.4 | 5.7 | 0.0 | 86679 | 86960 | -85540 | C | AluSq2 | SINE/Alu | -14 | 298 | 1 | 124 | |
| | 3783 | 12.4 | 2.8 | 0.3 | 87785 | 88389 | -84111 | C | Tigger1 | DNA/TcMar-Tigger | 0 | 241 8 | 1799 | 125 | |
| | 2326 | 9.8 | 6.7 | 0.8 | 88390 | 88749 | -83751 | C | THE1D | LTR/ERVL-MaLR | 0 | 381 | 1 | 126 | |
| | 6464 | 20.4 | 3.7 | 4.3 | 88750 | 89064 | -83436 | C | THE1D-int | LTR/ERVL-MaLR | 0 | 165 1 | 1336 | 126 | |
| | 1687 | 11.7 | 0.4 | 0.4 | 89065 | 89294 | -83206 | C | AluSz6 | SINE/Alu | -16 | 296 | 67 | 127 | |
| | 2204 | 13.9 | 0.0 | 0.0 | 89295 | 89603 | -82897 | + | AluSg | SINE/Alu | 2 | 310 | 0 | 128 | |
| | 6464 | 20.4 | 3.7 | 4.3 | 89604 | 90942 | -81558 | C | THE1D-int | LTR/ERVL-MaLR | -316 | 133 5 | 5 | 126 | |
| | 2155 | 11.9 | 7.3 | 1.1 | 90947 | 91303 | -81197 | C | THE1D | LTR/ERVL-MaLR | 0 | 381 | 3 | 126 | |
| | 2716 | 11.2 | 3.1 | 1.9 | 91308 | 91627 | -80873 | C | Tigger1 | DNA/TcMar-Tigger | -617 | 180 1 | 1473 | 125 | |
| | 2474 | 7.4 | 0.3 | 0.0 | 91628 | 91926 | -80574 | C | AluSp | SINE/Alu | -12 | 301 | 2 | 129 | |
| | 2716 | 11.2 | 3.1 | 1.9 | 91927 | 92061 | -80439 | C | Tigger1 | DNA/TcMar-Tigger | -946 | 147 2 | 1341 | 125 | |
| | 691 | 18.9 | 2.0 | 4.8 | 92060 | 92209 | -80291 | C | Tigger1 | DNA/TcMar-Tigger | - 2271 | 147 | 2 | 130 | |
| | 2112 | 13.6 | 0.7 | 0.3 | 92309 | 92610 | -79890 | + | AluSz | SINE/Alu | 1 | 303 | -9 | 131 | |
| | 23 | 65.2 | 0.0 | 0.0 | 93071 | 93093 | -79407 | + | AT_rich | Low_complexity | 1 | 23 | 0 | 132 | |
| | 259 | 25.2 | 8.8 | 1.4 | 93163 | 93299 | -79201 | + | Charlie 16a | DNA/hAT-Charlie | 195 | 341 | -1 | 133 | |
| | 2340 | 9.7 | 0.7 | 0.0 | 93378 | 93675 | -78825 | + | AluSq2 | SINE/Alu | 1 | 300 | -12 | 134 | |
| | | | | | | | | | | | | | | | |
| | | | | | | | | | | | | | | | |
| | | | | | | | | | | | | | | | |
| **Probe** 18 | 202 | 33.9 | 10. 4 | 2.4 | 94305 | 94419 | -78081 | + | MIR3 | SINE/MIR | 82 | 205 | -3 | 135 | 0 |
| | 206 | 12.9 | 0.0 | 0.0 | 94740 | 94770 | -77730 | + | (TTTA)n | Simple_repeat | 2 | 32 | 0 | 136 | |
| | 615 | 27.6 | 3.3 | 3.8 | 94907 | 95117 | -77383 | + | MIR | SINE/MIR | 34 | 243 | -19 | 137 | |
| **Excluded region 15** | 323 | 25.3 | 7.1 | 7.8 | 96452 | 96602 | -75898 | C | HAL1b | LINE/L1 | 1336 | 673 | 523 | 138 | 1 |
| | 2395 | 10.5 | 0.0 | 0.0 | 96603 | 96907 | -75593 | C | AluY | SINE/Alu | -6 | 305 | 1 | 139 | |
| | 323 | 25.3 | 7.1 | 7.8 | 96908 | 97051 | -75449 | C | HAL1b | LINE/L1 | 1487 | 522 | 380 | 138 | |
| **Probe19** | 323 | 25.3 | 7.1 | 7.8 | 96908 | 97051 | -75449 | C | HAL1b | LINE/L1 | 1487 | 522 | 380 | 138 | 1 |
| | 1346 | 25.5 | 13.0 | 3.7 | 97232 | 97965 | -74535 | C | L2a | LINE/L2 | -1 | 342 5 | 2625 | 140 | |
| | 795 | 20.8 | 10. 2 | 0.0 | 97979 | 98175 | -74325 | C | L2a | LINE/L2 | -869 | 2550 | 2334 | 140 | |
| | 1175 | 5.3 | 0.0 | 0.0 | 98188 | 98319 | -74181 | C | AluY | SINE/Alu | -179 | 132 | 1 | 141 | |
| | 957 | 25.0 | 3.7 | 5.0 | 98323 | 98646 | -73854 | C | L2a | LINE/L2 | 1091 | 2328 | 2009 | 140 | |
| | 1822 | 28.0 | 5.5 | 2.8 | 98660 | 99147 | -73353 | C | L2a | LINE/L2 | 1465 | 195 4 | 1460 | 140 | |
| **Excluded region 16** | 1822 | 28.0 | 5.5 | 2.8 | 98660 | 99147 | -73353 | C | L2a | LINE/L2 | 1465 | 195 4 | 1460 | 140 | 1 |
| | 2307 | 7.8 | 3.8 | 0.0 | 99148 | 99440 | -73060 | + | AluY | SINE/Alu | 1 | 304 | -7 | 142 | |
| | 1822 | 28.8 | 8.3 | 1.8 | 99441 | 100520 | -71980 | C | L2a | LINE/L2 | - 1960 | 145 9 | 259 | 140 | |
| **Probe20** | 1822 | 28.8 | 8.3 | 1.8 | 99441 | 100520 | -71980 | C | L2a | LINE/L2 | - 1960 | 145 9 | 259 | 140 | 0 |
| | 229 | 9.1 | 0.0 | 0.0 | 10054 0 | 100583 | -71917 | C | L1MA1 | LINE/L1 | 0 | 630 2 | 6259 | 143 | |
| **Excluded region 17** | 1871 | 12.6 | 0.0 | 0.0 | 10223 7 | 102490 | -70010 | + | AluSx | SINE/Alu | 44 | 297 | -15 | 144 | 1 |
| **Probe21** | 236 | 24.6 | 4.5 | 2.9 | 10276 1 | 102827 | -69673 | C | HAL1b | LINE/L1 | - 1785 | 224 | 157 | 138 | 0 |
| | 1602 | 16.4 | 3.7 | 0.3 | 102909 | 103217 | -69283 | C | MLT1C | LTR/ERVL-MaLR | -19 | 448 | 130 | 145 | |
| | 7752 | 5.3 | 1.0 | 0.2 | 103218 | 104175 | -68325 | + | LTR13A | LTR/ERVK | 1 | 966 | 0 | 146 | |
| **Excluded region 18** | 7752 | 5.3 | 1.0 | 0.2 | 103218 | 104175 | -68325 | + | LTR13A | LTR/ERVK | 1 | 966 | 0 | 146 | 1 |
| | 1602 | 16.4 | 3.7 | 0.3 | 104176 | 104189 | -68311 | C | MLT1C | LTR/ERVL-MaLR | -338 | 129 | 115 | 145 | |
| | 1941 | 15.5 | 0.3 | 0.7 | 104190 | 104485 | -68015 | C | AluSx3 | SINE/Alu | -16 | 296 | 2 | 147 | |
| | 1279 | 12.0 | 10. 2 | 1.1 | 104490 | 104734 | -67766 | + | MER47A | DNA/TcMar-Tigger | 30 | 296 | -70 | 148 | |
| **Probe 22a** | 1279 | 12.0 | 10. 2 | 1.1 | 104490 | 104734 | -67766 | + | MER47A | DNA/TcMar-Tigger | 30 | 296 | -70 | 148 | 1 |
| | 1976 | 26.4 | 3.6 | 4.5 | 104810 | 105732 | -66768 | C | L1MDa | LINE/L1 | - 3919 | 269 9 | 1780 | 149 | |
| | 298 | 16.3 | 0.0 | 0.0 | 105741 | 105789 | -66711 | + | MER47A | DNA/TcMar-Tigger | 307 | 355 | -11 | 150 | |
| | 181 | 32.9 | 3.5 | 2.3 | 106217 | 106303 | -66197 | + | L2 | LINE/L2 | 2804 | 289 1 | -528 | 151 | |
| | 667 | 17.2 | 9.0 | 0.0 | 106378 | 106499 | -66001 | + | AluJr | SINE/Alu | 1 | 133 | -179 | 152 | |
| | 584 | 28.8 | 7.0 | 1.0 | 106933 | 107118 | -65382 | C | MIRb | SINE/MIR | -63 | 205 | 9 | 153 | |
| | 979 | 25.1 | 18.2 | 0.2 | 107288 | 107655 | -64845 | C | LTR16 | LTR/ERVL | -4 | 434 | 1 | 154 | |
| **Ecluded region 19** | | | | | | | | | | | | | | | 0 |
| **Probe 22b** | 850 | 11.8 | 48.0 | 1.0 | 108472 | 108675 | -63825 | + | AluSz | SINE/Alu | 1 | 300 | -12 | 155 | 1 |
| | 2071 | 22.6 | 7.5 | 3.2 | 108679 | 109832 | -62668 | C | LlMC4a | LINE/L1 | -5 | 787 7 | 6672 | 156 | |
| | 1300 | 27.4 | 6.7 | 5.3 | 109826 | 110557 | -61943 | C | LlMC4a | LINE/L1 | - 1660 | 622 2 | 5481 | 156 | |
| | 503 | 25.1 | 17.0 | 0.4 | 111505 | 111716 | -60784 | C | MIR | SINE/MIR | -14 | 248 | 2 | 157 | |
| | 26 | 76.9 | 0.0 | 0.0 | 111823 | 111848 | -60652 | + | AT_rich | Low_complexity | 1 | 26 | 0 | 158 | |
| | 25 | 48.0 | 0.0 | 0.0 | 111826 | 111850 | -60650 | + | AT_rich | Low_complexity | 1 | 25 | 0 | 159 | |
| **Excluded region 20** | 2266 | 11.9 | 0.0 | 0.7 | 112029 | 112338 | -60162 | C | AluSz6 | SINE/Alu | -1 | 311 | 4 | 160 | 5 |
| | 434 | 30.8 | 9.8 | 1.8 | 112397 | 112439 | -60061 | C | MIRc | SINE/MIR | -18 | 250 | 211 | 161 | |
| | 347 | 21.8 | 1.3 | 0.0 | 112440 | 112517 | -59983 | + | MADE2 | DNA/TcMar-Mariner | 1 | 79 | -1 | 162 | |
| | 434 | 30.8 | 9.8 | 1.8 | 112518 | 112678 | -59822 | C | MIRc | SINE/MIR | -58 | 210 | 30 | 161 | |
| | 709 | 17.2 | 7.0 | 5.1 | 113509 | 113565 | -58935 | C | MIR | SINE/MIR | -48 | 214 | 158 | 163 | |
| | 1081 | 17.9 | 1.0 | 2.0 | 113566 | 113770 | -58730 | C | MER6B | DNA/TcMar-Tigger | -3 | 207 | 5 | 164 | |
| | 709 | 17.2 | 7.0 | 5.1 | 113771 | 113884 | -58616 | C | MIR | SINE/MIR | -105 | 157 | 40 | 163 | |
| | 922 | 13.4 | 0.0 | 0.8 | 115087 | 115220 | -57280 | + | FLAM_C | SINE/Alu | 1 | 133 | -10 | 165 | |
| | 2194 | 12.4 | 0.0 | 0.3 | 115855 | 116153 | -56347 | C | AluSx | SINE/Alu | -14 | 298 | 1 | 166 | |
| | 21 | 52.4 | 0.0 | 0.0 | 116662 | 116682 | -55818 | + | AT_rich | Low_complexity | 1 | 21 | 0 | 167 | |
| | 228 | 22.7 | 0.0 | 0.0 | 118269 | 118312 | -54188 | C | MARNA | DNA/TcMar-Mariner | -263 | 323 | 280 | 168 | |
| | 334 | 29.6 | 11.7 | 2.5 | 118335 | 118514 | -53986 | C | MARNA | DNA/TcMar-Mariner | -358 | 228 | 33 | 168 | |
| | 258 | 28.7 | 4.7 | 4.7 | 119667 | 119816 | -52684 | C | MER5A1 | DNA/hAT-Charlie | -7 | 159 | 10 | 169 | |
| | 2160 | 12.5 | 0.0 | 0.0 | 121296 | 121598 | -50902 | + | AluSz6 | SINE/Alu | 1 | 303 | -9 | 170 | |
| | 2590 | 4.8 | 0.3 | 2.6 | 121961 | 122276 | -50224 | C | AluY | SINE/Alu | -2 | 309 | 1 | 171 | |
| | 2312 | 9.6 | 0.3 | 1.0 | 122525 | 122837 | -49663 | C | AluSq2 | SINE/Alu | -1 | 311 | 1 | 172 | |
| **Probe25** | 383 | 25.5 | 1.0 | 1.0 | 124840 | 124938 | -47562 | + | L3 | LINE/CR1 | 2392 | 249 0 | 1609 | 173 | 0 |
| | 314 | 31.5 | 4.2 | 0.7 | 124992 | 125135 | -47365 | + | MIRc | SINE/MIR | 119 | 267 | -1 | 174 | |
| | 347 | 26.4 | 16. 3 | 1.0 | 125363 | 125534 | -46966 | + | L3 | LINE/CR1 | 2843 | 304 0 | 1059 | 173 | |
| | 274 | 30.5 | 0.9 | 3.8 | 125573 | 125681 | -46819 | C | L2c | LINE/L2 | -15 | 337 2 | 3267 | 175 | |
| | 501 | 32.6 | 2.8 | 3.6 | 125939 | 126189 | -46311 | + | L3 | LINE/CR1 | 3577 | 382 5 | -274 | 173 | |
| | 399 | 25.0 | 5.7 | 0.2 | 126418 | 126549 | -45951 | C | MLT1H1 | LTR/ERVL-Ma LR | -368 | 181 | 1 | 176 | |
| | 24 | 45.8 | 0.0 | 0.0 | 127392 | 127415 | -45085 | + | AT_rich | Low_complexity | 1 | 24 | 0 | 177 | |
| | 283 | 26.2 | 12.5 | 0.9 | 127944 | 128047 | -44453 | C | L1MC5 | LINE/L1 | -36 | 792 5 | 7810 | 178 | |
| | 327 | 26.4 | 0.0 | 0.0 | 128140 | 128230 | -44270 | C | L1MC5 | LINE/L1 | -396 | 756 5 | 7475 | 178 | |
| **Excluded region 21** | 327 | 26.4 | 0.0 | 0.0 | 128140 | 128230 | -44270 | C | L1MC5 | LINE/L1 | -396 | 756 5 | 7475 | 178 | 3 |
| | 504 | 29.0 | 6.4 | 3.1 | 128273 | 128412 | -44088 | C | L1MC4 | LINE/L1 | -20 | 802 2 | 7869 | 179 | |
| | 2235 | 10.0 | 0.3 | 4.5 | 128413 | 128733 | -43767 | + | AluSz6 | SINE/Alu | 1 | 308 | -4 | 180 | |
| | 504 | 29.0 | 6.4 | 3.1 | 128734 | 128841 | -43659 | C | L1MC4 | LINE/L1 | -174 | 786 8 | 7766 | 179 | |
| | 27 | 40.7 | 0.0 | 0.0 | 128958 | 128984 | -43516 | + | AT_rich | Low_complexity | 1 | 27 | 0 | 181 | |
| | 2216 | 10.3 | 0.0 | 0.7 | 129002 | 129293 | -43207 | C | AluSx1 | SINE/Alu | -22 | 290 | 1 | 182 | |
| | 26 | 69.2 | 0.0 | 0.0 | 129304 | 129329 | -43171 | + | AT_rich | Low_complexity | 1 | 26 | 0 | 183 | |
| | 716 | 29.2 | 6.6 | 2.7 | 129439 | 129758 | -42742 | C | L1MC4 | LINE/L1 | -495 | 754 7 | 7216 | 179 | |
| | 284 | 25.5 | 7.7 | 12. 0 | 129803 | 129944 | -42556 | C | L1ME4a | LINE/L1 | -90 | 603 4 | 5888 | 184 | |
| | 2477 | 8.5 | 0.0 | 0.0 | 129945 | 130249 | -42251 | C | AluSx | SINE/Alu | -7 | 305 | 1 | 185 | |
| | 284 | 25.5 | 7.7 | 12. 0 | 130250 | 130445 | -42055 | C | LlME4a | LINE/L1 | -237 | 588 7 | 5710 | 184 | |
| **Probe26** | 348 | 38.5 | 0.5 | 2.2 | 130725 | 130910 | -41590 | C | MIRb | SINE/MIR | -35 | 233 | 51 | 186 | 0 |
| | 494 | 23.5 | 3.3 | 1.6 | 130919 | 131039 | -41461 | C | L1M6 | LINE/L1 | - 4691 | 180 5 | 1683 | 187 | |
| | 379 | 28.8 | 9.6 | 4.4 | 131119 | 131336 | -41164 | C | MLT1J | LTR/ERVL-Ma LR | -48 | 464 | 236 | 188 | |
| | 22 | 63.6 | 0.0 | 0.0 | 131455 | 131476 | -41024 | + | AT_rich | Low_complexity | 1 | 22 | 0 | 189 | |
| | 559 | 27.4 | 4.7 | 5.1 | 131889 | 132146 | -40354 | + | L2a | LINE/L2 | 3170 | 3426 | 0 | 190 | |
| | 350 | 23.1 | 2.6 | 0.0 | 132152 | 132229 | -40271 | C | L1ME5 | UNE/L1 | -321 | 5873 | 5794 | 191 | |
| | 443 | 28.0 | 21. 4 | 3.8 | 132249 | 132461 | -40039 | C | MIR | SINE/MIR | -4 | 258 | 8 | 192 | |
| | 269 | 25.0 | 12. 0 | 0.7 | 132474 | 132606 | -39894 | C | L1M5 | UNE/L1 | -339 | 5784 | 5637 | 193 | |
| | 582 | 25.6 | 0.8 | 0.0 | 132696 | 132828 | -39672 | + | L2a | LINE/L2 | 3293 | 3426 | 0 | 194 | |
| **Excluded region 22** | 2247 | 9.0 | 0.0 | 0.0 | 132904 | 133181 | -39319 | C | AluSg | SINE/Alu | -31 | 279 | 2 | 195 | 1 |
| **Probe27** | 2247 | 9.0 | 0.0 | 0.0 | 132904 | 133181 | -39319 | C | AluSg | SINE/Alu | -31 | 279 | 2 | 195 | 1 |
| | 2851 | 6.5 | 2.2 | 0.3 | 133284 | 133639 | -38861 | + | THE1C | LTR/ERVL-MaLR | 3 | 365 | -10 | 196 | |
| | 10891 | 9.9 | 3.9 | 0.6 | 133640 | 135167 | -37333 | + | THE1C-int | LTR/ERVL-MaLR | 1 | 1578 | -2 | 196 | |
| | 2549 | 7.5 | 2.2 | 4.5 | 135168 | 135307 | -37193 | + | THE1C | LTR/ERVL-MaLR | 19 | 160 | -215 | 196 | |
| **Excluded 23 region** | 2549 | 7.5 | 2.2 | 4.5 | 135168 | 135307 | -37193 | + | THE1C | LTR/ERVL-MaLR | 19 | 160 | -215 | 196 | 2 |
| | 2027 | 12.1 | 0.0 | 8.5 | 135308 | 135638 | -36862 | C | AluSx1 | SINE/Alu | -6 | 306 | 2 | 197 | |
| | 2549 | 7.5 | 2.2 | 4.5 | 135639 | 135862 | -36638 | + | THE1C | LTR/ERVL-MaLR | 161 | 375 | 0 | 196 | |
| | 256 | 26.8 | 7.8 | 2.7 | 136283 | 136424 | -36076 | C | L1M6B | UNE/L1 | -156 | 213 | 65 | 198 | |
| | 2419 | 8.7 | 0.0 | 0.7 | 136753 | 137063 | -35437 | C | AluSq2 | SINE/Alu | -3 | 309 | 1 | 199 | |
| **Probe28a** | 289 | 30.0 | 4.7 | 5.4 | 137189 | 137336 | -35164 | C | L2a | LINE/L2 | -4 | 3422 | 3276 | 200 | 1 |
| | 258 | 29.4 | 6.7 | 1.8 | 137612 | 137715 | -34785 | + | MIRb | SINE/MIR | 116 | 224 | -44 | 201 | |
| | 397 | 25.0 | 3.8 | 2.5 | 139471 | 139630 | -32870 | C | Charlie18a | DNA/hAT-Charlie | -2 | 340 | 179 | 202 | |
| | 1647 | 17.7 | 2.4 | 4.0 | 139631 | 140006 | -32494 | + | L1MB4 | LINE/L1 | 5777 | 614 6 | -34 | 203 | |
| | 458 | 5.7 | 0.0 | 0.0 | 140640 | 140692 | -31808 | C | AluYb8 | SINE/Alu | -260 | 58 | 6 | 204 | |
| | 245 | 20.4 | 2.0 | 0.0 | 140696 | 140744 | -31756 | C | L1M5 | UNE/L1 | -453 | 567 1 | 5622 | 205 | |
| | 360 | 20.5 | 13.3 | 0.0 | 141105 | 141238 | -31262 | C | L1ME4a | LINE/L1 | -7 | 611 7 | 5952 | 206 | |
| **Excluded region 24** | 604 | 23.5 | 13.9 | 0.4 | 141588 | 141796 | -30704 | C | MIRc | SINE/MIR | -10 | 258 | 22 | 207 | 9 |
| | 355 | 33.1 | 1.8 | 3.6 | 141846 | 142014 | -30486 | C | MIR3 | SINE/MIR | -23 | 185 | 20 | 208 | |
| | 290 | 30.1 | 1.1 | 0.0 | 142104 | 142196 | -30304 | C | MIR3 | SINE/MIR | -1 | 207 | 114 | 209 | |
| | 245 | 23.2 | 11.5 | 6.1 | 142805 | 142882 | -29618 | C | L2c | LINE/L2 | -20 | 336 7 | 3286 | 210 | |
| | 189 | 7.4 | 0.0 | 0.0 | 143821 | 143847 | -28653 | + | (CTGGGG)n | Simple_repeat | 6 | 32 | 0 | 211 | |
| | 24 | 54.2 | 0.0 | 0.0 | 144054 | 144077 | -28423 | + | GC_rich | Low_complexity | 1 | 24 | 0 | 212 | |
| | 183 | 8.0 | 0.0 | 0.0 | 144078 | 144102 | -28398 | + | (CTG)n | Simple _repeat | 1 | 25 | 0 | 213 | |
| | 1181 | 17.2 | 11.5 | 1.5 | 145589 | 145671 | -26829 | + | MER33 | DNA/hAT-Charlie | 1 | 81 | -243 | 214 | |
| | 2001 | 15.5 | 0.0 | 0.3 | 145672 | 145974 | -26526 | + | AluJr | SINE/Alu | 1 | 302 | -10 | 215 | |
| | 1181 | 17.2 | 11.5 | 1.5 | 145975 | 146185 | -26315 | + | MER33 | DNA/hAT-Charlie | 82 | 324 | 0 | 214 | |
| | 188 | 32.9 | 7.8 | 1.1 | 146389 | 146554 | -25946 | C | L2 | LINE/L2 | - 1148 | 227 1 | 2095 | 216 | |
| | 247 | 23.3 | 8.6 | 4.0 | 146683 | 146808 | -25692 | + | L2c | LINE/L2 | 3229 | 335 8 | -17 | 217 | |
| | 2357 | 7.8 | 0.3 | 0.0 | 146879 | 147193 | -25307 | + | AluSp | SINE/Alu | 1 | 313 | 0 | 218 | |
| | 295 | 29.2 | 6.9 | 0.0 | 147406 | 147535 | -24965 | + | HAL1 | LINE/L1 | 150 | 288 | - 2219 | 219 | |
| | 793 | 22.6 | 5.8 | 4.9 | 147869 | 148110 | -24390 | C | MER46C | DNA/TcMar-Tigger | 0 | 338 | 95 | 220 | |
| | 1758 | 10.8 | 0.0 | 0.4 | 148122 | 148352 | -24148 | C | AluJb | SINE/Alu | -81 | 231 | 2 | 221 | |
| | 722 | 16.0 | 7.9 | 7.5 | 148393 | 148639 | -23861 | + | L1MB2 | LINE/L1 | 5942 | 617 8 | -5 | 222 | |
| | 298 | 22.6 | 0.0 | 0.0 | 148651 | 148712 | -23788 | C | MER46C | DNA/TcMar-Tigger | -274 | 64 | 3 | 220 | |
| | 2096 | 9.5 | 4.7 | 1.6 | 149417 | 149712 | -22788 | + | AluSx1 | SINE/Alu | 1 | 305 | -7 | 223 | |
| | 2301 | 9.8 | 0.9 | 2.2 | 149713 | 150028 | -22472 | + | AluSq | SINE/Alu | 1 | 312 | -1 | 224 | |
| | 264 | 29.2 | 8.3 | 12. 8 | 150088 | 150137 | -22363 | C | MIRb | SINE/MIR | -17 | 251 | 202 | 225 | |
| | 2099 | 11.0 | 0.3 | 7.2 | 150138 | 150465 | -22035 | C | AluSx | SINE/Alu | -5 | 307 | 1 | 226 | |
| | 266 | 27.9 | 6.0 | 7.6 | 150466 | 150634 | -21866 | C | MIRc | SINE/MIR | -67 | 201 | 38 | 225 | |
| | 278 | 21.4 | 15.0 | 4.8 | 151220 | 151310 | -21190 | + | L2a | LINE/L2 | 3303 | 340 5 | -21 | 227 | |
| | 2280 | 10.7 | 0.0 | 0.0 | 151311 | 151601 | -20899 | C | AluSx1 | SINE/Alu | -21 | 291 | 1 | 228 | |
| | 278 | 21.4 | 15.0 | 4.8 | 151602 | 151622 | -20878 | + | L2a | LINE/L2 | 3406 | 342 6 | 0 | 227 | |
| | 28 | 68.6 | 0.0 | 0.0 | 152478 | 152512 | -19988 | + | AT_rich | Low_complexity | 1 | 35 | 0 | 229 | |
| | 2204 | 11.1 | 1.3 | 0.0 | 152585 | 152906 | -19594 | + | AluSx | SINE/Alu | 10 | 312 | 0 | 230 | |
| | 2129 | 11.3 | 0.0 | 0.7 | 152925 | 153250 | -19250 | C | AluSz | SINE/Alu | 0 | 312 | 1 | 231 | |
| **Probe29** | 1328 | 11.5 | 3.0 | 4.3 | 154064 | 154300 | -18200 | C | L1MA6 | LINE/L1 | -7 | 629 3 | 6060 | 232 | 1 |
| | 1331 | 9.1 | 0.5 | 0.0 | 154301 | 154486 | -18014 | + | L1MA6 | LINE/L1 | 5791 | 597 7 | -323 | 232 | |
| | 1253 | 11.9 | 0.0 | 0.0 | 154521 | 154688 | -17812 | + | AluSp | SINE/Alu | 137 | 304 | -9 | 233 | |
| | 186 | 4.3 | 0.0 | 0.0 | 154690 | 154712 | -17788 | + | (CA)n | Simple_repeat | 2 | 24 | 0 | 234 | |
| | 505 | 17.1 | 1.7 | 4.4 | 155541 | 155656 | -16844 | C | Charlie4z | DNA/hAT-Charlie | 0 | 167 | 55 | 235 | |
| **Excluded region 25** | 2345 | 9.2 | 0.0 | 4.8 | 155799 | 156123 | -16377 | + | AluSg4 | SINE/Alu | 1 | 310 | -2 | 236 | 6 |
| | 2161 | 10.1 | 2.1 | 0.0 | 156545 | 156830 | -15670 | C | AluSx | SINE/Alu | -20 | 292 | 1 | 237 | |
| | 2127 | 12.2 | 0.0 | 1.7 | 156920 | 15722 | -15278 | C | AluSz | SINE/Alu | -14 | 298 | 1 | 238 | |
| | 2272 | 9.2 | 0.0 | 1.4 | 157475 | 157817 | -14683 | + | AluSx | SINE/Alu | 6 | 312 | 0 | 239 | |
| | 2219 | 3.4 | 2.7 | 0.0 | 157830 | 157956 | -14544 | + | AluY | SINE/Alu | 1 | 127 | -184 | 240 | |
| | 369 | 0.0 | 0.0 | 0.0 | 157957 | 157997 | -14503 | + | (TAAA)n | Simple_repeat | 2 | 42 | 0 | 241 | |
| | 2219 | 3.4 | 2.7 | 0.0 | 157998 | 158132 | -14368 | + | AluY | SINE/Alu | 128 | 269 | -42 | 240 | |
| **Probe30** | 2231 | 12.0 | 0.3 | 0.7 | 160325 | 160633 | -11867 | C | AluSx1 | SINE/Alu | -4 | 308 | 1 | 242 | 2 |
| | 1987 | 14.8 | 0.3 | 5.8 | 160810 | 161034 | -11466 | C | Tigger3a | DNA/TcMar-Tigger | -20 | 328 | 106 | 243 | |
| | 1922 | 13.6 | 0.0 | 0.7 | 161035 | 161313 | -11187 | + | AluSx | SINE/Alu | 1 | 277 | -35 | 244 | |
| | 270 | 0.0 | 0.0 | 0.0 | 161319 | 161348 | -11152 | + | (TAAA)n | Simple_repeat | 3 | 32 | 0 | 245 | |
| | 1987 | 14.8 | 0.3 | 5.8 | 161349 | 161461 | -11039 | C | Tigger3a | DNA/TcMar-Tigger | -243 | 105 | 2 | 243 | |
| **Probe31** | 408 | 29.6 | 1.0 | 11.8 | 161656 | 161862 | -10638 | + | MER20B | DNA/hAT-Charlie | 2 | 188 | -595 | 246 | 0 |
| | 628 | 26.9 | 8.4 | 2.9 | 162861 | 163086 | -9414 | C | MIR | SINE/MIR | -23 | 239 | 2 | 247 | |
| | 542 | 30.2 | 3.3 | 0.9 | 163485 | 163698 | -8802 | C | L2 | LINE/L2 | -745 | 2674 | 2456 | 248 | |
| | 428 | 34.8 | 16. 6 | 1.9 | 164306 | 164914 | -7586 | + | L3 | LINE/CR1 | 655 | 1352 | - 2747 | 249 | |
| | 181 | 19.1 | 4.8 | 0.0 | 165048 | 165089 | -7411 | + | MIRb | SINE/MIR | 144 | 187 | -81 | 250 | |
| | 879 | 27.8 | 2.1 | 1.3 | 165105 | 165341 | -7159 | + | Tigger13a | DNA/TcMar-Tigger | 12 | 250 | -521 | 251 | |
| | 450 | 29.4 | 10. 1 | 0.0 | 165344 | 165571 | -6929 | + | Tigger13a | DNA/TcMar-Tigger | 342 | 592 | -179 | 252 | |
| | 460 | 22.3 | 7.1 | 4.4 | 165562 | 165716 | -6784 | + | Tigger13a | DNA/TcMar-Tigger | 607 | 765 | -6 | 253 | |
| | 308 | 24.3 | 0.0 | 0.0 | 165721 | 165786 | -6714 | + | MIRb | SINE/MIR | 197 | 262 | 0 | 254 | |
| | 195 | 36.4 | 1.0 | 1.0 | 165816 | 165915 | -6585 | + | L3 | LINE/CR1 | 1344 | 1443 | - 2656 | 249 | |
| | 585 | 27.5 | 20. 2 | 0.7 | 166018 | 166396 | -6104 | + | L1M5 | LINE/L1 | 2518 | 2973 | 3173 | 255 | |
| **Excluded region 26** | 585 | 27.5 | 20. 2 | 0.7 | 166018 | 166396 | -6104 | + | L1M5 | LINE/L1 | 2518 | 2973 | 3173 | 255 | 6 |
| | 2492 | 6.5 | 0.0 | 0.0 | 166397 | 166690 | -5810 | C | AluY | SINE/Alu | -16 | 295 | 2 | 256 | |
| | 1414 | 15.4 | 1.4 | 19. 3 | 166699 | 166938 | -5562 | C | AluJb | SINE/Alu | -2 | 300 | 115 | 257 | |
| | 276 | 3.0 | 0.0 | 0.0 | 166939 | 166971 | -5529 | + | (TC)n | Simple_repeat | 2 | 34 | 0 | 258 | |
| | 1414 | 15.4 | 1.4 | 19. 3 | 166972 | 167083 | -5417 | C | AluJb | SINE/Alu | -188 | 114 | 1 | 257 | |
| | 237 | 28.2 | 10. 3 | 2.2 | 167084 | 167217 | -5283 | + | L1M5 | LINE/L1 | 2981 | 3118 | - 3028 | 255 | |
| | 746 | 18.4 | 0.0 | 3.8 | 167220 | 167355 | -5145 | + | FLAM_C | SINE/Alu | 2 | 132 | -11 | 259 | |
| | 299 | 25.1 | 8.5 | 1.1 | 167398 | 167562 | -4938 | + | L1M5 | LINE/L1 | 3219 | 3395 | - 2751 | 255 | |
| | 1486 | 16.0 | 0.0 | 3.7 | 167618 | 167867 | -4633 | C | AluJo | SINE/Alu | -20 | 292 | 52 | 260 | |
| | 771 | 30.1 | 6.1 | 5.2 | 167896 | 168116 | -4384 | + | L1M5 | LINE/L1 | 3410 | 3626 | - 2520 | 255 | |
| | 2460 | 9.3 | 0.3 | 0.0 | 168117 | 168428 | -4072 | C | AluSp | SINE/Alu | 0 | 313 | 1 | 261 | |
| | 771 | 30.1 | 6.1 | 5.2 | 168429 | 168679 | -3821 | + | L1M5 | LINE/L1 | 3627 | 3886 | 2260 | 255 | |
| | 706 | 21.9 | 4.8 | 8.3 | 168751 | 169044 | -3456 | + | L1M5 | LINE/L1 | 3929 | 4208 | - 1938 | 255 | |
| | 2031 | 12.3 | 1.4 | 0.7 | 169045 | 169336 | -3164 | + | AluSx1 | SINE/Alu | 1 | 294 | -18 | 262 | |
| | 716 | 22.1 | 1.1 | 5.0 | 169349 | 169534 | -2966 | + | L1M4 | LINE/L1 | 2 | 180 | - 6362 | 263 | |
| | 927 | 20.2 | 1.2 | 1.7 | 169546 | 169718 | -2782 | C | FAM | SINE/Alu | -13 | 172 | 1 | 264 | |
| | 2029 | 23.8 | 8.0 | 2.8 | 169720 | 170776 | -1724 | + | L1M4 | LINE/L1 | 188 | 1298 | 5244 | 263 | |
| **Probe32** | 2029 | 23.8 | 8.0 | 2.8 | 169720 | 170776 | -1724 | + | L1M4 | LINE/L1 | 188 | 1298 | - 5244 | 263 | 0 |
| | 1480 | 20.6 | 5.8 | 0.0 | 170776 | 171221 | -1279 | + | L1M2 | LINE/L1 | 1 | 472 | - 6377 | 265 | |
| | 607 | 26.4 | 0.7 | 0.0 | 171233 | 171376 | -1124 | + | L1M2b | LINE/L1 | 498 | 642 | 6567 | 266 | |
| | 3991 | 25.2 | 2.7 | 3.3 | 171348 | 172500 | 0 | + | L1M2 | LINE/L1 | 581 | 1642 | 5207 | 265 | |
| **Exduded region 27** | 3991 | 25.2 | 2.7 | 3.3 | 171348 | 172500 | 0 | + | L1M2 | LINE/L1 | 581 | 1642 | 5207 | 265 | 0 |

**Table 5**

| **Description of the 6 characterized large rearrangements as detected by MLPA and Molecular Combing** | | | | | | | |
|---|---|---|---|---|---|---|---|
| **Sample** | **Gene** | **MLPA status** | **Molecular Combing** | **Breakpoints (bp)** | **Mechanism** | **Mutation name** | **Reference** |
| **1** | *BRCA1* | Dup ex 13 | 6.1± 1.6 kb/Dup ex 13 | 38483825-38489905 | Alu-Alu HR | c.4186-1785_4358-1667dup6081 | Puget et al. (1999) |
| **2** | *BRCA1* | Del ex 2 | 40.8 ± 3.5 kb / Del ex 2 | NBR1 38 562 663 - 38 562 427; BRCA1 38 525 728 - 38 525 492 | Pseudogen-Alu | c-33024_80+3832del36936 | Puget N, 2002 Am J Hum Genet 70:858-865 |
| **3** | *BRCA1* | Del ex 2 | 39.0 ± 2.6 kb / Del ex 2 | NBRJ 38 562 663 - 38 562 427, BRCA1 38 525 728 - 38 525 492 | Pseudogen-Alu | c-33024_80+3832del36936 | Puget N, 2002 Am J Hum Genet 70 :858-865 |
| **4** | *BRCA1* | Dup ex 18-20 | 6.7 ± 1.2 kb / Dup ex 18-20 | 38460514-38470596 | Alu-Alu HR | c.5075-1093_5277+2089dup10082 | Staaf et al. (2008) |
| **5** | *BRCA1* | Del ex 15 | 4.1 ± 1.2 kb / Del ex 15 | 38478177-38481174 | Alu-Alu HR | c.4484+857_4676-1396del | Puget et al. (1999b) |
| **6** | *BRCA1* | Del ex 8-13 | 20 ± 2.8 kb / Del ex 8-13 | 38,507,324_38,483,560 | Alu-Alu HR | c.442-1901_4358-1404del23763 | Puget et al. (1999b) |

| | | | | | | | |
|---|---|---|---|---|---|---|---|
| All patients were previously characterized by high resolution aCGH, and the reported values were originally described by Rouleau et al (Rouleau 2007). | | | | | | | |

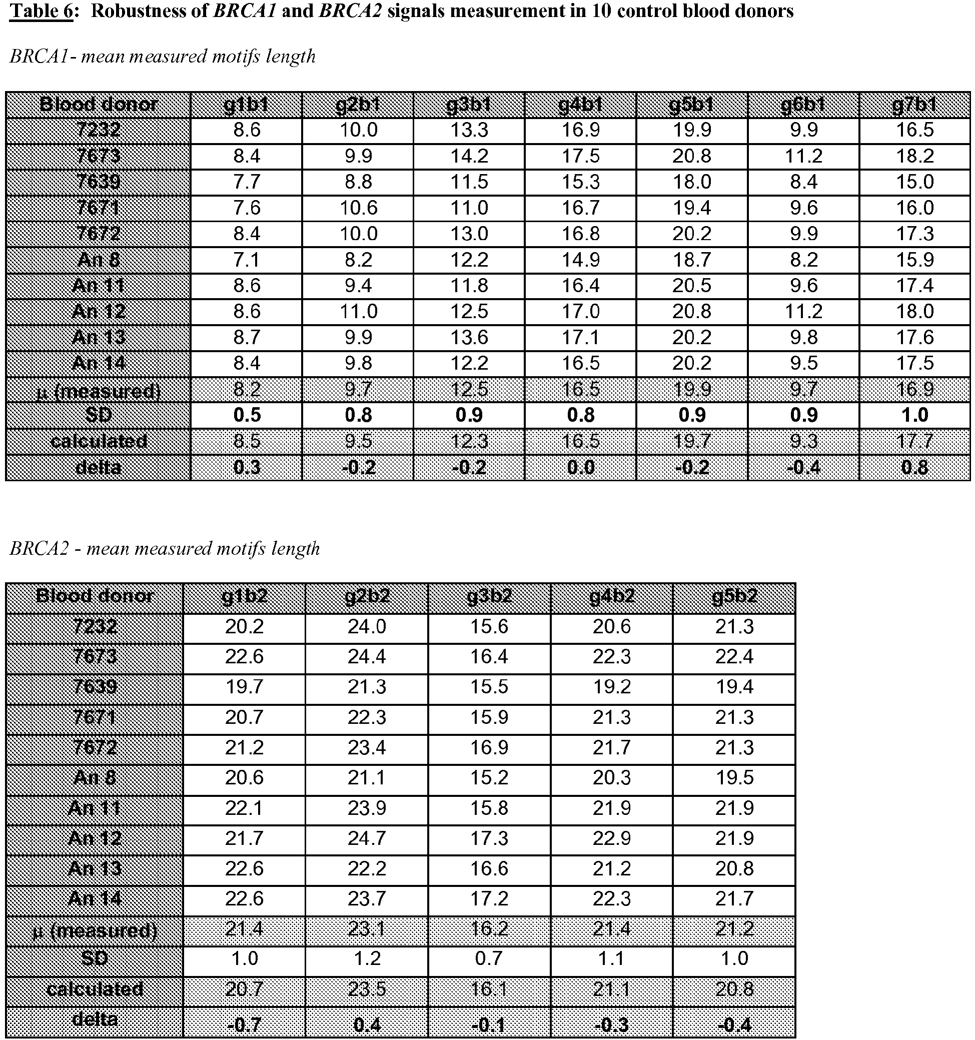

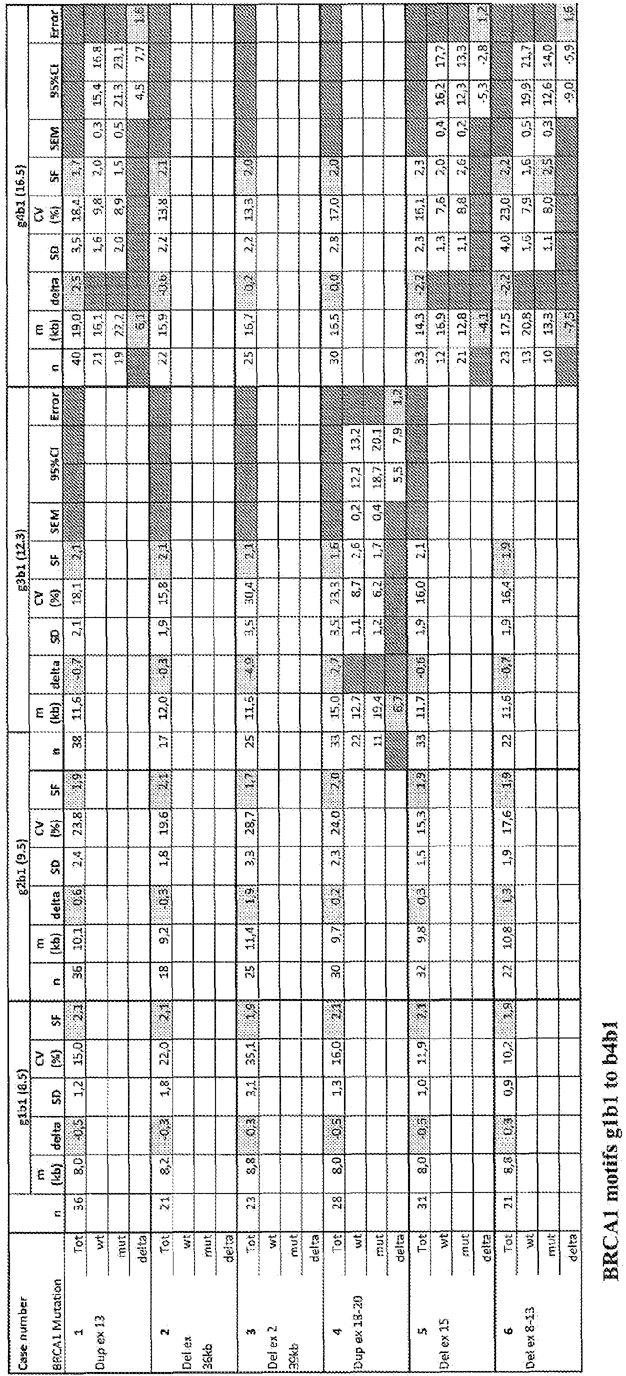

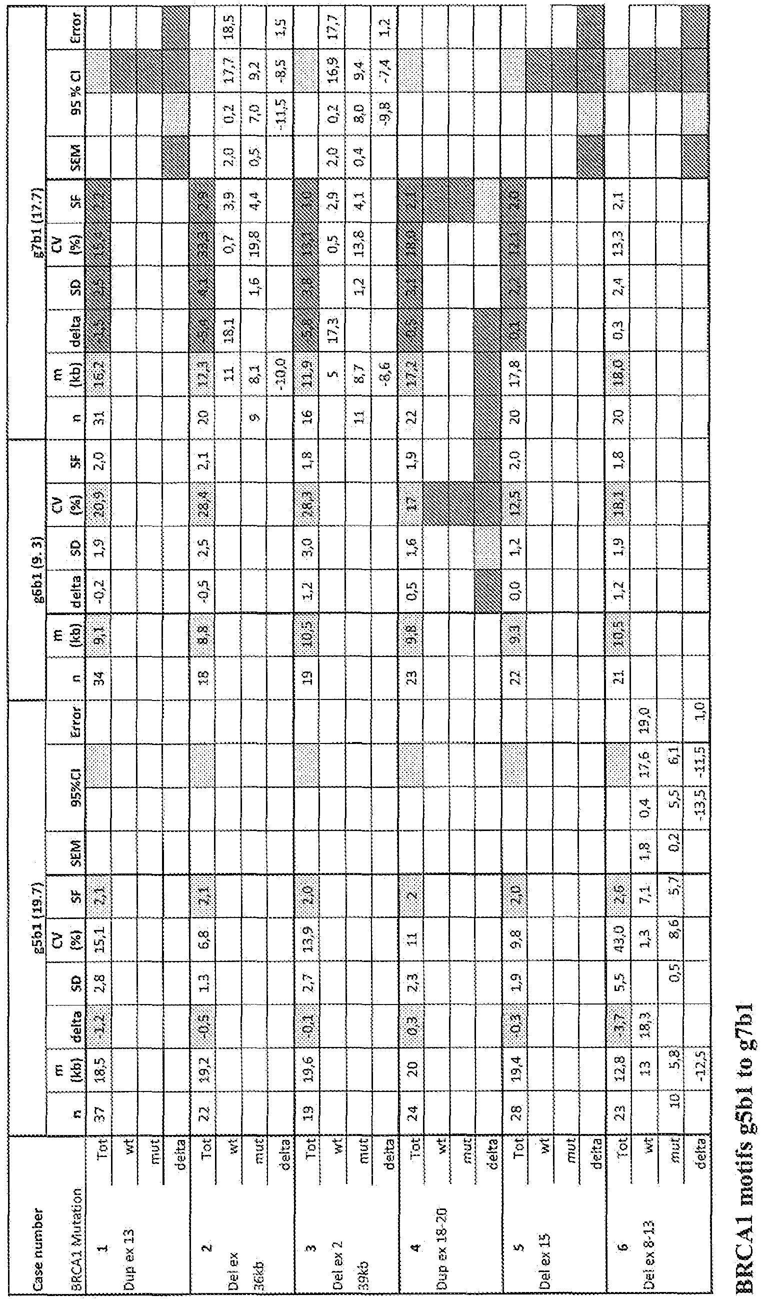

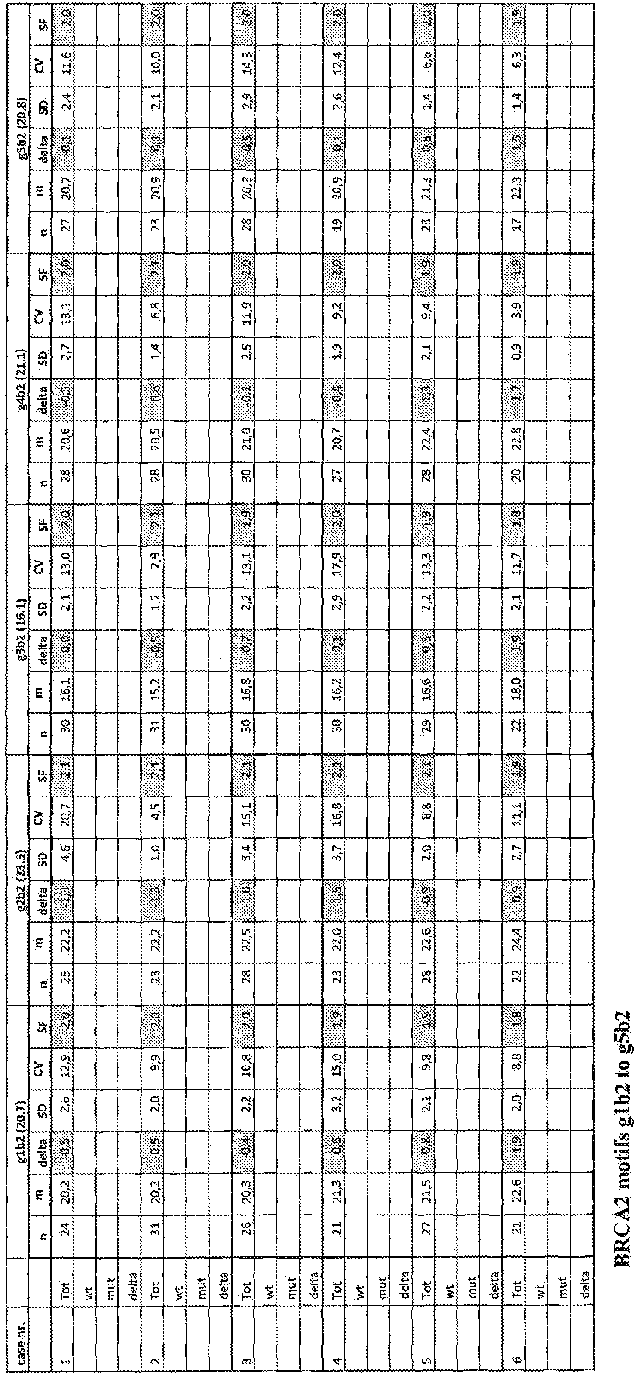

**Table 8**

| | | | | | |
|---|---|---|---|---|---|
| SEQ ID NO° | 1 | BRCA1-1A-F | DNA | Homo sapiens | GGGACGGAAAGCTATGATGT |
| SEQ ID NO° | 2 | BRCA1-1A-R | DNA | Homo sapiens | GGGCAGAGGTGACAGGTCTA |
| SEQ ID NO° | 3 | BRCA1-1B-F | DNA | Homo sapiens | CCTCTGACCTGATCCCTTGA |
| SEQ ID NO° | 4 | BRCA1-1B-R | DNA | Homo sapiens | ATCAGCAACAGTCCCATTCC |
| SEQ ID NO° | 5 | BRCA1-2-F | DNA | Homo sapiens | GCCCAGACTAGTGTTTCTTAACC |
| SEQ ID NO° | 6 | BRCA1-2-R | DNA | Homo sapiens | GGCATGAGGCAGCAATTTAG |
| SEQ ID NO° | 7 | BRCA1-3-F | DNA | Homo sapiens | TCTTTGAATCTGGGCTCTGC |
| SEQ ID NO° | 8 | BRCA1-3-R | DNA | Homo sapiens | GCTGTTGCTTTCTTTGAGGTG |
| SEQ ID NO° | 9 | BRCA1-4-F | DNA | Homo sapiens | CACAGGTATGTGGGCAGAGA |
| SEQ ID NO° | 10 | BRCA1-4-R | DNA | Homo sapiens | CCTCTGTTGATGGGGTCATAG |
| SEQ ID NO° | 11 | BRCA1-5-F | DNA | Homo sapiens | TTTGGTAGACCAGGTGAAATGA |
| SEQ ID NO° | 12 | BRCA1-5-R | DNA | Homo sapiens | CAAATTATGTGTGGAGGCAGA |
| SEQ ID NO° | 13 | BRCA1-6-F | DNA | Homo sapiens | GAAGAACGTGCTCTTTTCACG |
| SEQ ID NO° | 14 | BRCA1-6-R | DNA | Homo sapiens | AAAGTCTGATAACAGCTCCGAGA |
| SEQ ID NO° | 15 | BRCA1-7-F | DNA | Homo sapiens | TTCGATTCCCTAAGATCGTTTC |
| SEQ ID NO° | 16 | BRCA1-7-R | DNA | Homo sapiens | CACAGTTCTGTGTAATTTAATTTCGAT |
| SEQ ID NO° | 17 | BRCA1-8-F | DNA | Homo sapiens | AGGGAAGGCTCAGATACAAAC |
| SEQ ID NO° | 18 | BRCA1-8-R | DNA | Homo sapiens | TGCCATAGATAGAGGGCTTTTT |
| SEQ ID NO° | 19 | BRCA1-9-F | DNA | Homo sapiens | GCCATCTTCTTTCTCCTGCT |
| SEQ ID NO° | 20 | BRCA1-9-R | DNA | Homo sapiens | TTGACCTATTGCTGAATGTTGG |
| SEQ ID NO° | 21 | BRCA1-11-F | DNA | Homo sapiens | TTTTACCAAGGAAGGATTTTCG |
| SEQ ID NO° | 22 | BRCA1-11-R | DNA | Homo sapiens | GCTTGATCACAGATGTATGTATGAGTT |
| SEQ ID NO° | 23 | BRCA1-12-F | DNA | Homo sapiens | CCCCAGGGCTTTAAAGGTTA |
| SEQ ID NO° | 24 | BRCA1-12-R | DNA | Homo sapiens | TAGGGGTGGATATGGGTGAA |
| SEQ ID NO° | 25 | BRCA1-13A-F | DNA | Homo sapiens | ACTTCTTCAACGCGAAGAGC |
| SEQ ID NO° | 26 | BRCA1-13A-R | DNA | Homo sapiens | GACAGGCTGTGGGGTTTCT |
| SEQ ID NO° | 27 | BRCA1-15-F | DNA | Homo sapiens | TATCTGCTGGCCACTTACCA |
| SEQ ID NO° | 28 | BRCA1-15-R | DNA | Homo sapiens | TCTCGAGCCTTGAACATCCT |
| SEQ ID NO° | 29 | BRCA1-16-F | DNA | Homo sapiens | CGCTCAGCTTTCATTCCAGT |
| SEQ ID NO° | 30 | BRCA1-16-R | DNA | Homo sapiens | AAACGTTCACATGTATCCCCTAA |
| SEQ ID NO° | 31 | BRCA1-17-F | DNA | Homo sapiens | CCTGGCCAGTACCCAGTAGT |
| SEQ ID NO° | 32 | BRCA1-17-R | DNA | Homo sapiens | CTGAGCCCAGAGTTTCTGCT |
| SEQ ID NO° | 33 | BRCA1-18-F | DNA | Homo sapiens | GGGCCCAAAAACCAGTAAGA |
| SEQ ID NO° | 34 | BRCA1-18-R | DNA | Homo sapiens | GGGATTGAGCGTTCACAGAT |
| SEQ ID NO° | 35 | BRCA1-19-F | DNA | Homo sapiens | GCCATCCAGTCCAGTCTCAT |
| SEQ ID NO° | 36 | BRCA1-19-R | DNA | Homo sapiens | TGCAGTTCTACCCTCCACTTG |
| SEQ ID NO° | 37 | BRCA1-22-F | DNA | Homo sapiens | CGGGTAAGTGGTGAGCTTTC |
| SEQ ID NO° | 38 | BRCA1-22-R | DNA | Homo sapiens | GACTGTCATTTAAAGGCACTTTTT |
| SEQ ID NO° | 39 | BRCA1-23-F | DNA | Homo sapiens | TGGCTAGTGTTTTGGCCTGT |
| SEQ ID NO° | 40 | BRCA1-23-R | DNA | Homo sapiens | TTCAGTGTTGCTTCTCCATTTC |
| SEQ ID NO° | 41 | BRCA1-24-F | DNA | Homo sapiens | TGTCAGACTAGCCACAGTACCA |
| SEQ ID NO° | 42 | BRCA1-24-R | DNA | Homo sapiens | AAGCGCTTCTTCATATTCTCC |
| SEQ ID NO° | 43 | BRCA1-25-F | DNA | Homo sapiens | ACCACACTCTTCTGTTTTGATGT |
| SEQ ID NO° | 44 | BRCA1-25-R | DNA | Homo sapiens | GGCACATGTACACCATGGAA |
| SEQ ID NO° | 45 | BRCA1-26-F | DNA | Homo sapiens | TTGTGTAGGTTGCCCGTTC |
| SEQ ID NO° | 46 | BRCA1-26-R | DNA | Homo sapiens | TTCAGAGAGCTGGGCCTAAA |
| SEQ ID NO° | 47 | BRCA1-27-F | DNA | Homo sapiens | GGAGGCAATCTGGAATTGAA |
| SEQ ID NO° | 48 | BRCA1-27-R | DNA | Homo sapiens | GGATCCATGATTGCTGCTTT |
| SEQ ID NO° | 49 | BRCA1-28-F | DNA | Homo sapiens | CTCTGCTGTTTTTACAACTTTTTC |
| SEQ ID NO° | 50 | BRCA1-28-R | DNA | Homo sapiens | GGATCCATGATTGCTGCTTT |
| SEQ ID NO° | 51 | BRCA1-29-F | DNA | Homo sapiens | CCCTCTAGATACTTGTGTCCTTTTG |
| SEQ ID NO° | 52 | BRCA1-29-R | DNA | Homo sapiens | TCTGGCAGTCACAATTCAGG |
| SEQ ID NO° | 53 | BRCA1-30-F | DNA | Homo sapiens | TCCCATGACTGCATCATCTT |
| SEQ ID NO° | 54 | BRCA1-30-R | DNA | Homo sapiens | TTGAGATCAGGTCGATTCCTC |
| SEQ ID NO° | 55 | BRCA1-31-F | DNA | Homo sapiens | AAAACTCAACCCAAACAGTCA |
| SEQ ID NO° | 56 | BRCA1-31-R | DNA | Homo sapiens | CCAAGAATCACGAAGAGAGAGA |
| SEQ ID NO° | 57 | BRCA1-32-F | DNA | Homo sapiens | GACCTCATAGAGGTAGTGGAAAGAA |
| SEQ ID NO° | 58 | BRCA1-32-R | DNA | Homo sapiens | GCTCAAAGCCTTTAGAAGAAACA |
| SEQ ID NO° | 59 | BRCA1-33-F | DNA | Homo sapiens | GCACTGGGGAAAAGGTAGAA |
| SEQ ID NO° | 60 | BRCA1-33-R | DNA | Homo sapiens | CTCTTCAACCCAGACAGATGC |
| SEQ ID NO° | 61 | BRCA1-34-F | DNA | Homo sapiens | CAATACCCAATACAATGTAAATGC |
| SEQ ID NO° | 62 | BRCA1-34-R | DNA | Homo sapiens | CTGGGGATACTGAAACTGTGC |
| SEQ ID NO° | 63 | BRCA1-35-F | DNA | Homo sapiens | ATCAAGAAGCCTTCCCAGGT |
| SEQ ID NO° | 64 | BRCA1-35-R | DNA | Homo sapiens | TCCTTGGACGTAAGGAGCTG |
| SEQ ID NO° | 65 | BRCA1-36-F | DNA | Homo sapiens | TTCAGAACTTCCAAATACGGACT |
| SEQ ID NO° | 66 | BRCA1-36-R | DNA | Homo sapiens | GATGGAGCTGGGGTGAAAT |
| SEQ ID NO° | 67 | BRCA1-37-F | DNA | Homo sapiens | CGTGAGATTGCTCACAGGAC |
| SEQ ID NO° | 68 | BRCA1-37-R | DNA | Homo sapiens | CAAGGCATTGGAAAGGTGTC |
| SEQ ID NO° | 69 | BRCA1-38-F | DNA | Homo sapiens | AGAGGAATAGACCATCCAGAAGT |
| SEQ ID NO° | 70 | BRCA1-38-R | DNA | Homo sapiens | TCCTCCAGCACTAAAAACTGC |
| SEQ ID NO° | 71 | BRCA2-1-F | DNA | Homo sapiens | AAATGGAGGTCAGGGAACAA |
| SEQ ID NO° | 72 | BRCA2-1-R | DNA | Homo sapiens | TGGAAAGTTTGGGTATGCAG |
| SEQ ID NO° | 73 | BRCA2-2 -F | DNA | Homo sapiens | TCTCAATGTGCAAGGCAATC |
| SEQ ID NO° | 74 | BRCA2-2-R | DNA | Homo sapiens | TCTTGACCATGTGGCAAATAA |
| SEQ ID NO° | 75 | BRCA2-3a-F | DNA | Homo sapiens | AATCACCCCAACCTTCAGC |
| SEQ ID NO° | 76 | BRCA2-3a-R | DNA | Homo sapiens | GCCCAGGACAAACATTTTCA |
| SEQ ID NO° | 77 | BRCA2-3b-F | DNA | Homo sapiens | CCCTCGCATGTATGATCTGA |
| SEQ ID NO° | 78 | BRCA2-3b-R | DNA | Homo sapiens | CTCCTGAAGTCCTGGAAACG |
| SEQ ID NO° | 79 | BRCA2-3c-F | DNA | Homo sapiens | TGAAATCTTTTCCCTCTCATCC |
| SEQ ID NO° | 80 | BRCA2-3c-R | DNA | Homo sapiens | AGATTGGGCACATCGAAAAG |
| SEQ ID NO° | 81 | BRCA2-5-F | DNA | Homo sapiens | GGTCTTGAACACCTGCTACCC |
| SEQ ID NO° | 82 | BRCA2-5-R | DNA | Homo sapiens | CACTCCGGGGGTCCTAGAT |
| SEQ ID NO° | 83 | BRCA2-6-F | DNA | Homo sapiens | TCTTTAACTGTTCTGGGTCACAA |
| SEQ ID NO° | 84 | BRCA2-6-R | DNA | Homo sapiens | TGGCTAGAATTCAAAACACTGA |
| SEQ ID NO° | 85 | BRCA2-7-F | DNA | Homo sapiens | TTGAAGTGGGGTTTTTAAGTTACAC |
| SEQ ID NO° | 86 | BRCA2-7-R | DNA | Homo sapiens | CCAGCCAATTCAACATCACA |
| SEQ ID NO° | 87 | BRCA2-11-F | DNA | Homo sapiens | TTGGGACAATTCTGAGGAAAT |
| SEQ ID NO° | 88 | BRCA2-11-R | DNA | Homo sapiens | TGCAGGTTTTGTTAAGAGTTTCA |
| SEQ ID NO° | 89 | BRCA2-12-F | DNA | Homo sapiens | TGGCAAATGACTGCATTAGG |
| SEQ ID NO° | 90 | BRCA2-12-R | DNA | Homo sapiens | TCTTGAAGGCAAACTCTTCCA |
| SEQ ID NO° | 91 | BRCA2-13-F | DNA | Homo sapiens | GGAATTGTTGAAGTCACTGAGTTGT |
| SEQ ID NO° | 92 | BRCA2-13-R | DNA | Homo sapiens | ACCACCAAAGGGGGAAAAC |
| SEQ ID NO° | 93 | BRCA2-14-F | DNA | Homo sapiens | CAAGTCTTCAGAATGCCAGAGA |
| SEQ ID NO° | 94 | BRCA2-14-R | DNA | Homo sapiens | TAAACCCCAGGACAAACAGC |
| SEQ ID NO° | 95 | BRCA2-15-F | DNA | Homo sapiens | GGCTGTTTGTTGAGGAGAGG |
| SEQ ID NO° | 96 | BRCA2-15-R | DNA | Homo sapiens | GAAACCAGGAAATGGGGTTT |
| SEQ ID NO° | 97 | BRCA2-18-F | DNA | Homo sapiens | TGTTAGGGAGGAAGGAGCAA |
| SEQ ID NO° | 98 | BRCA2-18-R | DNA | Homo sapiens | GGATGTAACTTGTTACCCTTGAAA |
| SEQ ID NO° | 99 | BRCA2-19-F | DNA | Homo sapiens | TCAATAGCATGAATCTGTTGTGAA |
| SEQ ID NO° | 100 | BRCA2-19-R | DNA | Homo sapiens | GAGGTCTGCCACAAGTTTCC |
| SEQ ID NO° | 101 | BRCA2-20-F | DNA | Homo sapiens | GGCCCACTGGAGGTTTAAT |
| SEQ ID NO° | 102 | BRCA2-20-R | DNA | Homo sapiens | TTCCTTTCAATTTGTACAGAAACC |
| SEQ ID NO° | 103 | BRCA2-21-F | DNA | Homo sapiens | TGAATCAATGTGTGTGTGCAT |
| SEQ ID NO° | 104 | BRCA2-21-R | DNA | Homo sapiens | GTGTAGGGTCCAGCCCTATG |
| SEQ ID NO° | 105 | BRCA2-22a-F | DNA | Homo sapiens | CTGAGGCTAGGAAAGCTGGA |
| SEQ ID NO° | 106 | BRCA2-22a-R | DNA | Homo sapiens | CTGAGGCTAGGAAAGCTGGA |
| SEQ ID NO° | 107 | BRCA2-22b-F | DNA | Homo sapiens | GGTTTATCCCAGGATAGAATGG |
| SEQ ID NO° | 108 | BRCA2-22b-R | DNA | Homo sapiens | AGAAAATGTGGGGTGTAAACAG |
| SEQ ID NO° | 109 | BRCA2-25-F | DNA | Homo sapiens | CAGCAAACTTCAGCCATTGA |
| SEQ ID NO° | 110 | BRCA2-25-R | DNA | Homo sapiens | GGGACATGGCAACCAAATAC |
| SEQ ID NO° | 111 | BRCA2-26-F | DNA | Homo sapiens | GCACTTTCACGTCCTTTGGT |
| SEQ ID NO° | 112 | BRCA2-26-R | DNA | Homo sapiens | CGTCGTATTCAGGAGCCATT |
| SEQ ID NO° | 113 | BRCA2-27-F | DNA | Homo sapiens | CCCAGCTGGCAAACTTTTT |
| SEQ ID NO° | 114 | BRCA2-27-R | DNA | Homo sapiens | TCGGAGGTAATTCCCATGAC |
| SEQ ID NO° | 115 | BRCA2-28a-F | DNA | Homo sapiens | TCAAGAGCCATGCTGACATC |
| SEQ ID NO° | 116 | BRCA2-28a-R | DNA | Homo sapiens | AGGTAGGGTGGGGAAGAAGA |
| SEQ ID NO° | 117 | BRCA2-29-F | DNA | Homo sapiens | TGAGTCTACTTTGCCCATAGAGG |
| SEQ ID NO° | 118 | BRCA2-29-R | DNA | Homo sapiens | TTTTGCTTTCGGGAGCTTTA |
| SEQ ID NO° | 119 | BRCA2-30-F | DNA | Homo sapiens | TTTTTGCCTGCTTCATCCTC |
| SEQ ID NO° | 120 | BRCA2-30-R | DNA | Homo sapiens | GGTTTTTAAACCTGCACATGAA |
| SEQ ID NO° | 121 | BRCA2-31-F | DNA | Homo sapiens | TGAAATTTTGTTATGTGGTGCAT |
| SEQ ID NO° | 122 | BRCA2-31-R | DNA | Homo sapiens | TTTGAAATCTGTGGAGGTCTAGC |
| SEQ ID NO° | 123 | BRCA2-32-F | DNA | Homo sapiens | GTACCAAGGGTGGCAGAAAG |
| SEQ ID NO° | 124 | BRCA2-32-R | DNA | Homo sapiens | ATGGTGTTGGTTGGGTAGGA |
| SEQ ID NO° | 125 | BRCA1-SYNT1-F | DNA | Homo sapiens | TTCAGAAAATACATCACCCAAGTTC |
| SEQ ID NO° | 126 | BRCA1-SYNT1-R | DNA | Homo sapiens | TACCATTGCCTCTTACCCACAA |
| SEQ ID NO° | 127 | BRCA1-S3Big-F | DNA | Homo sapiens | AACCTTGATTAACACTTGAGCTATTTT |
| SEQ ID NO° | 128 | BRCA1-S3Big-R | DNA | Homo sapiens | CATGGGCATTAATTGCATGA |
| SEQ ID NO° | 129 | BRCA1-SExon21-F | DNA | Homo sapiens | CCTGCATGCTCATAATGCTAGA |
| SEQ ID NO° | 130 | BRCA1-SExon21-R | DNA | Homo sapiens | TTGGGATGGGTTTGAAGAGA |
| SEQ ID NO° 131 BRCA1-1A DNA Homo sapiens | | | | | |
| | | | | | |
| | | | | | |
| SEQ ID NO° 132 BRCA1-1B DNA Homo sapiens | | | | | |
| | | | | | |
| | | | | | |
| SEQ ID NO° 133 BRCA1-SYNT1 DNA Homo sapiens | | | | | |
| | | | | | |
| | | | | | |
| | | | | | |
| SEQ ID NO° | 134 | ForwardPrimerPrefix | DNA | Artificial Sequence | AAAAGGCGCGCC |
| SEQ ID NO° | 135 | ReversePrimerPrefix | DNA | Artificial Sequence | AAAATTAATTAA |

### References

Caburet, S., Conti, C., Schurra, C., Lebofsky, R., Edelstein, S.J., and Bensimon, A. (2005). Human ribosomal RNA gene arrays display a broad range of palindromic structures. Genome Res 15, 1079-1085.
Casilli, F., Di Rocco, Z.C., Gad, S., Tournier, I., Stoppa-Lyonnet, D., Frebourg, T., and Tosi, M. (2002).Rapid detection of novel BRCA1 rearrangements in high-risk breast-ovarian cancer families using multiplex PCR of short fluorescent fragments. Hum Mutat 20, 218-226.
Cavalieri, S., Funaro, A., Pappi, P., Migone, N., Gatti, R.A., and Brusco, A. (2008). Large genomic mutations within the ATM gene detected by MLPA, including a duplication of 41 kb from exon 4 to 20.Ann Hum Genet 72, 10-18.
Gad, S., Aurias, A., Puget, N., Mairal, A., Schurra, C., Montagna, M., Pages, S., Caux, V., Mazoyer, S., Bensimon, A., et al. (2001). Color bar coding the BRCA1 gene on combed DNA: a useful strategy for detecting large gene rearrangements. Genes Chromosomes Cancer 31, 75-84.
Gad, S., Bieche, 1., Barrois, M., Casilli, F., Pages-Berhouet, S., Dehainault, C., Gauthier-Villars, M., Bensimon, A., Aurias, A., Lidereau, R., et al. (2003). Characterization of a 161 kb deletion extending from the NBR1 to the BRCA1 genes in a French breast-ovarian cancer family. Hum Mutat 21, 654.
Gad, S., Caux-Moncoutier, V., Pages-Berhouet, S., Gauthier-Villars, M., Coupier, I., Pujol, P., Frenay, M., Gilbert, B., Maugard, C., Bignon, Y.J., et al. (2002a). Significant contribution of large BRCA1 gene rearrangements in 120 French breast and ovarian cancer families. Oncogene 21, 6841-6847.
Gad, S., Klinger, M., Caux-Moncoutier, V., Pages-Berhouet, S., Gauthier-Villars, M., Coupier, I., Bensimon, A., Aurias, A., and Stoppa-Lyonnet, D. (2002b). Bar code screening on combed DNA for large rearrangements of the BRCA1 and BRCA2 genes in French breast cancer families. J Med Genet39, 817-821.
Herrick, J., and Bensimon, A. (2009). Introduction to molecular combing: genomics, DNA replication, and cancer. Methods Mol Biol 521, 71-101.
Hofmann, W., Wappenschmidt, B., Berhane, S., Schmutzler, R., and Scherneck, S. (2002). Detection of large rearrangements of exons 13 and 22 in the BRCAl gene in German families. J Med Genet 39, E36.
King, M.C., Marks, J.H., and Mandell, J.B. (2003). Breast and ovarian cancer risks due to inherited mutations in BRCA1 and BRCA2. Science 302, 643-646.
Mazoyer, S. (2005). Genomic rearrangements in the BRCA1 and BRCA2 genes. Hum Mutat 25, 415-422.
Nathanson, K.L., Wooster, R., and Weber, B.L. (2001). Breast cancer genetics: what we know and what we need. Nat Med 7, 552-556.
Puget, N., Gad, S., Perrin-Vidoz, L., Sinilnikova, O.M., Stoppa-Lyonnet, D., Lenoir, G.M., and Mazoyer, S. (2002). Distinct BRCA1 rearrangements involving the BRCA1 pseudogene suggest the existence of a recombination hot spot. Am J Hum Genet 70, 858-865.
Rouleau, E., Lefol, C., Tozlu, S., Andrieu, C., Guy, C., Copigny, F., Nogues, C., Bieche, I., and Lidereau, R. (2007). High-resolution oligonucleotide array-CGH applied to the detection and characterization of large rearrangements in the hereditary breast cancer gene BRCA1. Clin Genet 72,199-207.
Schurra, C., and Bensimon, A. (2009). Combing genomic DNA for structural and functional studies. Methods Mol Biol 464, 71-90.
Staaf, J., Torngren, T., Rambech, E., Johansson, U., Persson, C., Sellberg, G., Tellhed, L., Nilbert, M., and Borg, A. (2008). Detection and precise mapping of germline rearrangements in BRCA1, BRCA2, MSH2, and MLH1 using zoom-in array comparative genomic hybridization (aCGH). Hum Mutat 29, 555-564.
Szabo, C., Masiello, A., Ryan, J.F., and Brody, L.C. (2000). The breast cancer information core:database design, structure, and scope. Hum Mutat 16, 123-131.
Walsh, T., Lee, M.K., Casadei, S., Thornton, A.M., Stray, S.M., Pennil, C., Nord, A.S., Mandell, J.B., Swisher, E.M., and King, M.C. (2010). Detection of inherited mutations for breast and ovarian cancer using genomic capture and massively parallel sequencing. Proc Natl Acad Sci USA 107, 12629-12633.

### Related Patents and Patent Applications

Lebofsky R, Walrafen P, Bensimon A: Genomic Morse Code US 7,985,542 B2 (Appl. 11/516,673)
Murphy PD, Allen AC, Alvares CP, Critz BS, Olson SJ, Schelter DB, Zeng B: Coding sequences of the human BRCA1 gene US 5,750,400
   Skolnick MH, Goldgar DE, Miki Y, Swenson J, Kamb A, Harshman KD, Shattuck-eidens DM, Tavtigian SV, Wiseman RW, Futreal AP: 17q-linked breast and ovarian cancer susceptibility gene US 5,710,001

## Claims

1. A composition of probes for detecting simultaneously one or more large or complex mutations or genetic rearrangements in the BRCA1 or BRCA2 locus, said composition comprising at least fourteen colour-labeled polynucleotide probes, specific of said BRCA1 or BRCA2 locus respectively, wherein each of said probes contains at least 200 contiguous nucleotides and is visually detectable at high resolution, wherein said composition of probes comprises less than 10% of the Alu repetitive nucleotide sequences present in said BRCA1 or BRCA2 locus of the reference genome Build GRCh37/hg19, and wherein said mutations are selected from the group consisting of duplication, triplication, deletion, inversion, insertion, translocation and large rearrangements,
wherein said at least fourteen colour-labeled polynucleotide probes specific of said BRCA1 locus are:
(i) probes amplified by PCR using pairs of primers, wherein said pairs of primers are selected from the group consisting of the sequences of SEQ ID NO: 1 and SEQ ID NO: 2, the sequences of SEQ ID NO: 3 and SEQ ID NO: 4, the sequences of SEQ ID NO: 5 and SEQ ID NO: 6, the sequences of SEQ ID NO: 7 and SEQ ID NO: 8, the sequences of SEQ ID NO: 9 and SEQ ID NO: 10, the sequences of SEQ ID NO: 11 and SEQ ID NO: 12, the sequences of SEQ ID NO: 13 and SEQ ID NO: 14, the sequences of SEQ ID NO: 15 and SEQ ID NO: 16, the sequences of SEQ ID NO: 17 and SEQ ID NO: 18, the sequences of SEQ ID NO: 19 and SEQ ID NO: 20, the sequences of SEQ ID NO: 21 and SEQ ID NO: 22, the sequences of SEQ ID NO: 23 and SEQ ID NO: 24, the sequences of SEQ ID NO: 25 and SEQ ID NO: 26, the sequences of SEQ ID NO: 27 and SEQ ID NO: 28, the sequences of SEQ ID NO: 29 and SEQ ID NO: 30, the sequences of SEQ ID NO: 31 and SEQ ID NO: 32, the sequences of SEQ ID NO: 33 and SEQ ID NO: 34, the sequences of SEQ ID NO: 35 and SEQ ID NO: 36, the sequences of SEQ ID NO: 37 and SEQ ID NO: 38, the sequences of SEQ ID NO: 39 and SEQ ID NO: 40, the sequences of SEQ ID NO: 41 and SEQ ID NO: 42, the sequences of SEQ ID NO: 43 and SEQ ID NO: 44, the sequences of SEQ ID NO: 45 and SEQ ID NO: 46, the sequences of SEQ ID NO: 47 and SEQ ID NO: 48, the sequences of SEQ ID NO: 49 and SEQ ID NO: 50, the sequences of SEQ ID NO: 51 and SEQ ID NO: 52, the sequences of SEQ ID NO: 53 and SEQ ID NO: 54, the sequences of SEQ ID NO: 55 and SEQ ID NO: 56, the sequences of SEQ ID NO: 57 and SEQ ID NO: 58, the sequences of SEQ ID NO: 59 and SEQ ID NO: 60, the sequences of SEQ ID NO: 61 and SEQ ID NO: 62, the sequences of SEQ ID NO: 63 and SEQ ID NO: 64, the sequences of SEQ ID NO: 65 and SEQ ID NO: 66, the sequences of SEQ ID NO: 67 and SEQ ID NO: 68, the sequences of SEQ ID NO: 69 and SEQ ID NO: 70, the sequences of SEQ ID NO: 125 and SEQ ID NO: 126, the sequences of SEQ ID NO: 127 and SEQ ID NO: 128, and the sequences of SEQ ID NO: 129 and SEQ ID NO: 130; or
(ii) probes comprising the sequence of SEQ ID NO: 131 or the sequence of SEQ ID NO: 132 or the sequence of SEQ ID NO: 133; and
wherein said at least fourteen colour-labeled polynucleotide probes specific of said BRCA2 locus are probes amplified by PCR using pairs of primers, wherein said pairs of primers are selected from the group consisting of the sequences of SEQ ID NO: 71 and SEQ ID NO: 72, the sequences of SEQ ID NO: 73 and SEQ ID NO: 74, the sequences of SEQ ID NO: 75 and SEQ ID NO: 76, the sequences of SEQ ID NO: 77 and SEQ ID NO: 78, the sequences of SEQ ID NO: 79 and SEQ ID NO: 80, the sequences of SEQ ID NO: 81 and SEQ ID NO: 82, the sequences of SEQ ID NO: 83 and SEQ ID NO: 84, the sequences of SEQ ID NO: 85 and SEQ ID NO: 86, the sequences of SEQ ID NO: 87 and SEQ ID NO: 88, the sequences of SEQ ID NO: 89 and SEQ ID NO: 90, the sequences of SEQ ID NO: 91 and SEQ ID NO: 92, the sequences of SEQ ID NO: 93 and SEQ ID NO: 94, the sequences of SEQ ID NO: 95 and SEQ ID NO: 96, the sequences of SEQ ID NO: 97 and SEQ ID NO: 98, the sequences of SEQ ID NO: 99 and SEQ ID NO: 100, the sequences of SEQ ID NO: 101 and SEQ ID NO: 102, the sequences of SEQ ID NO: 103 and SEQ ID NO: 104, the sequences of SEQ ID NO: 105 and SEQ ID NO: 106, the sequences of SEQ ID NO: 107 and SEQ ID NO: 108, the sequences of SEQ ID NO: 109 and SEQ ID NO: 110, the sequences of SEQ ID NO: 111 and SEQ ID NO: 112, the sequences of SEQ ID NO: 113 and SEQ ID NO: 114, the sequences of SEQ ID NO: 115 and SEQ ID NO: 116, the sequences of SEQ ID NO: 117 and SEQ ID NO: 118, the sequences of SEQ ID NO: 119 and SEQ ID NO: 120, the sequences of SEQ ID NO: 121 and SEQ ID NO: 122, and the sequences of SEQ ID NO: 123 and SEQ ID NO: 124.

2. The composition of claim 1, wherein each of said at least fourteen probes contains from 500 to 6000 contiguous nucleotides and contains less than 10% of Alu repetitive nucleotidic sequences.

3. The composition of any of claims 1 or 2, comprising at least two, in particular at least three, in particular at least four probes that are each tagged with a different detectable label or marker.

4. A method for detecting a duplication, deletion, inversion, insertion, translocation or large rearrangement in a BRCA1 or BRCA2 locus, BRCA1 or BRCA2 gene, BRCA1 or BRCA2 flanking sequence or intron, comprising:
(i) isolating a DNA sample,
(ii) molecularly combing said sample,
(iii) contacting the molecularly combed DNA with the composition of probes of claim 1 wherein the at least fourteen probes are each tagged with a detectable label or marker for a time and under conditions sufficient for hybridization to occur,
(iv) visualizing the hybridization of the composition of claim 1 to the DNA sample, and
(v) comparing said visualization with that obtain from a control sample of a normal or standard BRCA1 or BRCA2 locus, BRCA1 or BRCA2 gene, BRCA1 or BRCA2 flanking sequence or intron that does not contain a rearrangement or mutation.

5. The method of claim 4, wherein said probes are selected to detect a rearrangement or mutation of more than 1.5 kb.

6. The method of any one of claims 4 or 5, further comprising:
predicting or assessing a predisposition to ovarian or breast cancer based on the kind of genetic rearrangement or mutation detected in a coding or noncoding BRCA1 or BRCA2 locus sequence; or
determining the sensitivity of a subject to a therapeutic treatment based on the kind of genetic rearrangement or mutation detected in a coding or noncoding BRCA1 or BRCA 2 locus sequence.

7. A kit comprising a composition of probes for detecting a duplication, deletion, triplication, inversion, insertion, translocation or large rearrangement in a BRCA1 or BRCA2 locus, said kit comprising
a) at least fourteen colour-labeled polynucleotide probes, specific of said BRCA1 or BRCA2 locus respectively, wherein each of said probe contains at least 200 contiguous nucleotides and the composition of probes comprises less than 10 % of the Alu repetitive nucleotide sequences present in said BRCA1 or BRCA2 locus of the reference genome Build GRCh37/hg19, wherein said probes are tagged with visually detectable markers and are selected to identify a duplication, deletion, inversion, insertion, translocation or large rearrangement in a particular segment of a BRCA1 or BRCA2 locus, in particular within the BRCA1 or BRCA2 gene or a BRCA1 or BRCA2 flanking sequence or intron, wherein said at least fourteen colour-labeled polynucleotide probes specific of said BRCA1 locus are:
(i) probes amplified by PCR using pairs of primers, wherein said pairs of primers are selected from the group consisting of the sequences of SEQ ID NO: 1 and SEQ ID NO: 2, the sequences of SEQ ID NO: 3 and SEQ ID NO: 4, the sequences of SEQ ID NO: 5 and SEQ ID NO: 6, the sequences of SEQ ID NO: 7 and SEQ ID NO: 8, the sequences of SEQ ID NO: 9 and SEQ ID NO: 10, the sequences of SEQ ID NO: 11 and SEQ ID NO: 12, the sequences of SEQ ID NO: 13 and SEQ ID NO: 14, the sequences of SEQ ID NO: 15 and SEQ ID NO: 16, the sequences of SEQ ID NO: 17 and SEQ ID NO: 18, the sequences of SEQ ID NO: 19 and SEQ ID NO: 20, the sequences of SEQ ID NO: 21 and SEQ ID NO: 22, the sequences of SEQ ID NO: 23 and SEQ ID NO: 24, the sequences of SEQ ID NO: 25 and SEQ ID NO: 26, the sequences of SEQ ID NO: 27 and SEQ ID NO: 28, the sequences of SEQ ID NO: 29 and SEQ ID NO: 30, the sequences of SEQ ID NO: 31 and SEQ ID NO: 32, the sequences of SEQ ID NO: 33 and SEQ ID NO: 34, the sequences of SEQ ID NO: 35 and SEQ ID NO: 36, the sequences of SEQ ID NO: 37 and SEQ ID NO: 38, the sequences of SEQ ID NO: 39 and SEQ ID NO: 40, the sequences of SEQ ID NO: 41 and SEQ ID NO: 42, the sequences of SEQ ID NO: 43 and SEQ ID NO: 44, the sequences of SEQ ID NO: 45 and SEQ ID NO: 46, the sequences of SEQ ID NO: 47 and SEQ ID NO: 48, the sequences of SEQ ID NO: 49 and SEQ ID NO: 50, the sequences of SEQ ID NO: 51 and SEQ ID NO: 52, the sequences of SEQ ID NO: 53 and SEQ ID NO: 54, the sequences of SEQ ID NO: 55 and SEQ ID NO: 56, the sequences of SEQ ID NO: 57 and SEQ ID NO: 58, the sequences of SEQ ID NO: 59 and SEQ ID NO: 60, the sequences of SEQ ID NO: 61 and SEQ ID NO: 62, the sequences of SEQ ID NO: 63 and SEQ ID NO: 64, the sequences of SEQ ID NO: 65 and SEQ ID NO: 66, the sequences of SEQ ID NO: 67 and SEQ ID NO: 68, the sequences of SEQ ID NO: 69 and SEQ ID NO: 70, the sequences of SEQ ID NO: 125 and SEQ ID NO: 126, the sequences of SEQ ID NO: 127 and SEQ ID NO: 128, and the sequences of SEQ ID NO: 129 and SEQ ID NO: 130; or
(ii) probes comprising the sequence of SEQ ID NO: 131 or the sequence of SEQ ID NO: 132 or the sequence of SEQ ID NO: 133; and
wherein said at least fourteen colour-labeled polynucleotide probes specific of said BRCA2 locus are probes amplified by PCR using pairs of primers, wherein said pairs of primers are selected from the group consisting of the sequences of SEQ ID NO: 71 and SEQ ID NO: 72, the sequences of SEQ ID NO: 73 and SEQ ID NO: 74, the sequences of SEQ ID NO: 75 and SEQ ID NO: 76, the sequences of SEQ ID NO: 77 and SEQ ID NO: 78, the sequences of SEQ ID NO: 79 and SEQ ID NO: 80, the sequences of SEQ ID NO: 81 and SEQ ID NO: 82, the sequences of SEQ ID NO: 83 and SEQ ID NO: 84, the sequences of SEQ ID NO: 85 and SEQ ID NO: 86, the sequences of SEQ ID NO: 87 and SEQ ID NO: 88, the sequences of SEQ ID NO: 89 and SEQ ID NO: 90, the sequences of SEQ ID NO: 91 and SEQ ID NO: 92, the sequences of SEQ ID NO: 93 and SEQ ID NO: 94, the sequences of SEQ ID NO: 95 and SEQ ID NO: 96, the sequences of SEQ ID NO: 97 and SEQ ID NO: 98, the sequences of SEQ ID NO: 99 and SEQ ID NO: 100, the sequences of SEQ ID NO: 101 and SEQ ID NO: 102, the sequences of SEQ ID NO: 103 and SEQ ID NO: 104, the sequences of SEQ ID NO: 105 and SEQ ID NO: 106, the sequences of SEQ ID NO: 107 and SEQ ID NO: 108, the sequences of SEQ ID NO: 109 and SEQ ID NO: 110, the sequences of SEQ ID NO: 111 and SEQ ID NO: 112, the sequences of SEQ ID NO: 113 and SEQ ID NO: 114, the sequences of SEQ ID NO: 115 and SEQ ID NO: 116, the sequences of SEQ ID NO: 117 and SEQ ID NO: 118, the sequences of SEQ ID NO: 119 and SEQ ID NO: 120, the sequences of SEQ ID NO: 121 and SEQ ID NO: 122, and the sequences of SEQ ID NO: 123 and SEQ ID NO: 124; and optionally,
b) a standard describing a hybridization profile for a subject not having a duplication, deletion, inversion, insertion, translocation or large rearrangement in a BRCA1 or BRCA2 locus, BRCA1 or BRCA2 gene, BRCA1 or BRCA2 flanking sequence or intron;
c) one or more elements necessary to perform Molecular Combing,
d) instructions for use, and/or
e) packaging materials.

## Patentansprüche

1. Anordnung von Gensonden zur gleichzeitigen Detektion von einer oder mehreren großen oder komplexen Mutationen oder genetischen Umgruppierungen in dem BRCA1- oder BRCA2-Lokus, wobei die genannte Anordnung wenigstens vierzehn farblich markierte Polynukleotid-Gensonden umfasst, die jeweils spezifisch für den genannten BRCA1- oder BRCA2-Lokus sind, wobei jede der genannten Gensonden wenigstens 200 benachbarte Nukleotide enthält und visuell bei hoher Auflösung detektierbar ist, wobei die genannte Anordnung von Gensonden weniger als 10 % der Alu-repetitiven Nukleotidsequenzen umfasst, die in dem genannten BRCA1- oder BRAC2-Lokus des Referenzgenoms Aufbau GRC37/hg19 umfasst, und wobei die genannten Mutationen aus der Gruppe ausgewählt sind, bestehend aus Duplikation, Triplikation, Deletion, Inversion, Insertion, Translokation und großen Umgruppierungen,
wobei die genannten wenigstens vierzehn farblich markierten Polynukleotid-Gensonden, die für den genannten BRCA1-Lokus spezifisch sind, sind:
(i) Sonden, die durch PCR verwendende Primerpaare amplifiziert werden, wobei die genannten Primerpaare aus der Gruppe ausgewählt sind, bestehend aus den Sequenzen SEQ ID NR. 1 und SEQ ID NR. 2, den Sequenzen SEQ ID NR. 3 und SEQ ID NR. 4, den Sequenzen SEQ ID NR. 5 und SEQ ID NR. 6, den Sequenzen SEQ ID NR. 7 und SEQ ID NR. 8, den Sequenzen SEQ ID NR. 9 und SEQ ID NR. 10, den Sequenzen ID NR. 11 und SEQ ID NR. 12, den Sequenzen SEQ ID NR. 13 und SEQ ID NR. 14, den Sequenzen SEQ ID NE. 15 und SEQ ID NR. 16, den Sequenzen SEQ ID NR. 17 und SEQ ID NR. 18, den Sequenzen SEQ ID NR. 19 und SEQ ID NR. 20, den Sequenzen ID NR. 21 und SEQ ID NR. 22, den Sequenzen SEQ ID NR. 23 und SEQ ID NR. 24, den Sequenzen SEQ ID NR. 25 und SEQ ID NR. 26, den Sequenzen SEQ ID NR. 27 und SEQ ID NR. 28, den Sequenzen SEQ ID NR. 29 und SEQ ID NR. 30, den Sequenzen SEQ ID NR. 31 und SEQ ID NR. 32, den Sequenzen SEQ ID NR. 33 und SEQ ID NR. 34, den Sequenzen SEQ ID NR. 35 und SEQ ID NR. 36, den Sequenzen SEQ ID NR. 37 und SEQ ID NR. 38, den Sequenzen SEQ ID NR. 39 und SEQ ID NR. 40, den Sequenzen SEQ ID NR. 41 und SEQ ID NR. 42, den Sequenzen SEQ ID NR. 43 und SEQ ID NR. 44, den Sequenzen ID NR. 45 und SEQ ID NR. 46, den Sequenzen ID NR. 47 und SEQ ID NR. 48, den Sequenzen SEQ ID NR. 49 und SEQ ID NR. 50, den Sequenzen SEQ ID NR. 51 und SEQ ID NR. 52, den Sequenzen SEQ ID NR. 53 und SEQ ID NR. 54, den Sequenzen SEQ ID NR. 55 und SEQ ID NR. 56, den Sequenzen SEQ ID NR. 57 und SEQ ID NR. 58, den Sequenzen SEQ ID NR. 59 und SEQ ID NR. 60, den Sequenzen SEQ ID NR. 61 und SEQ ID NR. 62, den Sequenzen SEQ ID NR. 63 und SEQ ID NR. 64, den Sequenzen SEQ ID NR. 65 und SEQ ID NR. 66, den Sequenzen SEQ ID NR. 67 und SEQ ID NR. 68, den Sequenzen SEQ ID NR. 69 und SEQ ID NR. 70, den Sequenzen SEQ ID NR. 125 und SEQ ID NR. 126, den Sequenzen SEQ ID NR. 127 und SEQ ID NR. 128 und den Sequenzen SEQ ID NR. 129 und SEQ ID NR. 130 oder
(ii) Gensonden, umfassend die Sequenz SEQ ID NR. 131 oder die Sequenz SEQ ID NR. 132 oder die Sequenz SEQ ID NR. 133; und
wobei die genannten, wenigstens vierzehn farblich markierten Polynukleotid-Gensonden, die spezifisch für den genannten BRCA2-Lokus sind, Gensonden sind, die durch Primerpaare verwendendes PCR amplifiziert werden, wobei die genannten Primerpaare aus der Gruppe ausgewählt sind, bestehend aus den Sequenzen SEQ ID NR. 71 und SEQ ID NR. 72, den Sequenzen SEQ ID NR. 73 und SEQ ID NR. 74, den Sequenzen SEQ ID NR. 75 und SEQ ID NR. 76, den Sequenzen SEQ ID NR. 77 und SEQ ID NR. 78, den Sequenzen SEQ ID NR. 79 und SEQ ID NR. 80, den Sequenzen SEQ ID NR. 81 und SEQ ID NR. 82, den Sequenzen SEQ ID NR. 83 und SEQ ID NR. 84, den Sequenzen SEQ ID NR. 85 und SEQ ID NR. 86, den Sequenzen SEQ ID NR. 87 und SEQ ID NR. 88, den Sequenzen SEQ ID NR. 89 und SEQ ID NR. 90, den Sequenzen SEQ ID NR. 91 und SEQ ID NR. 92, den Sequenzen SEQ ID NR. 93 und SEQ ID NR. 94, den Sequenzen SEQ ID NR. 95 und SEQ ID NR. 96, den Sequenzen SEQ ID NR. 97 und SEQ ID NR. 98, den Sequenzen SEQ ID NR. 99 und SEQ ID NR. 100, den Sequenzen SEQ ID NR. 101 und SEQ ID NR. 102, den Sequenzen SEQ ID NR. 103 und SEQ ID NR. 104, den Sequenzen SEQ ID NR. 105 und SEQ ID NR. 106, den Sequenzen SEQ ID NR. 107 und SEQ ID NR. 108, den Sequenzen SEQ ID NR. 109 und SEQ ID NR. 110, den Sequenzen SEQ ID NR. 111 und SEQ ID NR. 112, den Sequenzen SEQ ID NR. 113 und SEQ ID NR. 114, den Sequenzen SEQ ID NR. 115 und SEQ ID NR. 116, den Sequenzen SEQ ID NR. 117 und SEQ ID NR. 118, den Sequenzen SEQ ID NR. 119 und SEQ ID NR. 120, den Sequenzen SEQ ID NR. 121 und SEQ ID NR. 122 und den Sequenzen SEQ ID NR. 123 und SEQ ID NR. 124.

2. Anordnung gemäß Anspruch 1, wobei jede der genannten wenigsten vier Gensonden zwischen 500 und 6000 benachbarte Nukleotide enthält und weniger als 10 % Alu-repetitive Nukleotidsequenzen enthält.

3. Anordnung gemäß Anspruch 1 oder 2, umfassend wenigstens zwei, insbesondere wenigstens drei, insbesondere wenigstens vier Gensonden, die jeweils mit einem unterschiedlichen, detektierbaren Label oder Marker markiert sind.

4. Verfahren zum Detektieren einer Duplikation, Deletion, Inversion, Insertion, Translokation oder großen Umgruppierung in einem BRCA1- oder BRCA2-Lokus, BRCA1- oder BRCA2-Gen, einer BRCA1- oder BRCA2-flanikierenden Sequenz oder einem Intron, umfassend:
(i) Isolieren einer DNA-Probe,
(ii) molekulares Kombinieren der genannten Probe,
(iii) Kontaktieren der molekular kombinierten DNA mit der Anordnung der Gensonden gemäß Anspruch 1, wobei die wenigstens vierzehn Gensonden jeweils mit einem detektierbaren Label oder Marker für einen Zeitraum unter für die Hybridisierung ausreichenden Bedingungen markiert sind,
(iv) Anzeige der Hybridisierung der Anordnung gemäß Anspruch 1 der DNA-Probe und
(v) Vergleichen der genannten Anzeige mit der, die von einer Kontrollprobe eines normalen oder standardmäßigen BRCA1- oder BRCA2-Lokus, BRAC1- oder BRCA2-Gens, einer BRCA1- oder BRCA2-flankierenden Sequenz oder eines Introns erhalten wird, das keine Umgruppierung oder Mutation enthält.

5. Verfahren gemäß Anspruch 4, wobei die genannten Gensonden ausgewählt sind, um eine Umgruppierung oder Mutation von mehr als 1,5 kB zu detektieren.

6. Verfahren gemäß einem der Ansprüche 4 bis 5, weiterhin umfassend:
Voraussage oder Bestimmung einer Prädisposition zu Eierstock- oder Brustkrebs, basierend auf der Art der genetischen Umgruppierung oder Mutation, die in einer kodierenden oder nicht kodierenden BRCA1- oder BRCA2-Lokussequenz detektiert wird; oder
Bestimmung der Sensibilität eines Probanden auf eine therapeutische Behandlung, basierend auf der Art der genetischen Umgruppierung oder Mutation, die in einer kodierenden oder nicht kodierenden BRCA1- oder BRCA2-Lokussequenz detektiert wird.

7. Kit, umfassend eine Anordnung von Gensonden zur Detektion einer Duplikation, Deletion, Triplikation, Inversion, Insertion, Translokation oder großen Umgruppierung in einem BRCA1- oder BRCA2-Lokus, wobei das genannte Kit umfasst:
a) wenigstens vierzehn farblich markierte Polynukleotid-Gensonden, die jeweils spezifisch für den genannten BRCA1- oder BRCA2-Lokus sind, wobei jede der genannten Gensonden wenigstens 200 benachbarte Nukleotide enthält und die Anordnung von Gensonden weniger als 10 % der Alu-repetitiven Nukleotidsequenzen in dem genannten BRCA1- oder BRCA2-Lokus des Referenzgenoms Aufbau GRCh37/hg19 aufweist, wobei die genannten Gensonden mit visuell detektierbaren Markern markiert sind und ausgewählt sind, um eine Duplikation, Deletion, Inversion, Insertion, Translokation oder große Umgruppierung in einem bestimmten Segment eines BRCA1- oder BRCA2-Lokus, insbesondere innerhalb des BRCA1- oder BRCA2-Gens oder einer BRCA1- oder BRCA2-flankierenden Sequenz oder eines Introns zu identifizieren, wobei die genannten wenigstens vierzehn -farblich markierten Polynukleotid-Gensonden, die spezifisch für den genannten BRCA1-Lokus sind, sind:
(i) durch Primerpaare verwendendes PCR amplifizierte Gensonden, wobei die genannten Primerpaare aus der Gruppe ausgewählt sind, bestehend aus den Sequenzen SEQ ID NR. 1 und SEQ ID NR. 2, den Sequenzen SEQ ID NR. 3 und SEQ ID NR. 4, den Sequenzen SEQ ID NR. 5 und SEQ ID NR. 6, den Sequenzen SEQ ID NR. 7 und SEQ ID NR. 8, den Sequenzen SEQ ID NR. 9 und SEQ ID NR. 10, den Sequenzen SEQ ID NR. 11 und SEQ ID NR. 12, den Sequenzen SEQ ID NR. 13 und SEQ ID NR. 14, den Sequenzen SEQ ID NR. 15 und SEQ ID NR. 16, den Sequenzen SEQ ID NR. 17 und SEQ ID NR. 18, den Sequenzen SEQ ID NR. 19 und SEQ ID NR. 20, den Sequenzen SEQ ID NR. 21 und SEQ ID NR. 22, den Sequenzen ID NR. 23 und SEQ ID NR. 24, den Sequenzen SEQ ID NR. 25 und SEQ ID NR. 26, den Sequenzen SEQ ID NR. 27 und SEQ ID NR. 28, den Sequenzen SEQ ID NR. 29 und SEQ ID NR. 30, den Sequenzen SEQ ID NR. 31 und SEQ ID NR. 32, den Sequenzen SEQ ID NR. 33 und SEQ ID NR. 34, den Sequenzen SEQ ID NR. 35 und SEQ ID NR. 36, den Sequenzen SEQ ID NR. 37 und SEQ ID NR. 38, den Sequenzen ID NR. 39 und SEQ ID NR. 40, den Sequenzen SEQ ID NR. 41 und SEQ ID NR. 42, den Sequenzen SEQ ID NR. 43 und SEQ ID NR. 44, den Sequenzen SEQ ID NR. 45 und SEQ ID NR. 46, den Sequenzen SEQ ID NR. 47 und SEQ ID NR. 48, den Sequenzen SEQ ID NR. 49 und SEQ ID NR. 50, den Sequenzen SEQ ID NR. 51 und SEQ ID NR. 52, den Sequenzen SEQ ID NR. 53 und SEQ ID NR. 54, den Sequenzen SEQ ID NR. 55 und SEQ ID NR. 56, den Sequenzen SEQ ID NR. 57 und SEQ ID NR. 58, den Sequenzen SEQ ID NR. 59 und SEQ ID NR. 60, den Sequenzen SEQ ID NR. 61 und SEQ ID NR. 62, den Sequenzen SEQ ID NR. 63 und SEQ ID NR. 64, den Sequenzen SEQ ID NR. 65 und SEQ ID NR. 66, den Sequenzen SEQ ID NR. 67 und SEQ ID NR. 68, den Sequenzen SEQ ID NR. 69 und SEQ ID NR. 70, den Sequenzen SEQ ID NR. 125 und SEQ ID NR. 126, den Sequenzen SEQ ID NR. 127 und SEQ ID NR. 128 und den Sequenzen SEQ ID NR. 129 und SEQ ID NR. 130; oder
(ii) Gensonden, die die Sequenz SEQ ID NR. 131 oder die Sequenz SEQ ID NR. 132 oder die Sequenz ID NR. 133 umfassen; und
wobei die genannten wenigstens vierzehn farblich markierten Polynukleotid-Gensonden, die spezifisch für den genannten BRCA2-Lokus sind, durch Primerpaare verwendendes PCR amplifizierte Gensonden sind, wobei die genannten Primerpaare ausgewählt sind aus der Gruppe bestehend aus den Sequenzen SEQ ID NR. 71 und SEQ ID NR. 72, den Sequenzen SEQ ID NR. 73 und SEQ ID NR. 74, den Sequenzen SEQ ID NR. 75 und SEQ ID NR. 76, den Sequenzen SEQ ID NR. 77 und SEQ ID NR. 78, den Sequenzen SEQ ID NR. 79 und SEQ ID NR. 80, den Sequenzen SEQ ID NR. 81 und SEQ ID NR. 82, den Sequenzen SEQ ID NR. 83 und SEQ ID NR. 84, den Sequenzen SEQ ID NR. 85 und SEQ ID NR. 86, den Sequenzen SEQ ID NR. 87 und SEQ ID NR. 88, den Sequenzen SEQ ID NR. 89 und SEQ ID NR. 90, den Sequenzen 91 und SEQ ID NR. 92, den Sequenzen SEQ ID NR. 93 und SEQ ID NR. 94, den Sequenzen SEQ ID NR. 95 und SEQ ID NR. 96, den Sequenzen SEQ ID NR. 97 und SEQ ID NR. 98, den Sequenzen SEQ ID NR. 99 und SEQ ID NR. 100, den Sequenzen SEQ ID NR. 101 und SEQ ID NR. 102, den Sequenzen SEQ ID NR. 103 und SEQ ID NR. 104, den Sequenzen SEQ ID NR. 105 und SEQ ID NR. 106, den Sequenzen SEQ ID NR. 107 und SEQ ID NR. 108, den Sequenzen SEQ ID NR. 109 und SEQ ID NR. 110, den Sequenzen SEQ ID NR. 111 und SEQ ID NR. 112, den Sequenzen SEQ ID NR. 113 und SEQ ID NR. 114, den Sequenzen SEQ ID NR. 115 und SEQ ID NR. 116, den Sequenzen SEQ ID NR. 117 und SEQ ID NR. 118, den Sequenzen SEQ ID NR. 119 und SEQ ID NR. 120, den Sequenzen SEQ ID NR. 121 und SEQ ID NR. 122 und den Sequenzen SEQ ID NR. 123 und SEQ ID NR. 124 und optional
b) einen Standard, der ein Hybridisierungsprofil für einen Probanden beschreibt, das keine Duplikation, Deletion, Inversion, Insertion, Translokation oder große Umgruppierung in einem BRCA1- oder BRCA2-Lokus, BRCA1- oder BRCA2-Gen, einer BRCA1- oder BRAC2-flankierenden Sequenz oder einem Intron aufweist;
c) ein oder mehrere Element(e), das / die zur Durchführung einer molekularen Kombination notwendig ist / sind,
d) Gebrauchsanweisung(en) und / oder
e) Verpackungsmaterial.

## Revendications

1. Composition de sondes pour détecter simultanément un(e) ou plusieurs réarrangement(s) ou mutations génétiques grand(e)s ou complexe(s) dans le locus de BRCA1 ou de BRCA2, ladite composition comprenant au moins quatorze sondes polynucléotidiques colorées, respectivement, spécifiques du locus de BRCA1 ou de BRCA2, dans laquelle chacune desdites sondes contient au moins 200 nucléotides contigus et est visuellement détectable à haute résolution, dans laquelle ladite composition de sondes comprend moins de 10 % des séquences nucléotidiques répétées Alu présentes dans ledit locus de BRCA1 ou de BRCA2 du génome de référence Build GRCh37/hg19, et dans laquelle lesdites mutations sont sélectionnées, dans le groupe consistant en une duplication, triplication, délétion, inversion, insertion, translocation et de grands réarrangements,
dans laquelle lesdites au moins quatorze sondes polynucléotidiques colorées, spécifiques dudit locus de BRCA1 sont :
(i) des sondes amplifiées par PCR utilisant des paires d'amorces, dans lesquelles lesdites paires d'amorces sont sélectionnées dans le groupe consistant en les séquences de SEQ ID NO : 1 et de SEQ ID NO : 2, séquences de SEQ ID NO : 3 et de SEQ ID NO : 4, séquences de SEQ ID NO : 5 et de SEQ ID NO : 6, séquences de SEQ ID NO : 7 et de SEQ ID NO : 8, séquences de SEQ ID NO : 9 et de SEQ ID NO : 10, séquences de SEQ ID NO : 11 et de SEQ ID NO : 12, séquences de SEQ ID NO : 13 et de SEQ ID NO : 14, séquences de SEQ ID NO : 15 et de SEQ ID NO : 16, séquences de SEQ ID NO : 17 et de SEQ ID NO : 18, séquences de SEQ ID NO : 19 et de SEQ ID NO : 20, séquences de SEQ ID NO : 21 et de SEQ ID NO : 22, séquences de SEQ ID NO : 23 et de SEQ ID NO : 24, séquences de SEQ ID NO : 25 et de SEQ ID NO : 26, séquences de SEQ ID NO : 27 et de SEQ ID NO : 28, séquences de SEQ ID NO : 29 et de SEQ ID NO : 30, séquences de SEQ ID NO : 31 et de SEQ ID NO : 32, séquences de SEQ ID NO : 33 et de SEQ ID NO : 34, séquences de SEQ ID NO : 35 et de SEQ ID NO : 36, séquences de SEQ ID NO : 37 et de SEQ ID NO : 38, séquences de SEQ ID NO : 39 et de SEQ ID NO : 40, séquences de SEQ ID NO : 41 et de SEQ ID NO : 42, séquences de SEQ ID NO : 43 et de SEQ ID NO : 44, séquences de SEQ ID NO : 45 et de SEQ ID NO : 46, séquences de SEQ ID NO : 47 et de SEQ ID NO : 48, séquences de SEQ ID NO : 49 et de SEQ ID NO : 50, séquences de SEQ ID NO : 51 et de SEQ ID NO : 52, séquences de SEQ ID NO : 53 et de SEQ ID NO : 54, séquences de SEQ ID NO : 55 et de SEQ ID NO : 56, séquences de SEQ ID NO : 57 et de SEQ ID NO : 58, séquences de SEQ ID NO : 59 et de SEQ ID NO : 60, séquences de SEQ ID NO : 61 et de SEQ ID NO : 62, séquences de SEQ ID NO : 63 et de SEQ ID NO : 64, séquences de SEQ ID NO : 65 et de SEQ ID NO : 66, séquences de SEQ ID NO : 67 et de SEQ ID NO : 68, séquences de SEQ ID NO : 69 et de SEQ ID NO : 70, séquences de SEQ ID NO : 125 et de SEQ ID NO : 126, séquences de SEQ ID NO : 127 et de SEQ ID NO : 128, et séquences de SEQ ID NO : 129 et de SEQ ID NO : 130 ; ou
(ii) des sondes comprenant la séquence de SEQ ID NO : 131 ou la séquence de SEQ ID NO : 132 ou la séquence de SEQ ID NO : 133 ; et
dans lesquelles lesdites au moins quatorze sondes polynucléotidiques colorées, spécifiques dudit locus de BRCA2 sont des sondes amplifiées par PCR en utilisant des paires d'amorces, dans lesquelles lesdites paires d'amorces sont sélectionnées dans le groupe consistant en les séquences de SEQ ID NO : 71 et de SEQ ID NO : 72, séquences de SEQ ID NO : 73 et de SEQ ID NO : 74, séquences de SEQ ID NO : 75 et de SEQ ID NO : 76, séquences de SEQ ID NO : 77 et de SEQ ID NO : 78, séquences de SEQ ID NO : 79 et de SEQ ID NO : 80, séquences de SEQ ID NO : 81 et de SEQ ID NO : 82, séquences de SEQ ID NO : 83 et de SEQ ID NO : 84, séquences de SEQ ID NO : 85 et de SEQ ID NO : 86, séquences de SEQ ID NO : 87 et de SEQ ID NO : 88, séquences de SEQ ID NO : 89 et de SEQ ID NO : 90, séquences de SEQ ID NO : 91 et de SEQ ID NO : 92, séquences de SEQ ID NO : 93 et de SEQ ID NO : 94, séquences de SEQ ID NO : 95 et de SEQ ID NO : 96, séquences de SEQ ID NO : 97 et de SEQ ID NO : 98, séquences de SEQ ID NO : 99 et de SEQ ID NO : 100, séquences de SEQ ID NO : 101 et de SEQ ID NO : 102, séquences de SEQ ID NO : 103 et de SEQ ID NO : 104,
séquences de SEQ ID NO : 105 et de SEQ ID NO : 106, séquences de SEQ ID NO : 107 et de SEQ ID NO : 108, séquences de SEQ ID NO : 109 et de SEQ ID NO : 110, séquences de SEQ ID NO : 111 et de SEQ ID NO : 112, séquences de SEQ ID NO : 113 et de SEQ ID NO : 114, séquences de SEQ ID NO : 115 et de SEQ ID NO : 116, séquences de SEQ ID NO : 117 et de SEQ ID NO : 118, séquences de SEQ ID NO : 119 et de SEQ ID NO : 120, séquences de SEQ ID NO : 121 et de SEQ ID NO : 122, et séquences de SEQ ID NO : 123 et de SEQ ID NO : 124.

2. Composition de la revendication 1, dans laquelle chacune desdites au moins quatorze sondes contient de 500 à 6 000 nucléotides contigus et contient moins de 10 % de séquences nucléotidiques répétées Alu.

3. Composition de l'une quelconque des revendications 1 ou 2, comprenant au moins deux, en particulier, au moins trois, en particulier au moins quatre sondes portant chacune une étiquette ou un marqueur détectable différent(e).

4. Procédé de détection d'une duplication, délétion, inversion, insertion, translocation ou d'un grand réarrangement dans un locus de BRCA1 ou de BRCA2, un gène de BRCA1 ou de BRCA2, une séquence flanquante ou un intron de BRCA1 ou de BRCA2 comprenant :
(i) l'isolement d'un échantillon d'ADN,
(ii) le peignage moléculaire dudit échantillon,
(iii) la mise en contact de l'ADN peigné moléculairement avec la composition de sondes de la revendication 1, dans laquelle les au moins quatorze sondes portent chacune une étiquette ou un marqueur détectable pendant un temps et dans des conditions suffisantes pour réaliser une hybridation,
(iv) la visualisation de l'hybridation de la composition de la revendication 1 de l'échantillon d'ADN, et
(v) la comparaison de ladite visualisation avec celle obtenue à partir d'un échantillon témoin d'un locus de BRCA1 ou de BRCA2, d'un gène de BRCA1 ou de BRCA2, d'une séquence flanquante ou d'un intron de BRCA1 ou de BRCA2 ne contenant pas de réarrangement ou de mutation.

5. Procédé de la revendication 4, dans lequel lesdites sondes sont sélectionnées pour détecter un réarrangement ou une mutation de plus de 1,5 kb.

6. Procédé de l'une quelconque des revendications 4 ou 5, comprenant, en outre :
la prévision ou l'évaluation d'une prédisposition à un cancer du sein ou des ovaires basée sur le type de réarrangement ou de mutation génétique détectée dans une séquence codante ou non codante du locus de BRCA1 ou de BRCA2 ; ou
la détermination de la sensibilité d'un sujet à un traitement thérapeutique basé sur le type de réarrangement ou de mutation génétique détectée dans une séquence codante ou non codante du locus de BRCA1 ou de BRCA2.

7. Kit comprenant une composition de sondes pour détecter une duplication, délétion, triplication, inversion, insertion, translocation ou un grand réarrangement dans un locus de BRCA1 ou de BRCA2, ledit kit comprenant :
a) au moins quatorze sondes polynucléotidiques colorées, respectivement, spécifiques dudit locus de BRCA1 ou de BRCA2, dans lesquelles chacune desdites sondes contient au moins 200 nucléotides contigus, et la composition de sondes comprend moins de 10 % des séquences nucléotidiques répétées Alu présentes dans ledit locus de BRCA1 ou de BRCA2 du génome de référence Build GRCh37/hg19, dans lesquelles lesdites sondes portent des marqueurs visuellement détectables et sont sélectionnées pour identifier une duplication, délétion, inversion, insertion, translocation ou un grand réarrangement dans un segment particulier d'un locus de BRCA1 ou de BRCA2, en particulier, dans le gène de BRCA1 ou de BRCA2 ou une séquence flanquante ou un intron de BRCA1 ou de BRCA2, dans lequel (laquelle) lesdites au moins quatorze sondes polynucléotidiques colorées, spécifiques dudit locus de BRCA1 sont :
(i) des sondes amplifiées par PCR en utilisant des paires d'amorces, dans lesquelles lesdites paires d'amorces sont sélectionnées dans le groupe consistant en les séquences de SEQ ID NO : 1 et de SEQ ID NO : 2, séquences de SEQ ID NO : 3 et de SEQ ID NO : 4, séquences de SEQ ID NO : 5 et de SEQ ID NO : 6, séquences de SEQ ID NO : 7 et de SEQ ID NO : 8, séquences de SEQ ID NO : 9 et de SEQ ID NO : 10, séquences de SEQ ID NO : 11 et de SEQ ID NO : 12, séquences de SEQ ID NO : 13 et de SEQ ID NO : 14, séquences de SEQ ID NO : 15 et de SEQ ID NO : 16, séquences de SEQ ID NO : 17 et de SEQ ID NO : 18, séquences de SEQ ID NO : 19 et de SEQ ID NO : 20, séquences de SEQ ID NO : 21 et de SEQ ID NO : 22, séquences de SEQ ID NO : 23 et de SEQ ID NO : 24, séquences de SEQ ID NO : 25 et de SEQ ID NO : 26, séquences de SEQ ID NO : 27 et de SEQ ID NO : 28, séquences de SEQ ID NO : 29 et de SEQ ID NO : 30, séquences de SEQ ID NO : 31 et de SEQ ID NO : 32, séquences de SEQ ID NO : 33 et de SEQ ID NO : 34, séquences de SEQ ID NO : 35 et de SEQ ID NO : 36, séquences de SEQ ID NO : 37 et de SEQ ID NO : 38, séquences de SEQ ID NO : 39 et de SEQ ID NO : 40, séquences de SEQ ID NO : 41 et de SEQ ID NO : 42, séquences de SEQ ID NO : 43 et de SEQ ID NO : 44, séquences de SEQ ID NO : 45 et de SEQ ID NO : 46, séquences de SEQ ID NO : 47 et de SEQ ID NO : 48, séquences de SEQ ID NO : 49 et de SEQ ID NO : 50, séquences de SEQ ID NO : 51 et de SEQ ID NO : 52, séquences de SEQ ID NO : 53 et de SEQ ID NO : 54, séquences de SEQ ID NO : 55 et de SEQ ID NO : 56, séquences de SEQ ID NO : 57 et de SEQ ID NO : 58, séquences de SEQ ID NO : 59 et de SEQ ID NO : 60, séquences de SEQ ID NO : 61 et de SEQ ID NO : 62, séquences de SEQ ID NO : 63 et de SEQ ID NO : 64, séquences de SEQ ID NO : 65 et de SEQ ID NO : 66, séquences de SEQ ID NO : 67 et de SEQ ID NO : 68, séquences de SEQ ID NO : 69 et de SEQ ID NO : 70, séquences de SEQ ID NO : 125 et de SEQ ID NO : 126, séquences de SEQ ID NO : 127 et de SEQ ID NO : 128, et séquences de SEQ ID NO : 129 et de SEQ ID NO : 130 ; ou
(ii) des sondes comprenant la séquence de SEQ ID NO : 131 ou la séquence de SEQ ID NO : 132 ou la séquence de SEQ ID NO : 133 ; et
dans lesquelles lesdites au moins quatorze sondes polynucléotidiques colorées, spécifiques dudit locus de BRCA2 sont des sondes amplifiées par PCR en utilisant des paires d'amorces, dans lesquelles lesdites paires d'amorces sont sélectionnées dans le groupe consistant en les séquences de SEQ ID NO : 71 et de SEQ ID NO : 72, séquences de SEQ ID NO : 73 et de SEQ ID NO : 74, séquences de SEQ ID NO : 75 et de SEQ ID NO : 76, séquences de SEQ ID NO : 77 et de SEQ ID NO : 78, séquences de SEQ ID NO : 79 et de SEQ ID NO : 80, séquences de SEQ ID NO : 81 et de SEQ ID NO : 82, séquences de SEQ ID NO : 83 et de SEQ ID NO : 84, séquences de SEQ ID NO : 85 et de SEQ ID NO : 86, séquences de SEQ ID NO : 87 et de SEQ ID NO : 88, séquences de SEQ ID NO : 89 et de SEQ ID NO : 90,
séquences de SEQ ID NO : 91 et de SEQ ID NO : 92, séquences de SEQ ID NO : 93 et de SEQ ID NO : 94, séquences de SEQ ID NO : 95 et de SEQ ID NO : 96, séquences de SEQ ID NO : 97 et de SEQ ID NO : 98, séquences de SEQ ID NO : 99 et de SEQ ID NO : 100, séquences de SEQ ID NO : 101 et de SEQ ID NO : 102, séquences de SEQ ID NO : 103 et de SEQ ID NO : 104, séquences de SEQ ID NO : 105 et de SEQ ID NO : 106, séquences de SEQ ID NO : 107 et de SEQ ID NO : 108, séquences de SEQ ID NO : 109 et de SEQ ID NO : 110, séquences de SEQ ID NO : 111 et de SEQ ID NO : 112, séquences de SEQ ID NO : 113 et de SEQ ID NO : 114, séquences de SEQ ID NO : 115 et de SEQ ID NO : 116, séquences de SEQ ID NO : 117 et de SEQ ID NO : 118, séquences de SEQ ID NO : 119 et de SEQ ID NO : 120, séquences de SEQ ID NO : 121 et de SEQ ID NO : 122, et séquences de SEQ ID NO : 123 et de SEQ ID NO : 124 ; et, facultativement,
b) un standard décrivant un profil d'hybridation pour un sujet n'ayant pas de duplication, délétion, inversion, insertion, translocation ou de grand réarrangement dans, un locus de BRCA1 ou de BRCA2, un gène de BRCA1 ou de BRCA2, une séquence flanquante ou un intron de BRCA1 ou de BRCA2 ;
c) un ou plusieurs éléments nécessaires pour réaliser un peignage moléculaire,
d) des instructions d'utilisation, et / ou
e) des matériaux de conditionnement.


### SEQUENCE LISTING

<110> Genomic Vision
<120> Methods for the detection, visualization and high resolution physical mapping of genomic rearrangements in breast and ovarian cancer genes and loci BRCA1 and BRCA2 using genomic morse code in conjunction with molecular combing
<130> B10076A_PCT_AD/kn
<140> PCT/IB
   <141> 2012-10-30
<150> US 61/553,906
   <151> 2011-10-31
<160> 135
<170> PatentIn version 3.5
<210> 1
   <211> 20
   <212> DNA
   <213> Homo sapiens
<400> 1
   gggacggaaa gctatgatgt 20
<210> 2
   <211> 20
   <212> DNA
   <213> Homo sapiens
<400> 2
   gggcagaggt gacaggtcta 20
<210> 3
   <211> 20
   <212> DNA
   <213> Homo sapiens
<400> 3
   cctctgacct gatcccttga 20
<210> 4
   <211> 20
   <212> DNA
   <213> Homo sapiens
<400> 4
   atcagcaaca gtcccattcc 20
<210> 5
   <211> 23
   <212> DNA
   <213> Homo sapiens
<400> 5
   gcccagacta gtgtttctta acc 23
<210> 6
   <211> 20
   <212> DNA
   <213> Homo sapiens
<400> 6
   ggcatgaggc agcaatttag 20
<210> 7
   <211> 20
   <212> DNA
   <213> Homo sapiens
<400> 7
   tctttgaatc tgggctctgc 20
<210> 8
   <211> 21
   <212> DNA
   <213> Homo sapiens
<400> 8
   gctgttgctt tctttgaggt g 21
<210> 9
   <211> 20
   <212> DNA
   <213> Homo sapiens
<400> 9
   cacaggtatg tgggcagaga 20
<210> 10
   <211> 21
   <212> DNA
   <213> Homo sapiens
<400> 10
   cctctgttga tggggtcata g 21
<210> 11
   <211> 22
   <212> DNA
   <213> Homo sapiens
<400> 11
   tttggtagac caggtgaaat ga 22
<210> 12
   <211> 21
   <212> DNA
   <213> Homo sapiens
<400> 12
   caaattatgt gtggaggcag a 21
<210> 13
   <211> 21
   <212> DNA
   <213> Homo sapiens
<400> 13
   gaagaacgtg ctcttttcac g 21
<210> 14
   <211> 23
   <212> DNA
   <213> Homo sapiens
<400> 14
   aaagtctgat aacagctccg aga 23
<210> 15
   <211> 22
   <212> DNA
   <213> Homo sapiens
<400> 15
   ttcgattccc taagatcgtt tc 22
<210> 16
   <211> 27
   <212> DNA
   <213> Homo sapiens
<400> 16
   cacagttctg tgtaatttaa tttcgat 27
<210> 17
   <211> 21
   <212> DNA
   <213> Homo sapiens
<400> 17
   agggaaggct cagatacaaa c 21
<210> 18
   <211> 22
   <212> DNA
   <213> Homo sapiens
<400> 18
   tgccatagat agagggcttt tt 22
<210> 19
   <211> 20
   <212> DNA
   <213> Homo sapiens
<400> 19
   gccatcttct ttctcctgct 20
<210> 20
   <211> 22
   <212> DNA
   <213> Homo sapiens
<400> 20
   ttgacctatt gctgaatgtt gg 22
<210> 21
   <211> 22
   <212> DNA
   <213> Homo sapiens
<400> 21
   ttttaccaag gaaggatttt cg 22
<210> 22
   <211> 27
   <212> DNA
   <213> Homo sapiens
<400> 22
   gcttgatcac agatgtatgt atgagtt 27
<210> 23
   <211> 20
   <212> DNA
   <213> Homo sapiens
<400> 23
   ccccagggct ttaaaggtta 20
<210> 24
   <211> 20
   <212> DNA
   <213> Homo sapiens
<400> 24
   taggggtgga tatgggtgaa 20
<210> 25
   <211> 20
   <212> DNA
   <213> Homo sapiens
<400> 25
   acttcttcaa cgcgaagagc 20
<210> 26
   <211> 19
   <212> DNA
   <213> Homo sapiens
<400> 26
   gacaggctgt ggggtttct 19
<210> 27
   <211> 20
   <212> DNA
   <213> Homo sapiens
<400> 27
   tatctgctgg ccacttacca 20
<210> 28
   <211> 20
   <212> DNA
   <213> Homo sapiens
<400> 28
   tctcgagcct tgaacatcct 20
<210> 29
   <211> 20
   <212> DNA
   <213> Homo sapiens
<400> 29
   cgctcagctt tcattccagt 20
<210> 30
   <211> 23
   <212> DNA
   <213> Homo sapiens
<400> 30
   aaacgttcac atgtatcccc taa 23
<210> 31
   <211> 20
   <212> DNA
   <213> Homo sapiens
<400> 31
   cctggccagt acccagtagt 20
<210> 32
   <211> 20
   <212> DNA
   <213> Homo sapiens
<400> 32
   ctgagcccag agtttctgct 20
<210> 33
   <211> 20
   <212> DNA
   <213> Homo sapiens
<400> 33
   gggcccaaaa accagtaaga 20
<210> 34
   <211> 20
   <212> DNA
   <213> Homo sapiens
<400> 34
   gggattgagc gttcacagat 20
<210> 35
   <211> 20
   <212> DNA
   <213> Homo sapiens
<400> 35
   gccatccagt ccagtctcat 20
<210> 36
   <211> 21
   <212> DNA
   <213> Homo sapiens
<400> 36
   tgcagttcta ccctccactt g 21
<210> 37
   <211> 20
   <212> DNA
   <213> Homo sapiens
<400> 37
   cgggtaagtg gtgagctttc 20
<210> 38
   <211> 24
   <212> DNA
   <213> Homo sapiens
<400> 38
   gactgtcatt taaaggcact tttt 24
<210> 39
   <211> 20
   <212> DNA
   <213> Homo sapiens
<400> 39
   tggctagtgt tttggcctgt 20
<210> 40
   <211> 22
   <212> DNA
   <213> Homo sapiens
<400> 40
   ttcagtgttg cttctccatt tc 22
<210> 41
   <211> 22
   <212> DNA
   <213> Homo sapiens
<400> 41
   tgtcagacta gccacagtac ca 22
<210> 42
   <211> 21
   <212> DNA
   <213> Homo sapiens
<400> 42
   aagcgcttct tcatattctc c 21
<210> 43
   <211> 23
   <212> DNA
   <213> Homo sapiens
<400> 43
   accacactct tctgttttga tgt 23
<210> 44
   <211> 20
   <212> DNA
   <213> Homo sapiens
<400> 44
   ggcacatgta caccatggaa 20
<210> 45
   <211> 19
   <212> DNA
   <213> Homo sapiens
<400> 45
   ttgtgtaggt tgcccgttc 19
<210> 46
   <211> 20
   <212> DNA
   <213> Homo sapiens
<400> 46
   ttcagagagc tgggcctaaa 20
<210> 47
   <211> 20
   <212> DNA
   <213> Homo sapiens
<400> 47
   ggaggcaatc tggaattgaa 20
<210> 48
   <211> 20
   <212> DNA
   <213> Homo sapiens
<400> 48
   ggatccatga ttgctgcttt 20
<210> 49
   <211> 25
   <212> DNA
   <213> Homo sapiens
<400> 49
   tctctgctgt ttttacaact ttttc 25
<210> 50
   <211> 20
   <212> DNA
   <213> Homo sapiens
<400> 50
   ggatccatga ttgctgcttt 20
<210> 51
   <211> 25
   <212> DNA
   <213> Homo sapiens
<400> 51
   ccctctagat acttgtgtcc ttttg 25
<210> 52
   <211> 20
   <212> DNA
   <213> Homo sapiens
<400> 52
   tctggcagtc acaattcagg 20
<210> 53
   <211> 20
   <212> DNA
   <213> Homo sapiens
<400> 53
   tcccatgact gcatcatctt 20
<210> 54
   <211> 21
   <212> DNA
   <213> Homo sapiens
<400> 54
   ttgagatcag gtcgattcct c 21
<210> 55
   <211> 21
   <212> DNA
   <213> Homo sapiens
<400> 55
   aaaactcaac ccaaacagtc a 21
<210> 56
   <211> 22
   <212> DNA
   <213> Homo sapiens
<400> 56
   ccaagaatca cgaagagaga ga 22
<210> 57
   <211> 25
   <212> DNA
   <213> Homo sapiens
<400> 57
   gacctcatag aggtagtgga aagaa 25
<210> 58
   <211> 23
   <212> DNA
   <213> Homo sapiens
<400> 58
   gctcaaagcc tttagaagaa aca 23
<210> 59
   <211> 20
   <212> DNA
   <213> Homo sapiens
<400> 59
   gcactgggga aaaggtagaa 20
<210> 60
   <211> 21
   <212> DNA
   <213> Homo sapiens
<400> 60
   ctcttcaacc cagacagatg c 21
<210> 61
   <211> 24
   <212> DNA
   <213> Homo sapiens
<400> 61
   caatacccaa tacaatgtaa atgc 24
<210> 62
   <211> 21
   <212> DNA
   <213> Homo sapiens
<400> 62
   ctggggatac tgaaactgtg c 21
<210> 63
   <211> 20
   <212> DNA
   <213> Homo sapiens
<400> 63
   atcaagaagc cttcccaggt 20
<210> 64
   <211> 20
   <212> DNA
   <213> Homo sapiens
<400> 64
   tccttggacg taaggagctg 20
<210> 65
   <211> 23
   <212> DNA
   <213> Homo sapiens
<400> 65
   ttcagaactt ccaaatacgg act 23
<210> 66
   <211> 19
   <212> DNA
   <213> Homo sapiens
<400> 66
   gatggagctg gggtgaaat 19
<210> 67
   <211> 20
   <212> DNA
   <213> Homo sapiens
<400> 67
   cgtgagattg ctcacaggac 20
<210> 68
   <211> 20
   <212> DNA
   <213> Homo sapiens
<400> 68
   caaggcattg gaaaggtgtc 20
<210> 69
   <211> 23
   <212> DNA
   <213> Homo sapiens
<400> 69
   agaggaatag accatccaga agt 23
<210> 70
   <211> 21
   <212> DNA
   <213> Homo sapiens
<400> 70
   tcctccagca ctaaaaactg c 21
<210> 71
   <211> 20
   <212> DNA
   <213> Homo sapiens
<400> 71
   aaatggaggt cagggaacaa 20
<210> 72
   <211> 20
   <212> DNA
   <213> Homo sapiens
<400> 72
   tggaaagttt gggtatgcag 20
<210> 73
   <211> 20
   <212> DNA
   <213> Homo sapiens
<400> 73
   tctcaatgtg caaggcaatc 20
<210> 74
   <211> 21
   <212> DNA
   <213> Homo sapiens
<400> 74
   tcttgaccat gtggcaaata a 21
<210> 75
   <211> 19
   <212> DNA
   <213> Homo sapiens
<400> 75
   aatcacccca accttcagc 19
<210> 76
   <211> 20
   <212> DNA
   <213> Homo sapiens
<400> 76
   gcccaggaca aacattttca 20
<210> 77
   <211> 20
   <212> DNA
   <213> Homo sapiens
<400> 77
   ccctcgcatg tatgatctga 20
<210> 78
   <211> 20
   <212> DNA
   <213> Homo sapiens
<400> 78
   ctcctgaagt cctggaaacg 20
<210> 79
   <211> 22
   <212> DNA
   <213> Homo sapiens
<400> 79
   tgaaatcttt tccctctcat cc 22
<210> 80
   <211> 20
   <212> DNA
   <213> Homo sapiens
<400> 80
   agattgggca catcgaaaag 20
<210> 81
   <211> 21
   <212> DNA
   <213> Homo sapiens
<400> 81
   ggtcttgaac acctgctacc c 21
<210> 82
   <211> 19
   <212> DNA
   <213> Homo sapiens
<400> 82
   cactccgggg gtcctagat 19
<210> 83
   <211> 23
   <212> DNA
   <213> Homo sapiens
<400> 83
   tctttaactg ttctgggtca caa 23
<210> 84
   <211> 22
   <212> DNA
   <213> Homo sapiens
<400> 84
   tggctagaat tcaaaacact ga 22
<210> 85
   <211> 25
   <212> DNA
   <213> Homo sapiens
<400> 85
   ttgaagtggg gtttttaagt tacac 25
<210> 86
   <211> 20
   <212> DNA
   <213> Homo sapiens
<400> 86
   ccagccaatt caacatcaca 20
<210> 87
   <211> 21
   <212> DNA
   <213> Homo sapiens
<400> 87
   ttgggacaat tctgaggaaa t 21
<210> 88
   <211> 23
   <212> DNA
   <213> Homo sapiens
<400> 88
   tgcaggtttt gttaagagtt tca 23
<210> 89
   <211> 20
   <212> DNA
   <213> Homo sapiens
<400> 89
   tggcaaatga ctgcattagg 20
<210> 90
   <211> 21
   <212> DNA
   <213> Homo sapiens
<400> 90
   tcttgaaggc aaactcttcc a 21
<210> 91
   <211> 25
   <212> DNA
   <213> Homo sapiens
<400> 91
   ggaattgttg aagtcactga gttgt 25
<210> 92
   <211> 19
   <212> DNA
   <213> Homo sapiens
<400> 92
   accaccaaag ggggaaaac 19
<210> 93
   <211> 22
   <212> DNA
   <213> Homo sapiens
<400> 93
   caagtcttca gaatgccaga ga 22
<210> 94
   <211> 20
   <212> DNA
   <213> Homo sapiens
<400> 94
   taaaccccag gacaaacagc 20
<210> 95
   <211> 20
   <212> DNA
   <213> Homo sapiens
<400> 95
   ggctgtttgt tgaggagagg 20
<210> 96
   <211> 20
   <212> DNA
   <213> Homo sapiens
<400> 96
   gaaaccagga aatggggttt 20
<210> 97
   <211> 20
   <212> DNA
   <213> Homo sapiens
<400> 97
   tgttagggag gaaggagcaa 20
<210> 98
   <211> 24
   <212> DNA
   <213> Homo sapiens
<400> 98
   ggatgtaact tgttaccctt gaaa 24
<210> 99
   <211> 24
   <212> DNA
   <213> Homo sapiens
<400> 99
   tcaatagcat gaatctgttg tgaa 24
<210> 100
   <211> 20
   <212> DNA
   <213> Homo sapiens
<400> 100
   gaggtctgcc acaagtttcc 20
<210> 101
   <211> 19
   <212> DNA
   <213> Homo sapiens
<400> 101
   ggcccactgg aggtttaat 19
<210> 102
   <211> 24
   <212> DNA
   <213> Homo sapiens
<400> 102
   ttcctttcaa tttgtacaga aacc 24
<210> 103
   <211> 21
   <212> DNA
   <213> Homo sapiens
<400> 103
   tgaatcaatg tgtgtgtgca t 21
<210> 104
   <211> 20
   <212> DNA
   <213> Homo sapiens
<400> 104
   gtgtagggtc cagccctatg 20
<210> 105
   <211> 20
   <212> DNA
   <213> Homo sapiens
<400> 105
   ctgaggctag gaaagctgga 20
<210> 106
   <211> 20
   <212> DNA
   <213> Homo sapiens
<400> 106
   ctgaggctag gaaagctgga 20
<210> 107
   <211> 22
   <212> DNA
   <213> Homo sapiens
<400> 107
   ggtttatccc aggatagaat gg 22
<210> 108
   <211> 22
   <212> DNA
   <213> Homo sapiens
<400> 108
   agaaaatgtg gggtgtaaac ag 22
<210> 109
   <211> 20
   <212> DNA
   <213> Homo sapiens
<400> 109
   cagcaaactt cagccattga 20
<210> 110
   <211> 20
   <212> DNA
   <213> Homo sapiens
<400> 110
   gggacatggc aaccaaatac 20
<210> 111
   <211> 20
   <212> DNA
   <213> Homo sapiens
<400> 111
   gcactttcac gtcctttggt 20
<210> 112
   <211> 20
   <212> DNA
   <213> Homo sapiens
<400> 112
   cgtcgtattc aggagccatt 20
<210> 113
   <211> 19
   <212> DNA
   <213> Homo sapiens
<400> 113
   cccagctggc aaacttttt 19
<210> 114
   <211> 20
   <212> DNA
   <213> Homo sapiens
<400> 114
   tcggaggtaa ttcccatgac 20
<210> 115
   <211> 20
   <212> DNA
   <213> Homo sapiens
<400> 115
   tcaagagcca tgctgacatc 20
<210> 116
   <211> 20
   <212> DNA
   <213> Homo sapiens
<400> 116
   aggtagggtg gggaagaaga 20
<210> 117
   <211> 23
   <212> DNA
   <213> Homo sapiens
<400> 117
   tgagtctact ttgcccatag agg 23
<210> 118
   <211> 20
   <212> DNA
   <213> Homo sapiens
<400> 118
   ttttgctttc gggagcttta 20
<210> 119
   <211> 20
   <212> DNA
   <213> Homo sapiens
<400> 119
   tttttgcctg cttcatcctc 20
<210> 120
   <211> 22
   <212> DNA
   <213> Homo sapiens
<400> 120
   ggtttttaaa cctgcacatg aa 22
<210> 121
   <211> 23
   <212> DNA
   <213> Homo sapiens
<400> 121
   tgaaattttg ttatgtggtg cat 23
<210> 122
   <211> 23
   <212> DNA
   <213> Homo sapiens
<400> 122
   tttgaaatct gtggaggtct agc 23
<210> 123
   <211> 20
   <212> DNA
   <213> Homo sapiens
<400> 123
   gtaccaaggg tggcagaaag 20
<210> 124
   <211> 20
   <212> DNA
   <213> Homo sapiens
<400> 124
   atggtgttgg ttgggtagga 20
<210> 125
   <211> 25
   <212> DNA
   <213> Homo sapiens
<400> 125
   ttcagaaaat acatcaccca agttc 25
<210> 126
   <211> 22
   <212> DNA
   <213> Homo sapiens
<400> 126
   taccattgcc tcttacccac aa 22
<210> 127
   <211> 27
   <212> DNA
   <213> Homo sapiens
<400> 127
   aaccttgatt aacacttgag ctatttt 27
<210> 128
   <211> 20
   <212> DNA
   <213> Homo sapiens
<400> 128
   catgggcatt aattgcatga 20
<210> 129
   <211> 22
   <212> DNA
   <213> Homo sapiens
<400> 129
   cctgcatgct cataatgcta ga 22
<210> 130
   <211> 20
   <212> DNA
   <213> Homo sapiens
<400> 130
   ttgggatggg tttgaagaga 20
<210> 131
   <211> 3548
   <212> DNA
   <213> Homo sapiens
<400> 131
   gggacggaaa gctatgatgt caccaccgtc cgggtgggtg tgctggggtt caccctccca 60
   tttccccaag accccctgcc aggacatagg cggacgcggg agagaaaacc aaagaggctc 120
   cctccttccc cttagcatct ctctcccgcc gtgttcagga agtggatggc tgccccagct 180
   cttgtccgca ctggtacacc tgcgtgcacg cgtgggtaca cagcaggccc gagcttcgcg 240
   cttgtgccgc tcatattcta cccctaagaa cttcgcttga actctgacct gcccttatat 300
   ccgagaaagt caaataagcc cagttcggcc tgtcccaaac cggcaggggc ccctcagacc 360
   acaccggcgg gctggacccc ggctctgagg cctctgttcc cagggctccg cccagatctt 420
   ctgggccccg ccccccggct gcgggggtgg gaggaggggc cgggggggcg cggccgcctg 480
   gctgggggcg gggcggaggg ggggccgcgg acccggggcg ggggctcggc gcgggcccgc 540
   gagatgccgg tgttggcggc ccgagcggct gcagttgcag gggcggggga ggcggcggcg 600
   gggcccggga gaggggtggc gtgggggacc ggcgcgtagc cgggaccatg gaggggcaga 660
   gcggccgctg caagatcgtg gtggtgggag acgcagagtg cggcaagacg gcgctgctgc 720
   aggtgttcgc caaggacgcc tatcccgggg tgagggacct gcgtcttggg agggggacgc 780
   taaggctgct ggggggtggg tgacaggggc cctggcgacg gatgggaatg ggtactcggg 840
   taaccaggga caagagacag ggggtcggag gacgcgggga ggccttgagg gctcaggaag 900
   gactgcagag gattggggtg ggaggaatta gggagcaggg tgagatagat ggggtttggg 960
   agaaccagag catccgggag ggagggcgag gggaatgtcg gaggtcctgg gcaatggaga 1020
   ggggaagaac tagggggctg aagggaccag aagggaacag gaggaggtct gggagcttag 1080
   cagagattct ccgggggggg gggggggggg gcaggagctc ccgggatctc ccctttgccc 1140
   aatcccagac caacttgtgt ccaggggctg ggctggacgg ggtgtgggag tgaggagggc 1200
   atttatctgg ggtgaggact tggagagatg atctcatctg gatccatccg tgtctgcaga 1260
   gttatgtccc caccgtgttt gagaactaca ctgcgagctt tgagatcgac aagcgccgca 1320
   ttgagctcaa catgtgggac acttcaggta gccaagtccc tgggggtcac cctgacttcc 1380
   aaggcggccc actctgtccc ctcccttggt tagaccctta ggttccaggt aagcccagcc 1440
   catccatcca attccaacag gaagggaaaa atcaatattc tgctaaaatc cagggaaact 1500
   gaggtagaac ttgcagagcc tgacagaaac catgtcctga aggagaaagc ctaggatctg 1560
   agcccctcag ctgggtcctg cctacctggg aaagttggga aggaatggct tttaatttgg 1620
   aacatgttcc ttcagagata agactgggtt tagaaaagac atttagaggc caggcacggt 1680
   ggctcacgcc tgtaatccta gcactttggg aggctggggt ggggggatca cctgaggtca 1740
   ggagtttgag accagcctgg ccaacatggt tgaaactccg tctctactaa aaatacaaaa 1800
   attaatcggg cgtgtggcac gtgcctgtaa tctcagctac caggaggctg aggcaagaga 1860
   atcgctggaa cctgggaggc ggaggctgca gtgagccgag atcatgccgc tgcactccag 1920
   cctgagcgat agagcgagac tccatctcaa aaaataaaaa agcagaaaag acatttagaa 1980
   tgtcttgagt gaggggtggt caggaggctg tttctctcca ttgaactaga taaatctgag 2040
   gtcaagtccc aggagaatgg gagagtgctc tccctgccac tgctcttttc ctcctcccaa 2100
   cataaggagg gtttttattt ttacaagagt tcccttcagg gctttagact gccaaagccc 2160
   agaaagcaca tgcaacattt tatgagaatg tctatagatt ttatgagctt ctcaaagggg 2220
   tccaaacctc agtcaagaat aaaaattatt actttttaaa ccactaggga agcagagagc 2280
   cgtttcccac catgtgacct cccttctgcc cgctccccca cttgggaaac ccagactcca 2340
   tgatgggtat taatgatggg tattaatggt tgctcttttc cattctctgc tcccagcatc 2400
   ccttgaccag gatctgtaag gtctcccatt cccttccagg cctcccatcc actcaggccc 2460
   ctcatgccct gtcttccttc aggttcctct tactatgata atgtccggcc tctggcctat 2520
   cctgattctg atgctgtgct catctgcttc gacattagcc gaccagaaac actggacagt 2580
   gttctcaaga aggtgggagc ctggggaaat agggcagcta gactgagggg gaccagacca 2640
   ccatggtcct gacataacat gggccaggag gagggagtga tggctggggt atggccatca 2700
   gctggttagc gagtgaagct ctcatccctg ccacccctgc ctccagcccc catccctccc 2760
   agccacccct ttcctgaaag tcctcagagc tggatacagc agctagggga ggtgggggag 2820
   tgaagggaga agcactcaca ggattccttc tctgctcttc caactccttg gcagtgggag 2880
   tcccagatgg aggggatggg atgggaagcc tgatcctgga gctcaggaaa gccctgtggc 2940
   ctcctctcca ggccccagtt tccatgacaa aagccagggg tgaatggaca gaagtcagct 3000
   agggcagccc cagttcccag gtgggggagg ggagggtggg ataaatttgt tcccaggaga 3060
   gagtatggga aaggcgagtg ggaatgggaa gtttccaggc tggcagaccc ttcatagcca 3120
   ctgagggaga agagtccaca ggcccacgcc agccctctcc tccccgctgc ttctctctca 3180
   ccccatcctg ctctcaaacc aagcctagca ttctcacctc cttcctcatg tgggagagtc 3240
   ctgagggata catggtttct gcgtgcttga ggaagagagg gcacactgct ggcatggcac 3300
   aaaggctcac gctgtgcctc cctccacccc tccacaattc tcttttcttc tcctacatag 3360
   tggcaaggag agactcaaga gttctgcccc aatgccaagg ttgtgctggt tggctgtaaa 3420
   ctggacatgc ggactgacct ggccacactg agggagctgt ccaagcagag gcttatccct 3480
   gttacacatg agcaggtggg acccttgacg tctgacctca tcccagccta gacctgtcac 3540
   ctctgccc 3548
<210> 132
   <211> 3561
   <212> DNA
   <213> Homo sapiens
<400> 132
   cctctgacct gatcccttga ctgcccccag ccttgacatt caaccccagc ccacagcctc 60
   catgcccctt tctaagctgc aggctaagac ctataacttt ctcccatgca ctccttcctt 120
   ttccagggca ctgtgctggc caagcaggtg ggggctgtgt cctatgttga gtgctcctcc 180
   cggtcctctg agcgcagcgt cagggatgtc ttccatgtgg ctacagtggc ctcccttggc 240
   cgtggccata ggcagctgcg ccgaactgac tcacgccggg gaatgcagcg atccgctcag 300
   ctgtcaggac ggccagaccg ggggaatgag ggcgagatac acaaggatcg agccaaaagc 360
   tgcaacctca tgtgaggggc taggagaggg cagagtgtga agaggggtgg tgagggacac 420
   aattgttccc ctgcctgcgc ccaggcttcc tgacctcctg atcctggctg ggaagttagg 480
   gcaggcagag cgagcaattc tgggcagggg agctggaggg cagaagggta tcatcgtttc 540
   tcatctcctc ctccctcctc ttctccagtg gatgttgagg gagctaacag ggctggcatc 600
   tggggcatga actgggatgg ggcaggtggg cgttagggaa gctggtatca aatggtgacc 660
   ttggtggagt ctcctatgtg aagagtaccc tccctctcca cccccagtcc ccatatcctg 720
   gttctggccc aaggaaaatg tccattctat gaccttctct tttcctctcc tctcacttct 780
   gcagctattc tcacacatct aacctctagg caacatgcac taaattcaaa agcaaggaga 840
   agcccttgcc ccccatcagt ccaccagccc tagaacctcc cttgcctcaa cagtcaccta 900
   ataaagccca cctccatgga aaacggctgt ggctttagtt ttgttgcttt ttaaaaaaat 960
   caatctacca atctttagca gtaagaggga aagttagacc tcagctgggg aactttcctg 1020
   tccatgtcca cagatagagc agaggacaaa gccataggtt ggatcagaag tgtcctttta 1080
   ggagtcagag ttgggagaag gagacatcct gggactgttc atcctagtta atgaagtggg 1140
   caattctcag gccattaggg ggttttagag cagaccgaca tataattagt cagcatttct 1200
   cagcccagcc aggcctgctg ctagtgtggg aggggtcctg ctcaccatct gtacccctgg 1260
   cttggagcct gctggtaccc tgggggttgt ggggataagg aggcatcagg ccgggcgcgc 1320
   tggctcacgc ctgtaatccc agcactttgg gaggccgagg tgggcggatc acaagttcag 1380
   gagatcgaga ccatcctggc taacacggtg aaaccccatc tctactaaaa atacaaaaaa 1440
   ttagccaggc gcggtggcag tgcctgtagt cccagctgct cgggaggctg aggcaggaga 1500
   atggtgtgaa cccgggtgaa cctgggaggc ggagcttgca gtgagccgag attgcgccac 1560
   tgcattctag cctggatgac agagcaagac tctgtctcca aaaaaaaaaa aaaaaaaaaa 1620
   aaagaaggca tcaaaagcct ccacatcaca gaagctaccc ctgtacagcg tgaagtttcc 1680
   taagaggtca gtagtttgat tctggggtct ccttagaggc tcaggccagg gacctttctc 1740
   tcctcccatg ctgagtttca tgatggcttt caggggagca tcagctgtta gagtcacccc 1800
   taccctgtcc cttaaaggaa agacggtgga gaggacggct gagcgcctgt tgtcaggaaa 1860
   gacagtactg gtctgttttc tcgggagtct ggtttcagat tgtcctgtat tcccttcctg     1920
   gctctggtcc cactggcctc ttttcggtga cattctcccc caggaaccat ccctggccct     1980
   tccctccccc agccctagcc agttctccca gacacactgg aagagaacac tgaccttacc     2040
   caactatctg ctgggatccc acccaaattt atagcccatt cctccctcat tcattcattc     2100
   agcaagtatg tactgaacac caactgtgtg gcatacactg gcttgggaga ttgcaaggac     2160
   cagtctctaa gcttttggag gccagcccag tgtggaagag aggtacctca ggtgtgaggg     2220
   tgccatggct gagggatatt tgtacatgta tgggatgcta tgggagctcc ttgcagcctg     2280
   agaagccagt cctgtgagcc aggtcctgag ggttgaagag gagttttccg ggcagggaag     2340
   gggtaggaaa ggcactctgg gcagagggta cagcatgtgt aaacacgtgg agatgagaat     2400
   gagcatagca ctgttggggc tcccatggca gggagaatag aagacaaggc taggaaggta     2460
   cactgaggct actgcagggt ccacagagga atcagaattt cattctgagg atgaatgaaa     2520
   tcatcctcag aggatgaagc caccaggaat ttcaggcaga gagtgaagtg atcagagttg     2580
   ttttttggat agatggttat ctggatgtgg tgttggagct gggagatttg gctctgaggt     2640
   gtgtcattta aaataatagc ttctcggcag tggctcacac ctataatccc agccaagatt     2700
   cctcctttgg gaggccaagc tgggaggatc gcttgaggcc aggagttaga gactgcagtg     2760
   agctatgatc atgccattgt cttccagcct gagtgtcaga gtgagaccct gtctctaaaa     2820
   aaaattaaaa aataaaaaat aaaaaatagc ttctcctttc ccttatgcca ggttccagtc     2880
   ttgagaggaa aggaatccct acccaccact ccctggatca tcagatatcc ctatcccaac     2940
   ctctcctatg ggactagttc atctcagcca gtctcaaaga ttctaggata acttcaatgg     3000
   catttgaaat tatctaagtg tgcttggata accaccccct caaactgaga cctggttagg     3060
   gactgactca aagaccctga gtcctcggct aagggtacag gagagggcag gggctccagg     3120
   cccagctagg tggatctcca tctgtctctg aggactgacc ctttccccac aaggacctgc     3180
   cataaaaatc gacttgcgat ttttagctga gtggcttctc ttttccactt tggacttctc     3240
   agtgtatagc aggttcaagc ctgcaaccac caaagtgcag agtgtggagt gtttgtgccc     3300
   cctctttcct ccaacctcca tatcctgcca tgtgagctca gggaatgcaa atgcatttaa     3360
   atatccatct aaagcaaaca taattagaaa aatcaatcag ctggaggacc ccccaaagtt     3420
   taatacattt tcaataccac caggaatgga tttttggtcc ctttctgcag gtctgggttg     3480
   ccagacgttt tatttctggg gaggagggct ctgggctgag gagctcagtg ggtgggagga     3540
   gggaatggga ctgttgctga t                                               3561
<210> 133
   <211> 4485
   <212> DNA
   <213> Homo sapiens
<400> 133
   ttcagaaaat acatcaccca agttcccatc cctacctgtc tatccacaaa accaaggcat        60
   tcctgagatt agttcattta ttatactaat ataacaagtg tttattaagt atctactact       120
   atattcaagt actattctag gagatagaaa tgtagcagtt tacaaaataa agcctgctct       180
   catagagctc atattctagt gtggtagaca gttgatacgg aattaaagaa tacatgggaa       240
   taagtgcatt aaagagaaaa attaagcagg gtaaggggaa acaggtagtt caatatctat       300
   gtgggggtga gatgtacatg gggggagtca ggaaaggttt cactgaggtg agactagagg       360
   atagcttaat aatgtaaaga aacacactat gcaacaatta ggggaagagc attccaagaa       420
   agagggagca gagaaggcaa accctgagca ggaccatgcc tgtgtatgca ggacatcaga       480
   taggtcaagg tgctaaaatg taataatcca ggaggatatt gtagggaaag actatcagag       540
   aggtagctgg taacttctgg taggaaccta taggctattt taaatcttta gctttattct       600
   ggtcttttta attttctttt tttttttcag acagagtctc gttctgtcgc ccaggctgga       660
   gtgcagtggc accatctcgg ctctctgtaa cctccgcctc ctgaattcaa gtgattctcc       720
   tgcctcagcc tcccgagtag ctgggactaa aggcatgcac caccatgcct tggcctccca       780
   aagtactggg attacaggag tgagccacca tgccagccat ctttttaatt tttaatgtta       840
   attaattttt gtagagacag gatctcacta tgatgcccat gctggtcttg aatgcctggc       900
   atcaagcaat cttcctgctt cggcttccca aagtgctggg attacaggtg tgagctacta       960
   tacccggcct ttagctttct tctgaatgtg aacctttttt tttttttttg gagatggagt      1020
   ctcactcact ctgctgctca ggctggagtg cagtggtgtg gtcttggctc actgcaacct      1080
   ctgcctctcg gattgaagtg attcttgtgc ctcagcattc caagtagctg ggactacagg      1140
   cgcgtgctgc cacacccggc taattttttt gtatttttgg tagggaaggg gtttcaccat      1200
   attgcccagg ctggtcttga agtcctgacc tcaagtgatc catctgcctc gaccgggatt      1260
   acaggcgtga gccactacac ttagctctaa atgtgaattt ttgaaacgga ttttttggat      1320
   aaagtccagg caagatatca aagaacgact aacctggcag tgtgacaaga atgtggtttt      1380
   ttccttaaat atttaacttt ttagaaaagg atcacaaggg ccaggtgcgg tggctcacgc      1440
   tgtaatccca gcattttggg aggccaaggc gggccagcct gggtgacaga gaatccatct      1500
   caaaaaaaga aaaaaaaaaa agaaaaggat cacaagaaaa gcttgtggac agtaacctta      1560
   ttgtgaaggg ttgtaataca actcttgtaa tcatggggtt tttgacatag cacagggcag      1620
   tgaaaagaaa aacaatgaac taagtcagga ggctgggttt ctactaccag ttgtgtatat      1680
   aagcagagcc accttgggct aaccactcta cctgaacctg tttccttctc ttgccattca      1740
   ccctgccaga ctccttgggc tattgcaaga ataaaattaa atgctacttg ggaaaatgct      1800
   tcacaacctg agatgacttg ggaaaaatgc ttcacaacct gagataactt gtaccaacat      1860
   tggtattatt actgggacca aatgtgactt taaaaagaaa aacaaccttg acaaagaaaa 1920
   ctctgattgg ttactaaatc cctatttctg agataagcta catttcaaag aaattctccg 1980
   taaaagaaaa attggattca gttatcatac cagatggctt tcattctcac cactgactca 2040
   attctgaaac aattatattt cagtatggta attataatct aaactatata aacacactgt 2100
   aaacacaaac tttgaacaga tgaaaactcc gatatgtaaa aaggtaatga atgttgaagg 2160
   aagactgtga aaagggaaaa gaaaaaaaat taaaatgttc cccttctagg tcctgatgag 2220
   agtaaatgtt tactataaaa atgattcaaa tattttaaac acttttcaaa ccaggcaata 2280
   ttttaggcct actgtatatt tgcattttga gcttccaata cggataagtg actggaaaaa 2340
   gcagctaggt ttaggttgaa aaacaacaac ccaccgggga acacatttta gcaaattctt 2400
   ctgaaagtca aaaatgttat agtcataggt aaaaagttac aaagaactac caattgtcag 2460
   aaatagctgc caatattgac ttagaagaca gcagaaggaa ttttagttca agaaacctaa 2520
   aacaggctga aaaccttacc taccctatag ctaccacaaa taacactgtt tccagtcatg 2580
   atcattcctg atcacatatt aagacataac tgcaaattgt gctatactgt actatattaa 2640
   aaggaagtga aatatgatcc ctatcctaga actttccata caaatgaatg taaaacacca 2700
   taaaaattaa tcttaaggcc gggcgcggtg gctcacgcct gtaatcccag cactttggga 2760
   ggccgaggtg ggcggatcac gaggtcagga agtggagacc atcctggcta acacggtgaa 2820
   accccgtctc tactaaaaat acaaaaaatt agccgggcgt ggtggtggac gcctgtagtc 2880
   ccagctactt ggggggccga ggcaggagaa tggcgtgaac ccgggaggcg gagcttgcag 2940
   tgagccgaga tggcgccact gcactccggc ctgggtgaaa gagcgagact ccgtctcaaa 3000
   aacaaaacaa acaaaaatta atcttaagcc aggcgcagtg gctcacgcca gcactttgga 3060
   aggccgaggc gggtggatca cgagatcagg acttcaagac cagcctgacc aacgtgatga 3120
   aaccctatct ctactaaaaa tacaaaatta gccggccacg gtggcgtgcg cctataatcc 3180
   cagctactca ggaggctgag gcaggagaag cgcttgaact tgaacctggc aggcggaggt 3240
   tgcagtgagc caagatggcg ccactgcact ccagcctggg cgacagagcc agactccaac 3300
   cccccacccc gaaaaaaaaa ggtccaggcc gggcgcagtg gctcaggact gtaatcccag 3360
   cactttggaa ggctgaggcg ggtggatcac aaggtcagga gatcgagacc atcttggcta 3420
   acatggtgaa accccgtctc tactaaaaat acaaaaaatt agccgggcat agtggtgggc 3480
   gcctgtagtc ccagctactc gggaggctga ggcaggagaa tggcctgaac ccgggaggcg 3540
   gagctggcag tgagccaaga tcgtgccact gcactccagc ctaggcagca gagcgagacc 3600
   gtgtctcaaa aaaacaaaac aaaacaaaac aaaaagtctg ggagcggtgg ctcacgcctg 3660
   taatcccagc actttcggag gccaaggcag gaggatcacc tgaggtcagg agttcgagac 3720
   caacctgacc aatatggaga aaccctgtct ctactaaaaa tacaaaatta gctggtgtga 3780
   tggcacatgc ctgcaatccc aggtactccg gaggctgagg cagcagaatt gcttgaaccc 3840
   gggaggtgga ggttgtagtg agccgagatt gtgccactgc actccagcct gggcaacaag 3900
   agccaaagtc tgtctcaaaa aaaaaaaaaa aaaaaaaaaa agaaattaat cttaacagga 3960
   aacagaaaaa agcaatgaaa agctagaaaa cataatagtt gattgaaaat aacaatttag 4020
   cattttcatt cttacatctt taatttttat gtatctgagt ttttaattga tggtttaatt 4080
   tgccagaatg agaaagaaca tcctattttt atgactctct cccatggaaa tgaaacataa 4140
   atgtatccaa atgccacact attgaggatt ttcctgatca ctgattgtca tgagtaagtt 4200
   ttgtgctttt tcaaaagcag ttttttccta caatgtcatt tcctgcttct ctggctctga 4260
   ttttcaataa attgataaat tgtgaatcct gttttcctct tatttttgtt tagctataat 4320
   gttgaagggc aagggagagg atggttattt ataaatcttg tatcgctctg aaaacacaac 4380
   atacattttc cttaatctga ttaacttgac ttcaaatatg aaaaacaact ttcataaagc 4440
   agaaaagaat ttaccctttt ttattgtggg taagaggcaa tggta 4485
<210> 134
   <211> 12
   <212> DNA
   <213> Artificial Sequence
<220>
   <223> Synthetic primer prefix appended to the 5' end of primers for cloning purposes
<400> 134
   aaaaggcgcg cc 12
<210> 135
   <211> 12
   <212> DNA
   <213> Artificial Sequence
<220>
   <223> Synthetic primer prefix appended to the 5' end of primers for cloning purposes
<400> 135
   aaaattaatt aa 12